# EUROPÄISCHE PATENTSCHRIFT

(11) **EP 4 121 217 B1**
(45) Veröffentlichungstag und Bekanntmachung des Hinweises auf die Patenterteilung: **11.12.2024**
(21) Anmeldenummer: 21712390.0
(22) Anmeldetag: 15.03.2021
(51) Int. Cl.: B05B 11/00

(54) **VERFAHREN ZUR MONTAGE VON ABGABEVORRICHTUNGEN UND ABGABEVORRICHTUNG**
DISPENSING DEVICE AND METHOD FOR MOUNTING DISPENSING DEVICES
PROCÉDÉ DE MONTAGE DE DISPOSITIFS DE DISTRIBUTION ET DISPOSITIF DE DISTRIBUTION

(30) Priorität: 18.03.2020 EP 20163813
(43) Veröffentlichungstag der Anmeldung: 25.01.2023
(73) Patentinhaber: Boehringer Ingelheim Microparts GmbH, 44227 Dortmund (DE)
(72) Erfinder: HAUSMANN, Matthias, 55216 Ingelheim am Rhein (DE)
(74) Vertreter: Lutze, Oliver
(86) Internationale Anmeldenummer: PCT/EP2021/056511
(87) Internationale Veröffentlichungsnummer: WO 2021/185751

(56) Entgegenhaltungen:
- WO-A1-2007/051536
- US-A1- 2007 282 276

## Beschreibung

Die vorliegende Erfindung betrifft ein Verfahren zur Montage von Abgabevorrichtungen zur Abgabe eines Arzneimittels gemäß dem Oberbegriff des Anspruchs 1 sowie eine Abgabevorrichtung zur Abgabe eines Arzneimittels gemäß dem Oberbegriff des Anspruchs 19.

Insbesondere betrifft die vorliegende Erfindung die Anordnung bzw. Montage eines Dichtelements in einem dem Dichtelement zugeordneten Aufnahmeraum der Abgabevorrichtung. Das Dichtelement dient vorzugsweise zur Abdichtung eines Förderelements zur Förderung eines Arzneimittels aus einem Behälter der Abgabevorrichtung. Hierzu liegt das Dichtelement in der fertig montierten Abgabevorrichtung dichtend an dem Förderelement an. Das Förderelement ist vorzugsweise relativ zu dem Dichtelement bewegbar. Insbesondere wird das Förderelement zur Förderung des Arzneimittels relativ zu dem Dichtelement bewegt.

Bei der Abdichtung des Förderelements mittels des Dichtelements sind verschiedene Anforderungen zu erfüllen. Einerseits soll auch bei einer langen Lagerung bzw. Lebensdauer der Abgabevorrichtung eine zuverlässige Abdichtung gewährleistet sein. Mit anderen Worten soll also eine dauerhafte und zuverlässige "statische Abdichtung" des Förderelements durch das Dichtelement erreicht werden. Andererseits soll bei der Benutzung der Abgabevorrichtung, insbesondere also bei einer Bewegung des Förderelements relativ zu dem Dichtelement, eine zuverlässige Abdichtung gewährleistet sein, d.h. es soll eine sogenannte "dynamische Abdichtung" erreicht werden.

Die gewünschte Abdichtung sowohl im statischen Fall, bei dem das Dichtelement relativ zu dem Förderelement ruht, als auch im dynamischen Fall, bei dem das Förderelement relativ zu dem Dichtelement bewegt wird, stellen unter Umständen verschiedene, zueinander konträre Anforderungen dar. Eine besonders gute statische Dichtung kann dadurch erreicht werden, dass das Dichtelement möglichst großflächig und/oder fest an dem Förderelement anliegt. Dies ist jedoch nachteilig für die dynamische Abdichtung, da eine großflächige und feste Anlage des Dichtelements an dem Förderelement zu einem erhöhten Abrieb bzw. einer Beschädigung des Dichtelements und zu einem erhöhten Kraftaufwand bei der Bewegung des Förderelements relativ zu dem Dichtelement bzw. zu einer verlangsamten Bewegung des Förderelements führen können.

Des Weiteren sollte für eine gute statische Dichtung die Abdichtung des Förderelements durch das Dichtelement möglichst diffusionsdicht sein. Eine Diffusion durch das Dichtelement sollte also verhindert oder zumindest minimiert werden.

Die WO 2004/053362 A1 betrifft ein Kolbenpumpsystem mit einem in einer Führungsröhre geführten Kolben, der entlang seiner Längsachse eine Hubbewegung ausführen kann, wobei in der Führungsröhre eine in einer Nut gehaltene O-Ringdichtung vorgesehen ist, die den Kolben abdichtet. Es wurde erkannt, dass eine gute Abdichtung erreicht werden kann, wenn der Füllgrad der Dichtung in der Nut optimal eingestellt wird. Der "Füllgrad" entspricht dem Quotienten aus dem Volumen der (unverformten) Dichtung und dem Volumen der Nut. Es wird vorgeschlagen, einen Füllgrad von mehr als 90 % zu verwenden, also einen ausreichend voluminösen Dichtring einzusetzen.

Ein Verfahren, durch das eine zuverlässige Abdichtung eines Förderelements einer Abgabevorrichtung erreicht werden soll, ist beispielsweise aus der WO 2007/051536 A1 bzw. der US 2007/0282276 A1 bekannt. Es wurde erkannt, dass die Dichtelemente gewissen Fertigungstoleranzen unterliegen, durch die sich die Dichtelemente verschiedener Chargen in ihrer Größe unterscheiden, sodass eine zuverlässige Abdichtung problematisch ist. Zur Behebung dieses Problems wird für jede Charge von Dichtelementen eine insbesondere zum Volumen korrespondierende Größe der Dichtelemente bestimmt. Jede Charge von Dichtelementen wird einer bestimmten Gruppe von Komponenten der Abgabevorrichtung zugeordnet, wobei die Komponenten jeweils einen nutförmigen Aufnahmeraum für das Dichtelement bilden und die verschiedenen Gruppen von Komponenten sich in der Größe des Aufnahmeraums unterscheiden. Jede Charge von Dichtelementen wird dann mit einer bestimmten Gruppe von Komponenten der Abgabevorrichtung kombiniert, sodass ein gewünschter Füllgrad und dadurch eine gute Abdichtung durch das Dichtelement erreicht werden.

Bei dem vorgenannten Verfahren besteht ein erhöhter Lagerbedarf zur Lagerung der unterschiedlichen Chargen von Dichtelementen und der unterschiedlichen Gruppen von Komponenten. Zudem ist das Verfahren nicht flexibel für verschiedene Dichtungsmaterialien einsetzbar.

Es ist eine Aufgabe der vorliegenden Erfindung, eine einfache und flexible Lösung zur zuverlässigen und dauerhaften Abdichtung eines Förderelements einer Abgabevorrichtung zur Abgabe eines Arzneimittels anzugeben.

Die obige Aufgabe wird gelöst durch ein Verfahren gemäß Anspruch 1 oder eine Abgabevorrichtung gemäß Anspruch 19. Vorteilhafte Weiterbildungen sind Gegenstand der Unteransprüche.

Vorschlagsgemäß wird bzw. ist ein Dichtelement in einem Abgaberaum einer Abgabevorrichtung mit einem zuvor bestimmten Montageparameter eingebaut.

Der Montageparameter kann für jedes Dichtelement individuell oder stichprobenartig für eine Charge von Dichtelementen bestimmt werden.

Die Bestimmung des Montageparameters kann entweder in einem Aufnahmeraum der Abgabevorrichtung oder, insbesondere im Fall einer stichprobenartigen Bestimmung, in einer Prüfanlage erfolgen. Hierzu weist die Prüfanlage vorzugsweise einen Aufnahmeraum auf, der insbesondere die gleichen Abmessungen wir der Aufnahmeraum der Abgabevorrichtung aufweist.

Insbesondere betrifft die vorliegende Erfindung ein Verfahren zur Montage von Abgabevorrichtungen zur Abgabe eines Arzneimittels sowie eine Abgabevorrichtung zur Abgabe eines Arzneimittels, wobei jeweils ein Dichtelement mit dem bestimmten Montageparameter in dem Aufnahmeraum der Abgabevorrichtung angeordnet und fixiert wird bzw. ist.

Vorzugsweise wird das in dem Aufnahmeraum einer Abgabevorrichtung oder Prüfanlage angeordnete Dichtelement verformt und dabei anhand des Verformungsverhaltens und/oder Reibungsverhaltens des Dichtelements während der Verformung der Montageparameter bestimmt.

Nach der Bestimmung des Montageparameters wird das Dichtelement oder ein anderes zu montierendes Dichtelement unter Verwendung des Montageparameters in dem Aufnahmeraum einer Abgabevorrichtung fixiert.

Die Abgabevorrichtung, die montiert wird bzw. in der das Dichtelement fixiert wird, kann die gleiche Abgabevorrichtung sein, in der bzw. in deren Aufnahmeraum das Dichtelement zur Bestimmung des Montageparameters angeordnet wurde. Es ist jedoch auch möglich, dass die Abgabevorrichtung, die montiert wird bzw. in der das Dichtelement fixiert wird, eine andere Abgabevorrichtung bzw. ein anderes Exemplar der gleichen Abgabevorrichtung ist oder ein anderes Exemplar des gleichen Aufnahmeraums aufweist, als die/der bei der Bestimmung des Montageparameters verwendete Abgabevorrichtung bzw. Aufnahmeraum. Dies ist insbesondere bei dem nachfolgend näher erläuterten Chargenverfahren der Fall.

Durch die Bestimmung des Montageparameters anhand des Verformungsverhaltens und/oder Reibungsverhaltens bzw. die Verwendung des Montageparameters können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Der Montageparameter ist vorzugsweise ein einstellbarer, insbesondere geometrischer, Wert, der bei der Montage der Abgabevorrichtung einzuhalten ist und/oder bei der fertig montierten Abgabevorrichtung realisiert wird. Vorzugsweise kann der Montageparameter in den Einstellungen eines Montageablaufs bevorzugt variabel vorgegeben werden. Vorzugsweise ist der Montageparameter ein geometrischer Parameter, insbesondere eine, vorzugsweise relative, Position eines Bauteils oder ein Streckenmaß, wie z. B. eine Höhe des Aufnahmeraums der Abgabevorrichtung. Insbesondere ist der Montageparameter eine Position eines Fixierelements relativ zu einem Führungselement, wobei das Dichtelement mittels des Fixierelements in dem Führungselement bzw. einem Aufnahmeraum des Führungselements fixiert wird.

Bevorzugt liegen mehrere Dichtelemente in Chargen vor. Dabei wird vorzugsweise für jede Charge separat anhand einer Stichprobe von Dichtelementen dieser Charge ein Montageparameter bestimmt. Dieser für die Charge bestimmte Montageparameter wird vorzugsweise bei jeder Fixierung eines Dichtelements der Charge in einem Aufnahmeraum verwendet.

Vorzugsweise erfolgt die Bestimmung des Montageparameters für eine Charge separat bzw. unabhängig, insbesondere zeitlich und/oder räumlich getrennt, von der Montage von Dichtelementen bzw. Abgabevorrichtungen, bevorzugt in einer anderen Anlage als die Montage von Dichtelementen bzw. Abgabevorrichtungen. Mit anderen Worten erfolgen die Bestimmung des Montageparameters und die Montage von Abgabevorrichtungen vorzugsweise separat voneinander. Dieses Verfahren wird nachfolgend insbesondere auch als "Chargenverfahren" bezeichnet.

Der Montageparameter wird vorzugsweise jeweils so gewählt, dass sich bei fertig montierten Abgabevorrichtungen für unterschiedliche Chargen bzw. chargenunabhängig der gleiche Verformungswert des Dichtelements in dem Aufnahmeraum ergibt. Der Verformungswert ist insbesondere ein Maß dafür, wie stark das Dichtelement verformt, insbesondere komprimiert, ist. Hierdurch können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Der Verformungswert wird bei der Montage vorzugsweise mittels einer, insbesondere axialen, Position eines Fixierelements bzw. Anlageelements, mit dem das Dichtelement verformt wird, eingestellt. Insbesondere wird also mittels des Montageparameters ein bestimmter Verformungswert des Dichtelements realisiert.

Vorzugsweise wird zur Bestimmung des Montageparameters einer Charge zunächst für jedes Dichtelement der Stichprobe separat ein Montageparameter bestimmt und ein Mittelwert dieser separat bzw. für einzelne Dichtelemente bestimmten Montageparameter als Montageparameter für alle Dichtelemente der Charge festgelegt. Mit anderen Worten ist der für eine Charge bestimmte Montageparameter also vorzugsweise ein Mittelwert aus mehreren Montageparametern. Hierdurch können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Bei der Bestimmung des Montageparameters wird vorzugsweise ein Zentralelement durch eine Öffnung des Dichtelements geführt, wobei das Volumen des Aufnahmeraums durch das Zentralelement verringert oder begrenzt wird. Dies ist einer erleichterten Montage und einer gleichbleibenden Qualität bei der Herstellung bzw. Montage einer Vielzahl von Abgabevorrichtungen, insbesondere in einem automatisierten Prozess, zuträglich.

Vorzugsweise wird das Zentralelement nach dem Fixieren des Dichtelements aus dem Aufnahmeraum und/oder der Öffnung des Dichtelements entfernt. Die Verwendung des Zentralelements bietet den Vorteil, dass die Verformung des Dichtelements während der Bestimmung des Montageparameters einer Verformung entspricht, die das Dichtelement bei der fertig montierten Abgabevorrichtung aufweist, ohne dass bei der Bestimmung des Montageparameters auch bereits das Förderelement montiert sein muss. Die Verwendung des Zentralelements ist daher einer einfachen und zulässigen Bestimmung des Montageparameters und/oder Sicherstellung einer zuverlässigen Abdichtung bzw. Dichtwirkung zuträglich. Ferner ermöglicht die Verwendung des Zentralelements es, die Bestimmung des Montageparameters unabhängig von der Montage des Dichtelements bzw. vor der Montage des Dichtelements und/oder in einer anderen Anlage als die Montage des Dichtelements vorzunehmen. Dies ist einem optimalen Ablauf bei der Bestimmung des Montageparameters und/oder der Montage des Dichtelements, insbesondere in einem automatisierten Prozess, zuträglich.

Es ist bevorzugt, dass ein Durchmesser des Zentralelements einem Durchmesser eines Förderelements der Abgabevorrichtung entspricht, das zur Förderung von Arzneimitteln, insbesondere aus einem Behälter der Abgabevorrichtung, ausgebildet ist und/oder das einer Austragseinrichtung der Abgabevorrichtung zur Erzeugung eines Aerosols, zugeordnet bzw. damit verbunden ist. Dies ist einer zuverlässigen Abdichtung bzw. Dichtwirkung zuträglich.

Die Anzahl von Dichtelementen der Stichprobe beträgt vorzugsweise weniger als 50 %o, bevorzugt weniger als 20 %o, insbesondere weniger als 10 %o, besonders bevorzugt weniger als 5 ‰, ganz besonders bevorzugt weniger als 2 ‰, der Anzahl von Dichtelementen der Charge. Dies ermöglicht einen geringen Aufwand bei der Bestimmung des Montageparameters bzw. eine schnelle und effiziente Bestimmung des Montageparameters.

Alternativ oder zusätzlich weichen die Volumina der Dichtelemente einer Charge vorzugsweise um weniger als 10 %, insbesondere weniger als 5 %, besonders bevorzugt weniger als 4 %, vom Soll-Volumen bzw. mittleren Volumen der Dichtelemente der Charge ab. Dies ist einer gleichbleibenden Qualität bei der Herstellung bzw. Montage einer Vielzahl von Abgabevorrichtungen, insbesondere in einem automatisierten Prozess, zuträglich.

Die voranstehend erläuterten Merkmale bezogen sich insbesondere auf ein nachfolgend auch als "Chargenverfahren" bezeichnetes Verfahren, bei dem die Dichtelemente in Chargen hergestellt werden bzw. vorliegen und für jede Charge zunächst anhand einer Stichprobe ein Montageparameter bestimmt wird, wobei dieser Montageparameter später für die Montage jedes Dichtelements der Charge verwendet wird.

Es ist jedoch auch möglich, bei jeder Montage eines Dichtelements zunächst den Montageparameter für dieses Dichtelement zu bestimmen und anschließend, insbesondere unmittelbar nach der Bestimmung des Montageparameters, das Dichtelement unter Verwendung des bestimmten Montageparameters in dem Aufnahmeraum zu fixieren. Dies ist insbesondere unabhängig davon, ob die Dichtelemente in Chargen vorliegen bzw. hergestellt wurden und/oder ob die einzelnen Dichtelemente stark voneinander abweichen oder nicht. Das nachfolgend erläuterte Verfahren, das im weiteren Verlauf auch als "Individualverfahren" bezeichnet wird, ist daher besonders vorteilhaft, wenn einzelne Dichtelemente stark voneinander abweichen, kann jedoch auch für beliebige, insbesondere auch innerhalb einer Charge sehr ähnliche, Dichtelemente realisiert werden.

Bei dem Individualverfahren, bei dem vorzugsweise ebenfalls das in dem Aufnahmeraum angeordnete Dichtelement verformt wird und während der Verformung, wie oben beschrieben, ein Montageparameter bestimmt wird, wird das Dichtelement unmittelbar nach dem Verformen des Dichtelements und dem Bestimmen des Montageparameters mittels eines auf das Dichtelement wirkenden Fixierelements bzw. Anlageelements in dem Aufnahmeraum fixiert. Hierdurch können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Bei dem Individualverfahren, bei dem das Dichtelement unmittelbar nach dem Verformen des Dichtelements und dem Bestimmen des Montageparameters in dem Aufnahmeraum fixiert wird, wird der Montageparameter vorzugsweise für jedes zu montierende Dichtelement separat bestimmt und jeweils nur bei der Fixierung desjenigen Dichtelements verwendet, für das er bestimmt wurde. Dies ist von Vorteil, um bei Dichtelementen, die stark voneinander abweichen, eine zuverlässige Abdichtung bzw. Dichtwirkung sicherzustellen.

Die nachfolgend näher erläuterten Aspekte gelten vorzugsweise sowohl für das Verfahren, bei dem zunächst anhand einer Stichprobe der Montageparameter für alle Dichtelemente einer Charge bestimmt wird und die Montage (unter Verwendung des Montageparameters) separat von der Bestimmung des Montageparameters erfolgt (Chargenverfahren), als auch für das Verfahren, bei dem für jedes Dichtelement separat ein Montageparameter bestimmt wird und das Dichtelement unmittelbar nach der Bestimmung des Montageparameters unter Verwendung dieses Montageparameters in dem Aufnahmeraum fixiert wird (Individualverfahren).

Es ist bevorzugt, dass der Montageparameter eine, insbesondere axiale, Position eines Fixierelements und/oder Anlageelements relativ zu einem Führungselement einer Abgabevorrichtung darstellt bzw. hierzu korrespondiert bzw. dass die (relative) Position des Fixierelements und/oder Anlageelements durch den Montageparameter definiert oder festgelegt wird. Hierbei weist das Führungselement vorzugsweise den Aufnahmeraum auf oder bildet diesen, zumindest teilweise. Weiter ist der Aufnahmeraum vorzugsweise durch das Fixierelement bzw. Anlageelement begrenzt.

Vorzugsweise ist das Fixierelement bzw. Anlageelement zum Fixieren des Dichtelements in dem Aufnahmeraum ausgebildet bzw. fixiert das Fixierelement bzw. Anlageelement das Dichtelement in dem Aufnahmeraum. Besonders bevorzugt ist das Anlageelement zwischen dem Dichtelement und dem Fixierelement angeordnet bzw. anordenbar, wobei das Anlageelement das Dichtelement unmittelbar kontaktiert und im Aufnahmeraum fixiert. Das Anlageelement ist vorzugsweise mittels des Fixierelements fixiert bzw. fixierbar. Insbesondere wird also mittels des Fixierelements unmittelbar das Anlageelement fixiert und damit indirekt bzw. mittelbar das Dichtelement in dem Aufnahmeraum fixiert. Alternativ kann das Anlageelement auch einstückig mit dem Fixierelement ausgebildet sein. Hierdurch können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Unterschiedliche Montageparameter korrespondieren vorzugsweise zu unterschiedlichen Volumina des Aufnahmeraums bzw. zu unterschiedlichen Differenzen zwischen den Volumina des Dichtelements und des Aufnahmeraums. Durch den Montageparameter ist also das Volumen des Aufnahmeraums einstellbar bzw. variierbar, sodass Fertigungstoleranzen sowie Größen- und/oder Materialunterschiede von Dichtelementen und/oder des Aufnahmeraums ausgeglichen werden können. Dies ist einer zuverlässigen Abdichtung bzw. Dichtwirkung und gleichbleibenden Qualität bei der Produktion einer Vielzahl von Abgabevorrichtungen, insbesondere in einem automatisierten Prozess, zuträglich.

Der Montageparameter wird vorzugsweise derart gewählt bzw. bestimmt, dass bei einer Fixierung des Dichtelements ein zu der Verformung des Dichtelements korrespondierender Verformungswert einen Schwellwert erreicht oder überschreitet. Für unterschiedliche Dichtelemente, die sich beispielsweise in ihrer äußeren Form und/oder durch das Material, aus dem sie bestehen, unterscheiden, sind vorzugsweise unterschiedliche Schwellwerte vorgesehen. Der Schwellwert ist vorzugsweise vorgegeben oder vorgebbar. Auf diese Weise kann auf einfache Weise eine zuverlässige Abdichtung bzw. Dichtwirkung und damit eine gleichbleibende Qualität bei der Produktion einer Vielzahl von Abgabevorrichtungen erreicht werden. Zudem ist das Verfahren auf unterschiedliche Dichtelemente anpassbar und damit flexibel einsetzbar.

Der Verformungswert ist vorzugsweise ein Maß dafür, wie stark das Dichtelement verformt ist. Bei der Verformung des Dichtelements in dem, insbesondere ringförmigen, Aufnahmeraum, beispielsweise durch einen Stößel, erfolgt vorzugsweise anfänglich zunächst eine, insbesondere elastische, Deformation des Dichtelements. Bei der Deformation bleibt das Volumen des Dichtelements vorzugsweise zumindest näherungsweise konstant und es ändert sich zumindest im Wesentlichen nur die äußere geometrische Form des Dichtelements. Bei dieser Verformung bzw. Deformation des Dichtelements passt sich die Form des Dichtelements an die Form des Aufnahmeraums an und füllt diesen zusehends weiter aus. Dies setzt sich fort, bis das Dichtelement den Aufnahmeraum zumindest näherungsweise vollständig ausfüllt und/oder die Form des Dichtelements der Form des Aufnahmeraums zumindest näherungsweise entspricht.

Wenn nun das Dichtelement weiter verformt bzw. komprimiert oder in dem Aufnahmeraum gepresst wird, setzt vorzugsweise eine Volumenkompression des Dichtelements ein. Bei der Volumenkompression verringert sich - im Gegensatz zur (elastischen) Deformation - das Volumen des Dichtelements.

Zusammenfassend lässt sich das Verhalten des Dichtelements bei Verformung in dem Aufnahmeraum zumindest näherungsweise in zwei Phasen aufteilen, wobei in der ersten Phase im Wesentlichen eine Deformation bei konstantem Volumen erfolgt, während in der zweiten Phase eine Volumenkompression erfolgt.

Selbstverständlich ist es nicht ausgeschlossen, dass gegen Ende der ersten Phase bzw. während der Deformation bereits eine (geringe) Volumenkompression einsetzt.

Der vorgenannte Schwellwert bzw. Schwellwert für den Verformungswert - also der Schwellwert, der für die Bestimmung bzw. Wahl des Montageparameters herangezogen wird - korrespondiert vorzugsweise zu einer einsetzenden Volumenkompression des Dichtelements. Mit anderen Worten ist der Schwellwert vorzugsweise derjenige Wert des Verformungswerts, bei dem eine Volumenkompression des Dichtelements einsetzt. Hierdurch kann eine zuverlässige Abdichtung bzw. Dichtwirkung sichergestellt werden. Des Weiteren wird ein übermäßiger Verschleiß bzw. Abrieb vermieden. Dies ist einer gleichbleibenden Qualität bei der Herstellung bzw. Montage bei einer Vielzahl von Abgabevorrichtungen, insbesondere in einem automatisierten Prozess, zuträglich.

Besonders bevorzugt wird das Dichtelement durch Bewegung eines Stößels und/oder des Anlageelements bzw. Fixierelements relativ zu dem Dichtelement verformt. Der Stößel kann auf das Anlage- bzw. Fixierelement einwirken und damit das mittelbar das Anlage- bzw. Fixierelement relativ zu dem Dichtelement bewegen und damit das Dichtelement über das Anlage- bzw. Fixierelement verformen. Es ist jedoch auch möglich, direkt mit dem Stößel auf das Dichtelement einzuwirken bzw. das Dichtelement zu verformen.

Die Bewegung des Stößels und/oder Anlageelements bzw. Fixierelements relativ zu dem Dichtelement erfolgt vorzugsweise in axialer und/oder radialer Richtung. Insbesondere ist das Dichtelement in dem Aufnahmeraum angeordnet, sodass die Kraft bzw. der Druck, die/der mittels des Stößels und/oder Anlageelements bzw. Fixierelements auf das Dichtelement ausgeübt wird, in einer Verformung und/oder Kompression des Dichtelements in dem Aufnahmeraum resultiert.

Zur Bestimmung bzw. Untersuchung des Verformungsverhaltens des jeweiligen Dichtelements wird bei unterschiedlichen Verformungen bzw. Verformungswerten des Dichtelements die jeweils zur Verformung benötigte Kraft (direkt oder indirekt) bestimmt, insbesondere gemessen. Auf diese Weise kann die für einen bestimmen Verformungswert benötigte Kraft bestimmt werden. Die zur Verformung benötigte Kraft, die bestimmt bzw. gemessen wird, stellt insbesondere einen Kennwert dar, aus dem der Verformungswert bestimmt, berechnet oder abgeleitet werden kann bzw. wird. Alternativ kann auch die die Verformung bewirkende Kraft vorgegeben und die Verformung bzw. die zur Verformung korrespondiere Zustellung bzw. axiale Position des Stößels und/oder Anlageelements bzw. Fixierelements oder insbesondere die Höhe des Aufnahmeraums bestimmt, insbesondere gemessen, werden.

Alternativ oder zusätzlich zur Bestimmung bzw. Untersuchung des Verformungsverhaltens des Dichtelements kann, wie eingangs bereits erwähnt, auch das Reibungsverhalten des Dichtelements bestimmt bzw. untersucht werden. Zur Bestimmung bzw. Untersuchung des Reibungsverhaltens des jeweiligen Dichtelements werden vorzugsweise eine Begrenzung des Aufnahmeraums und das Dichtelement relativ zueinander bewegt und bei unterschiedlichen Verformungen bzw. Verformungswerten des Dichtelements jeweils die Reibungskraft zwischen dem Dichtelement und der Begrenzung bestimmt, insbesondere gemessen. Die gemessene Kraft stellt vorzugsweise den Kennwert dar, aus dem der Verformungswert bestimmt, berechnet oder abgeleitet werden kann bzw. wird. Vorzugsweise ist die relativ zu dem Dichtelement bewegte Begrenzung des Aufnahmeraums das vorgenannte Zentralelement, das während der Bestimmung des Montageparameters durch den Aufnahmeraum bzw. die Aufnahme des Dichtelements geführt ist. In diesem Fall wird das Zentralelement bzw. die Begrenzung vorzugsweise axial zu dem Dichtelement bewegt, insbesondere in einer oszillatorischen Bewegung bzw. Hin-und-Her-Bewegung. Es ist jedoch auch möglich, dass das Dichtelement relativ zu dem Aufnahmeraum bzw. der Begrenzung, insbesondere dem Zentralelement, rotiert wird. In diesem Fall ist die Reibungskraft zwischen dem Dichtelement und der Begrenzung vorzugsweise eine Torsionsreibungskraft. Entsprechend ist dann vorzugsweise auch der Kennwert bzw. die zur Bestimmung des Verformungswerts gemessene Kraft eine Torsionsreibungskraft.

Zu unterschiedlichen Zeitpunkten während der Verformung ist das Dichtelement in dem Aufnahmeraum vorzugsweise jeweils unterschiedlich stark verformt. Die unterschiedlichen Zeitpunkte korrespondieren daher vorzugsweise zu unterschiedlichen Vorschüben bzw. Positionen des Fixierelements bzw. Anlageelements.

Insbesondere wird zur Bestimmung des Verformungsverhaltens und/oder Reibungsverhaltens und/oder zur Bestimmung des Montageparameters eine Kraft-Weg-Kurve aufgenommen. Bevorzugt wird der Montageparameter anhand des Verlaufs der Kraft-Weg-Kurve bestimmt. Dies ist einer einfachen, schnellen und/oder genauen Bestimmung des Montageparameters zuträglich.

Die Kraft in der Kraft-Weg-Kurve ist vorzugsweise die zur Verformung des Dichtelements aufgewandte Kraft und/oder die Reibungskraft zwischen dem Dichtelement und der relativ zu dem Dichtelement bewegten Begrenzung des Aufnahmeraums.

Der Weg in der Kraft-Weg-Kurve ist vorzugsweise der Vorschub des Stößels bzw. Fixierelements bzw. Anlageelements oder die Höhe des Aufnahmeraums oder ein dazu korrespondierender Weg.

Das Dichtelement ist vorzugsweise zur Abdichtung eines Förderelements zur Förderung des Arzneimittels gegen ein den Aufnahmeraum aufweisendes oder bildendes Führungselement, in dem das Förderelement geführt ist, ausgebildet.

Vorzugsweise ist das Dichtelement ringförmig ausgebildet bzw. eine Formdichtung oder ein Dichtring, insbesondere O-Ring.

Das Dichtelement ist vorzugsweise ein separates, in den Aufnahmeraum einsetzbares Bauteil. Bevorzugt besteht das Dichtelement aus einem elastischen Material. Ein elastisches Material im Sinne der Erfindung ist vorzugsweise ein Material mit einem Elastizitätsmodul von weniger als 100 MPa, vorzugsweise weniger als 50 MPa, insbesondere weniger als 10 MPa.

Die Dichtelemente unterschiedlicher Chargen können aus unterschiedlichen Materialien bestehen, und/oder unterschiedliche Verformungseigenschaften, unterschiedliche Druckverformungsreste und/oder ein unterschiedliches Kriechverhalten bzw. unterschiedliche Kriechmoduln aufweisen. Durch das Verfahren, bei dem für jede Charge separat ein Montageparameter bestimmt wird, wird erreicht, dass auch für solche unterschiedlichen Dichtelemente bzw. Chargen von Dichtelementen jeweils eine zuverlässige Abdichtung bzw. Dichtwirkung erreicht wird. Insbesondere können durch die bei diesem Verfahren mögliche genaue Einstellung des Volumens des Aufnahmeraums auch Materialien für Dichtelemente eingesetzt werden, die - wie beispielsweise thermoplastische Elastomere (TPE) - teilplastisch sind bzw. ein vergleichsweise hohes Kriechverhalten aufweisen. Derartige Dichtelemente benötigen besonders exakt ausgelegte bzw. an das jeweilige Dichtelement angepasste Aufnahmeräume.

Gemäß einem weiteren, auch unabhängig realisierbaren Aspekt betrifft die vorliegende Erfindung eine Abgabevorrichtung zur Abgabe eines Arzneimittels. Die Abgabevorrichtung weist ein, insbesondere axial bewegbares, Förderelement zur Förderung des Arzneimittels aus einem Behälter der Abgabevorrichtung, ein Führungselement zur Führung des Förderelements und ein, insbesondere ringförmiges, Dichtelement zur Anordnung in einem Aufnahmeraum des Führungselements auf. Das Dichtelement ist vorzugsweise zur Abdichtung des Förderelements gegen das Führungselement und insbesondere als Dichtring ausgebildet. Weiter ist das Dichtelement vorzugsweise mittels eines an dem Führungselement befestigbaren Fixierelements bzw. Anlageelements in dem Aufnahmeraum fixierbar. Bevorzugt bildet das Führungselement die Druckkammer einer Kolbenpumpe oder weist das Führungselement die Druckkammer auf, wobei das Förderelement den Kolben der Kolbenpumpe bildet.

Das Fixierelement bzw. Anlageelement ist vorzugsweise in verschiedenen axialen Positionen relativ zu dem Führungselement an dem Führungselement befestigbar. Hierdurch ist es möglich und vorgesehen, dass das Dichtelement mit einem angepassten, bestimmten Montageparameter fixiert ist bzw. wird. So können auf einfache Weise Fertigungstoleranzen sowie Größen- und/oder Materialunterschiede von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Bevorzugt ist das Fixier- bzw. Anlageelement in verschiedenen axialen Positionen relativ zu dem Führungselement an dem Führungselement einrastbar. Dies erleichtert die korrekte bzw. gewünschte Positionierung des Fixier- bzw. Anlageelements und ist damit einer einfachen und fehlerfreien Montage zuträglich. Dies ist einer gleichbleibenden Qualität bei der Herstellung bzw. Montage einer Vielzahl von Abgabevorrichtungen, insbesondere in einem automatisierten Prozess, zuträglich.

Alternativ oder zusätzlich weist das Fixier- bzw. Anlageelement und/oder das Führungselement vorzugsweise eine Positioniereinrichtung zur Positionierung des Fixier- bzw. Anlageelements relativ zu dem Führungselement auf. Besonders bevorzugt weist die Positioniereinrichtung eine oder mehrere Rastelemente, insbesondere Rastnocken, auf oder ist hierdurch gebildet. Dies ist einer erleichterten Montage bzw. Festlegung der Position des Fixier- bzw. Anlageelements zuträglich. Hierdurch können auf einfache Weise Fertigungstoleranzen von Dichtelementen berücksichtigt bzw. ausgeglichen werden und eine zuverlässige Abdichtung bzw. Dichtwirkung sichergestellt werden. Dies ist einer gleichbleibenden Qualität bei der Herstellung bzw. Montage einer Vielzahl von Abgabevorrichtungen, insbesondere in einem automatisierten Prozess, zuträglich.

Die Positioniereinrichtung weist vorzugsweise eine schiefe Ebene oder Helixstruktur auf oder ist hierdurch gebildet. Dies ist einer einfachen Einstellung der Position des Fixier- bzw. Anlageelements relativ zu dem Führungselement zuträglich.

Die Positioniereinrichtung ist vorzugsweise stirnseitig an dem Fixier- bzw. Anlageelement und/oder dem Führungselement angeordnet.

Es ist bevorzugt, dass die Drehlage des Fixier- bzw. Anlageelements, insbesondere relativ zu dem Führungselement bzw. der Abgabevorrichtung, die axiale Position des Fixier- bzw. Anlageelements festlegt. Insbesondere ist die Positioniereinrichtung entsprechend ausgebildet.

Vorzugsweise wird durch die Position des Fixier- bzw. Anlageelements relativ zu dem Führungselement eine bestimmte Verformung bzw. ein bestimmter Verformungswert des Dichtelements und/oder eine Größe bzw. ein Volumen des Aufnahmeraums definiert, sodass die Verformung des Dichtelements und/oder die Größe bzw. das Volumen des Aufnahmeraums durch eine Variation der Position des Fixierelements variierbar bzw. aufeinander anpassbar sind.

Die vorgenannten und nachfolgenden Aspekte und Merkmale der vorliegenden Erfindung können in unterschiedlichen Kombinationen miteinander kombiniert, aber auch unabhängig voneinander realisiert werden.

Weitere Aspekte, Merkmale, Vorteile und Eigenschaften der vorliegenden Erfindung ergeben sich aus den Ansprüchen und der nachfolgenden Beschreibung bevorzugter Ausführungsbeispiele anhand der Zeichnung. Es zeigt:
- Fig. 1: einen schematischen Schnitt einer erfindungsgemäßen Abgabevorrichtung in einem ersten Zustand;
- Fig. 2: einen schematischen, um 90° gegenüber Fig. 1 gedrehten Schnitt der Abgabevorrichtung in einem zweiten Zustand;
- Fig. 3: einen schematischen Schnitt durch einen Druckerzeuger der Abgabevorrichtung;
- Fig. 4A: einen schematischen Schnitt durch den Druckerzeuger während einer ersten Situation bei einer Bestimmung des Montageparameters;
- Fig. 4B: einen schematischen Schnitt durch den Druckerzeuger während einer zweiten Situation bei einer Bestimmung des Montageparameters;
- Fig. 4C: einen schematischen Schnitt durch den Druckerzeuger gemäß Fig. 4A während der ersten Situation bei einer Bestimmung des Montageparameters, wobei das Dichtelement eine andere Größe als in Fig. 4A aufweist;
- Fig. 4D: einen schematischen Schnitt durch den Druckerzeuger gemäß Fig. 4C während der zweiten Situation bei einer Bestimmung des Montageparameters;
- Fig. 4E: einen schematischen Schnitt durch den Druckerzeuger nach Fixierung des Dichtelements;
- Fig. 5A: eine schematische Darstellung des Verlaufs eines Messwerts während einer Verformung bzw. Bestimmung eines Montageparameters;
- Fig. 5B: einen schematischen Schnitt durch ein unverformtes Dichtelement;
- Fig. 5C: einen schematischen Schnitt durch ein verformtes Dichtelement; und
- Fig. 6: eine schematische Seitenansicht des Druckerzeugers gemäß einem weiteren Ausführungsbeispiel.

In den teilweise nicht maßstabsgerechten, nur schematischen Figuren werden für gleiche oder ähnliche Teile dieselben Bezugszeichen verwendet, wobei entsprechende oder vergleichbare Eigenschaften und Vorteile erreicht werden können, auch wenn von einer wiederholten Beschreibung der Übersichtlichkeit halber abgesehen wird.

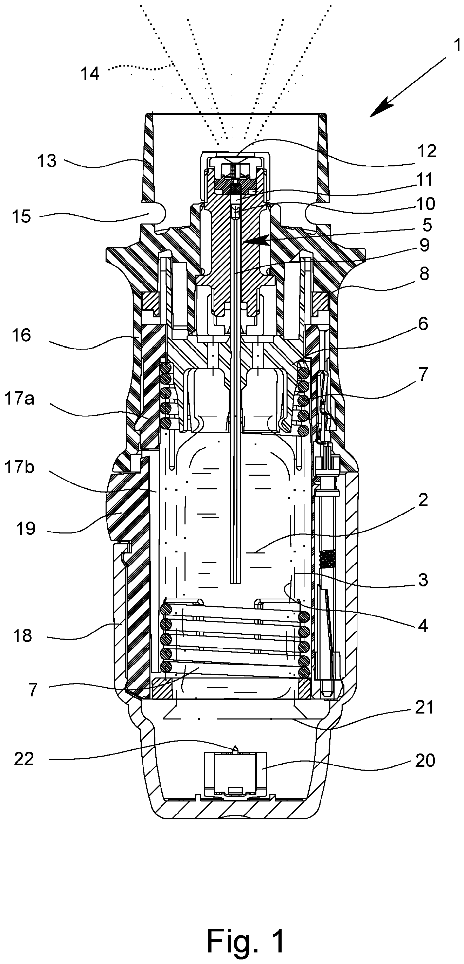

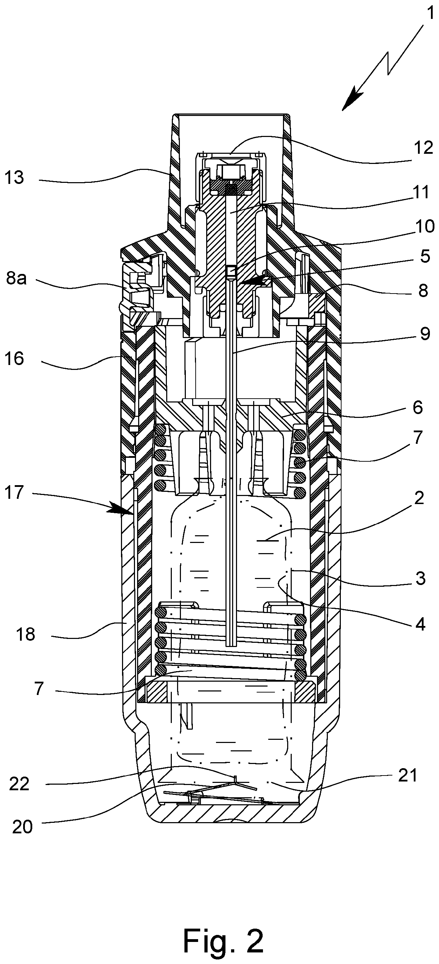

Fig. 1 und Fig. 2 zeigen eine vorschlagsgemäße Abgabevorrichtung 1 zur Abgabe eines Arzneimittels 2. Insbesondere ist die Abgabevorrichtung 1 als ein Zerstäuber bzw. zur Zerstäubung des Arzneimittels 2 ausgebildet. Das Arzneimittel 2 ist vorzugsweise ein Fluid, insbesondere eine Flüssigkeit.

Fig. 1 zeigt dabei die Abgabevorrichtung 1 in einem schematischen Schnitt in einem ersten Zustand, hier im nicht auslösebereiten Zustand und Fig. 2 in einem zweiten Zustand, hier im auslösebereiten Zustand.

Die Abgabevorrichtung 1 ist vorzugsweise als tragbare, portable und/oder mobile Abgabevorrichtung 1 ausgebildet.

Bei Zerstäubung des Arzneimittels 2 mittels der Abgabevorrichtung 1 wird vorzugsweise ein Aerosol 14 gebildet, insbesondere wobei das Aerosol 14 von einem nicht dargestellten Benutzer eingeatmet bzw. inhaliert werden kann. Die Abgabevorrichtung 1 ist also vorzugsweise als Inhalator ausgebildet.

Üblicherweise erfolgt das Inhalieren wenigstens einmal täglich, insbesondere mehrmals täglich, vorzugsweise in vorbestimmten Zeitabständen, beispielsweise in Abhängigkeit von der Erkrankung des Patienten.

Bei dem Aerosol 14 handelt es sich vorzugsweise um ein Partikel-Luft-Gemisch mit festen und/oder flüssigen Partikeln, die besonders bevorzugt lungengängig sind, also insbesondere jedenfalls teilweise oder im Durchschnitt kleiner als 5 µm im Durchmesser sind.

Die Abgabevorrichtung 1 ist vorzugsweise treibgasfrei, arbeitet also vorzugsweise ohne Treibgas. Stattdessen arbeitet die Abgabevorrichtung 1 vorzugsweise mit einem mechanischen Druckerzeuger 5 bzw. Mechanismus zur Erzeugung des Aerosols 14. Insbesondere handelt es sich hierbei um eine mechanisch betriebene Pumpe, die das Arzneimittel 2 unter Druck setzt, wodurch beim Austrag aus einer Austragsdüse 12 das Aerosol 14 erzeugt wird. Grundsätzlich kann die Abgabevorrichtung 1 jedoch auch auf andere, bevorzugt mechanische Weise, angetrieben werden bzw. arbeiten.

Die Abgabevorrichtung 1 weist einen vorzugsweise einsetzbaren und vorzugsweise auswechselbaren Behälter 3 mit der Substanz bzw. dem Arzneimittel 2 auf. Der Behälter 3 bildet vorzugsweise ein Reservoir für das zu zerstäubende Arzneimittel 2.

Vorzugsweise enthält der Behälter 3 eine ausreichende Menge an Arzneimittel 2, um beispielsweise bis zu 200 Dosiereinheiten zur Verfügung stellen zu können, also beispielsweise bis zu 200 Zerstäubungen oder Anwendungen zu ermöglichen. Ein typischer Behälter 3, wie in der WO 96/06011 A2 oder in der WO 00/49988 A2 offenbart, nimmt ein Volumen von ca. 2 ml bis 10 ml auf.

Der Behälter 3 ist vorzugsweise zumindest im Wesentlichen zylindrisch bzw. kartuschenartig ausgebildet. Er ist im Darstellungsbeispiel von unten, nach Öffnen der Abgabevorrichtung 1, in diesen einsetzbar und ggf. wechselbar. Er kann jedoch auch nicht auswechselbar sein und die Abgabevorrichtung 1 kann ein Wegwerfprodukt sein.

Der Behälter 3 ist vorzugsweise zumindest im Wesentlichen starr ausgebildet. Besonders bevorzugt ist der Behälter 3 aus Kunststoff, insbesondere thermoplastischem Kunststoff, ganz besonders bevorzugt Polypropylen, hergestellt. Vorzugsweise weist der Behälter 3 an seinem Boden eine ebene Fläche auf oder hat der Behälter 3 einen ebenen Behälterboden 21.

Vorzugsweise ist das Arzneimittel 2 in einem von einem kollabierbaren Beutel gebildeten Fluidraum 4 im Behälter 3 aufgenommen.

Die Abgabevorrichtung 1 weist vorzugsweise ferner den vorzugsweise mechanischen Druckerzeuger 5 zur Förderung und Zerstäubung des Arzneimittels 2, insbesondere jeweils in einer vorbestimmten, ggf. einstellbaren, Dosiermenge auf. Der Druckerzeuger 5 bildet vorzugsweise eine hoch genaue Pumpe bzw. Dosierpumpe, insbesondere eine Kolbenpumpe.

Der Druckerzeuger 5 weist vorzugsweise eine - im Darstellungsbeispiel nur teilweise dargestellte - Antriebsfeder 7 zum Antrieb des Druckerzeugers 5 auf. Die Antriebsfeder 7 ist vorzugsweise dazu ausgebildet, Energie, insbesondere mechanisch, zu speichern und an den Druckerzeuger 5 abzugeben, so dass der Druckerzeuger 5 das Arzneimittel 2 unter Druck setzt, wodurch aus dem Arzneimittel 2 bei Austrag aus der Austragsdüse 12 das Aerosol 14 gebildet wird. Der Druckerzeuger 5 wird also mit der Antriebsfeder 7 bzw. der durch die Antriebsfeder 7 zur Verfügung gestellten Energie angetrieben.

Der Druckerzeuger 5 weist vorzugsweise eine Halterung 6 für den Behälter 3, die zugeordnete, nur teilweise dargestellte Antriebseinheit, insbesondere in Form der Antriebsfeder 7, sowie ein insbesondere rohrförmiges Förderelement 9, ein Rückschlagventil 10 und/oder eine Pump- bzw. Druckkammer 11 auf.

Vorzugsweise weist die Abgabevorrichtung 1 ein Sperrspannwerk bzw. Sperrelement 8 auf, das zum vorzugsweise automatischen Sperren der Antriebsfeder 7 nach einem Energieeintrag in die Antriebsfeder 7 ausgebildet ist. Der Energieeintrag in die Antriebsfeder 7 erfolgt vorzugsweise mittels Strecken, Dehnen, Stauchen oder insbesondere Komprimieren der Antriebsfeder 7. Dies wird im Folgenden der Einfachheit halber unter dem Begriff "Spannen" der Antriebsfeder 7 zusammengefasst und dem dadurch erzielten Zustand der Antriebsfeder 7 das Attribut "gespannt" zugeordnet (ungeachtet dessen, ob die konkrete Antriebsfeder in der jeweiligen Ausführung hierfür gestreckt oder gestaucht wurde). Vorzugsweise wird die Antriebsfeder 7 nach dem Spannen bzw. Komprimieren von dem insbesondere manuell betätigbaren Sperrelement 8 in einem energetisch höheren bzw. geladenen oder gespannten Zustand gehalten, wobei die - insbesondere durch Spannen - in der Antriebsfeder 7 gespeicherte Energie durch Betätigung des Sperrelements 8 (direkt oder vorzugweise über einer Auslösetaste 8a) zur Druckerzeugung im Druckerzeuger 5 freigesetzt bzw. genutzt wird.

Bevorzugt ist die Antriebsfeder 7 eine Spiral- oder Schraubenfeder, wobei eine Speicherung von Energie bzw. ein Energieeintrag mittels axialer Kompression der Antriebsfeder 7 erfolgt.

Vorzugsweise weist die Abgabevorrichtung 1 bzw. der Druckerzeuger 5 eine Austragsdüse 12 insbesondere im Bereich eines optionalen Mundstücks 13 auf.

Der Behälter 3 wird vorzugsweise über die Halterung 6, insbesondere rastend, so in der Abgabevorrichtung 1 fixiert bzw. mit dem Förderelement 9 fluidisch verbunden, dass das Förderelement 9 in den Behälter 3 eintaucht. Die Halterung 6 kann dabei derart ausgebildet sein, dass der Behälter 3 gelöst und ausgetauscht werden kann.

Beim Energieeintrag in die Antriebsfeder 7 bzw. Spannen der Antriebsfeder 7 wird die Halterung 6 mit dem Behälter 3 und dem Förderelement 9 bei den Darstellungen nach unten bewegt (das heißt im Darstellungsbeispiel wird die Antriebsfeder 7 axial komprimiert bzw. gestaucht) und das Arzneimittel 2 aus dem Behälter 3 über das Rückschlagventil 10 in die Druckkammer 11 des Druckerzeugers 5 gesaugt.

Beim anschließenden Entladen bzw. Entspannen bzw. bei anschließender Relaxation der Antriebsfeder 7 nach Auslösen durch Betätigung des Sperrelements 8 wird das Arzneimittel 2 in der Druckkammer 11 unter Druck gesetzt, indem das Förderelement 9 mit seinem nun geschlossenen Rückschlagventil 10 von der Antriebsfeder 7 wieder nach oben bzw. in die Druckkammer 11 bewegt wird und hierbei als Druckstempel dient. Dieser Druck treibt das Arzneimittel 2 durch die Austragsdüse 12 aus, wobei es in das Aerosol 14 zerstäubt wird, wie in Fig. 1 angedeutet.

Wenn das Förderelement 9 bzw. das Rückschlagventil 10 als Druckstempel fungiert, korrespondiert eine Bewegung des Förderelements 9 bzw. des Behälters 3 zu einem in der Druckkammer 11 verdrängten Volumen bzw. einer ausgetragenen oder austragbaren Arzneimittelmenge.

Ein nicht dargestellter Benutzer bzw. Patient kann das zerstäubte Arzneimittel 2 bzw. das Aerosol 14 inhalieren, vorzugsweise wobei Zuluft über mindestens eine optionale Zuluftöffnung 15 in das Mundstück 13 saugbar ist.

Die Abgabevorrichtung 1 weist beim Darstellungsbeispiel vorzugsweise ein oberes Gehäuseteil 16 und ein demgegenüber drehbares Innenteil 17 (inneres Gehäuseteil) (vgl. Fig. 2) mit einem oberen Teil 17a und einem unteren Teil 17b (vgl. Fig. 1) auf. An dem Innenteil 17 ist vorzugsweise ein insbesondere manuell betätigbares bzw. drehbares unteres Gehäuseteil (Gehäuseunterteil) 18 bzw. eine Kappe vorzugsweise mittels eines Halteelementes 19 lösbar oder unlösbar befestigt, insbesondere aufgesteckt.

Zum Einsetzen und/oder Auswechseln des Behälters 3 ist das Gehäuseunterteil bzw. untere Gehäuseteil 18 vorzugsweise von der Abgabevorrichtung 1 lösbar. Der Behälter 3 kann jedoch auch nicht auswechselbar sein.

Das untere Gehäuseteil 18 kann gegen das obere Gehäuseteil 16 gedreht bzw. relativ zu dem oberen Gehäuseteil 16 verdreht werden, vorzugsweise wobei es dabei den in der Darstellung unteren Teil 17b des Innenteils 17 mitnimmt.

Insbesondere ist das untere Gehäuseteil 18 drehfest an dem Innenteil 17 angeordnet, vorzugsweise formschlüssig mit diesem gekoppelt. Das Innenteil 17 kann auf diese Weise mittels des unteren Gehäuseteils 18 gegen das Gehäuseteil 16 gedreht bzw. relativ zu dem Gehäuseteil 16 verdreht werden. Hier sind jedoch auch andere Lösungen möglich.

Durch das Drehen des oberen Gehäuseteils 16 relativ zu dem unteren Gehäuseteil 18 bzw. dem Innenteil 17 wird die Antriebsfeder 7 über ein nicht dargestelltes, auf die Halterung 6 wirkendes Getriebe in axialer Richtung geladen, insbesondere zusammengedrückt bzw. komprimiert. Mit dem Spannen wird der Behälter 3 vorzugsweise axial nach unten bewegt, bis der Behälter 3 eine in Fig. 2 angedeutete Endlage annimmt. In diesem Zustand ist die Antriebsfeder 7 geladen bzw. die Abgabevorrichtung 1 abgabebereit. Während des Zerstäubungsvorgangs wird der Behälter 3 vorzugsweise von der Antriebsfeder 7 wieder (nach oben) in seine Ausgangslage zurückbewegt.

Der Behälter 3 führt vorzugsweise eine axiale Bewegung oder Hubbewegung während dem Hinzufügen von Energie bzw. während des Spannvorgangs bzw. zur Fluidentnahme und/oder während der Zerstäubung bzw. Abgabe des Arzneimittels 2 aus.

Vorzugsweise wird beim ersten Laden bzw. erstmaligen Spannen der Antriebsfeder 7 eine Belüftungsöffnung des Behälter 3 geöffnet. Vorzugsweise erfolgt dies mittels einer Vorrichtung, die bevorzugt im Gehäuseteil 18 angeordnet ist und/oder ein Anstechelement 22 zum Anstechen des Behälters 3 bzw. einer bodenseitigen Versiegelung des Behälters 3 bei der erstmaligen Anlage zur Belüftung aufweist.

Vorzugsweise ist das Anstechelement 22 an einer axial wirkenden Feder 20 angeordnet, die beim ersten Laden an dem Behälterboden 21 zur Anlage kommt.

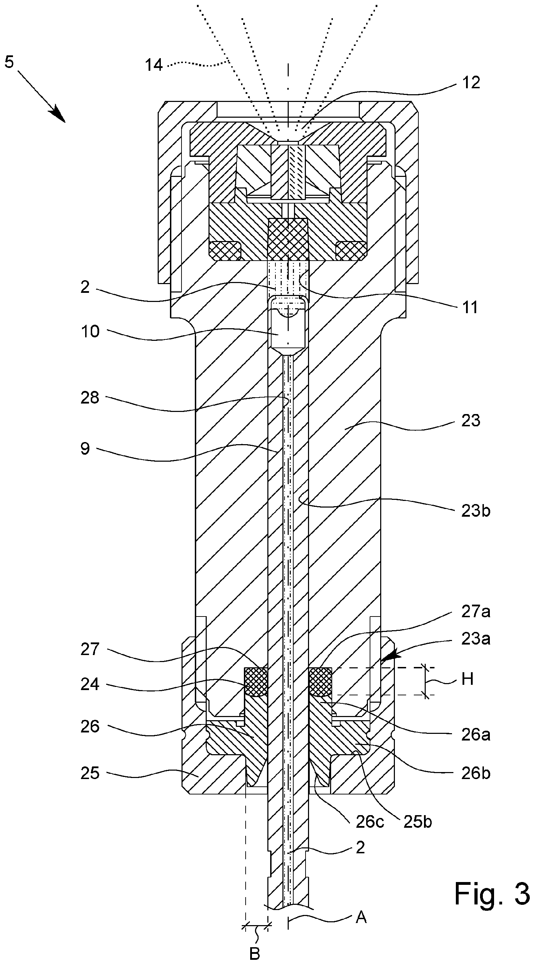

In Fig. 3 ist der Druckerzeuger 5 der Abgabevorrichtung 1 detaillierter, jedoch schematisch und nicht maßstabsgerecht, dargestellt.

Die Abgabevorrichtung 1 bzw. der Druckerzeuger 5 ist vorzugsweise zur Abgabe, insbesondere zum Pumpen bzw. Dosieren, des Arzneimittels 2, vorzugsweise einer Flüssigkeit, ausgebildet. Besonders bevorzugt ist der Druckerzeuger 5 als Kolbenpumpe ausgebildet. Insbesondere ist die Abgabevorrichtung 1 bzw. der Druckerzeuger 5 für sehr kleine Pumpvolumina bzw. Dosierungen ausgelegt. Das Pumpvolumen beträgt beim Darstellungsbeispiel vorzugsweise mehr als 1 µl, insbesondere mehr als 5 µl, und/oder weniger als 1 ml, insbesondere weniger als 500 µl, besonders bevorzugt weniger als 100 µl, ganz besonders bevorzugt weniger als 30 µl, und insbesondere im Wesentlichen 10 bis 20 µl, pro Kolbenhub.

Um eine präzise Förderung und Dosierung des gewünschten Volumens, insbesondere auch bei einer ersten Betätigung der Abgabevorrichtung 1 nach längerer Nichtbenutzung, sicherstellen zu können, darf insbesondere während der Nichtbenutzung keine Luft in den Druckerzeuger 5 eintreten, da sonst die Dosierung nicht mehr in der gewünschten Genauigkeit erfolgen kann.

Die Abgabevorrichtung 1 bzw. der Druckerzeuger 5 weist vorzugsweise eine Achse A auf. Die Achse A entspricht vorzugsweise einer Hauptachse, Längsachse und/oder Symmetrieachse des Druckerzeugers 5 bzw. Förderelements 9 und/oder der Bewegungsachse des Förderelements 9. Insbesondere erstreckt sich der Druckerzeuger 5 im Wesentlichen längs entlang der Achse A.

Die Bezeichnungen "axial" und "radial" beziehen sich insbesondere auf die Achse A. Eine "axiale" Richtung, Erstreckung, Bewegung o. dgl. ist daher vorzugsweise parallel zu der Achse A und eine "radiale" Richtung, Erstreckung, Bewegung o. dgl. ist vorzugsweise radial zu der Achse A.

Die Abgabevorrichtung 1 bzw. der Druckerzeuger 5 weist vorzugsweise ein Führungselement 23 zur Führung des Förderelements 9, ein Dichtelement 24, einen Aufnahmeraum 27 zum Aufnehmen des Dichtelements 24 und insbesondere ein Fixierelement 25 und/oder Anlageelement 26 zum Fixieren bzw. Einspannen und/oder Verformen des Dichtelements 24 in dem Aufnahmeraum 27auf.

Bei der (fertig montierten) Abgabevorrichtung 1 ist das Dichtelement 24 in dem Aufnahmeraum 27 angeordnet.

Das Fixierelement 25 ist vorzugsweise an dem Führungselement 23 fixierbar bzw. befestigbar bzw. bei der fertig montierten Abgabevorrichtung 1 an dem Führungselement 23 fixiert bzw. befestigt.

Vorzugsweise ist das Fixierelement 25 zum Fixieren bzw. Festsetzen des Dichtelements 24 in dem Aufnahmeraum 27 ausgebildet.

Das Anlageelement 26 ist vorzugsweise zum Kontaktieren des Dichtelements 24 bzw. Anlegen an das Dichtelement 24 ausgebildet. Vorzugsweise ist das Anlageelement 26 zwischen dem Dichtelement 24 und dem Fixierelement 25 anordenbar bzw. angeordnet.

Vorzugsweise ist die Position des Anlageelements 26 mittels des Fixierelements 25 fixierbar bzw. festlegbar. Das Fixierelement 25 ist vorzugsweise auch an dem Anlageelement 26 befestigbar, beispielsweise durch Crimpen, Aufschrauben, Kleben, Verschweißen oder dergleichen. Dies ist in Fig. 3 beispielhaft angedeutet.

Beim Darstellungsbeispiel sind das Fixierelement 25 und das Anlageelement 26 durch zwei separate Bauteile gebildet.

Die zweiteilige Ausbildung von Fixierelement 25 und Anlageelement 26 hat zum Beispiel den Vorteil, dass das Fixierelement 25 und das Anlageelement 26 besser für die jeweiligen Anforderungen optimiert werden können. Beispielsweise ist für das Anlageelement 26 eine besonders gute Passform mit geringen Toleranzen in dem Aufnahmeraum 27 wichtig, um eine gute Abdichtung zuverlässig zu gewährleisten. Daher sollte das Anlageelement 26 möglichst geringe Fertigungstoleranzen aufweisen, die für das Fixierelement 25 weniger entscheidend sind. Das Fixierelement 25 kann hingegen zum Beispiel für eine stoff-, form- oder kraftschlüssige Verbindung mit dem Druckerzeuger 5 oder Führungselement 23 oder einem sonstigen Bauteil - insbesondere zur Festlegung der axialen Position - optimiert sein.

Des Weiteren können das Anlageelement 26 und das Fixierelement 25 bei einer Ausführung als separate Bauteile aus verschiedenen Materialien hergestellt werden. Beispielsweise kann das Anlageelement 26 aus einem formstabileren und/oder steiferen bzw. härteren Material und/oder das Fixierelement 25 aus einem weicheren bzw. duktileren Material als das jeweils andere Bauteil gefertigt sein. Ein "duktileres Material" ist dabei insbesondere ein Material mit einer größeren Duktilität, wobei der Begriff "Duktilität" die Eigenschaft eines Materials bezeichnet, sich unter Scherbelastung vor einem Bruch dauerhaft zu verformen, insbesondere plastisch zu verformen. Insgesamt wird die Flexibilität bei der Herstellung und/oder Verwendung von separaten Bauteilen erhöht und die Herstellungsverfahren können optimiert werden.

Ein weiterer Vorteil der Ausführung des Anlageelements 26 und des Fixierelements 25 als separate Bauteile besteht darin, dass durch die Wahl verschiedener Materialien für diese Bauteile die Anforderungen an die Bauteile besser erfüllt werden können. Ein härteres bzw. formstabileres Material für das Anlageelement 26 ist bevorzugt, weil hierdurch eine dauerhafte bzw. gleichbleibende Fixierung des Dichtelements 24 in dem Aufnahmeraum 27 erreicht werden kann und somit eine dauerhafte bzw. gleichbleibende Abdichtung durch das Dichtelement 24 gewährleistet ist. Ein weicheres bzw. duktileres Material für das Fixierelement 25 ist bevorzugt, weil hierdurch eine vorzugsweise umformende Befestigung des Fixierelements 25 an dem Führungselement 23, insbesondere durch Verpressen bzw. Crimpen, erleichtert oder ermöglicht wird. Insgesamt ist daher die Wahl verschiedener Materialien für das Anlageelement 26 und das Fixierelement 25 zur Erfüllung der Anforderungen vorteilhaft.

Es ist jedoch auch möglich, dass das Anlageelement 26 einstückig mit dem Fixierelement 25 oder umgekehrt ausgebildet ist und/oder das Anlageelement 26 einen Abschnitt des Fixierelements 25 darstellt oder bildet. Die einstückige Ausbildung führt zu weniger Bauteilen, ist aber nur optional.

Das Führungselement 23 ist vorzugsweise als Führungsrohr ausgebildet und/oder bildet vorzugsweise einen insbesondere länglichen und/oder geraden Kanal 23b zur Aufnahme bzw. längs verschiebbaren Führung des Förderelements 9. Das Förderelement 9 ist vorzugsweise in dem Führungselement 23 anordenbar bzw. bei dem fertig montierten Druckerzeuger 5 bzw. Abgabevorrichtung 1 in dem Führungselement 23 angeordnet bzw. geführt. Vorzugsweise ist das Förderelement 9 relativ zu dem Führungselement 23 bewegbar, insbesondere längs bzw. axial verschiebbar in dem Führungselement 23 bzw. dem Kanal 23b geführt.

Vorzugsweise bildet das Förderelement 9 einen Kolben und/oder bildet das Führungselement 23 einen Zylinder bzw. Hohlzylinder. Das Führungselement 23 und das Förderelement 9 bilden vorzugsweise zusammen eine Zylinder-Kolben-Anordnung, insbesondere eine Kolbenpumpe oder einen Teil davon.

Das Förderelement 9 begrenzt vorzugsweise einen Pumpraum bzw. die Druckkammer 11 im Führungselement 23, wie in Fig. 3 angedeutet. Das Förderelement 9 ist vorzugsweise mit dem Rückschlagventil 10 versehen, das insbesondere an dem der Druckkammer 11 zugewandten Ende des Förderelements 9 angeordnet ist.

Das vorzugsweise hohle Förderelement 9 bildet beim Darstellungsbeispiel einen Zuführkanal 28 für das Arzneimittel 2 oder weist den Zuführkanal 28 auf. Das Arzneimittel 2 ist bei entsprechender axialer Bewegung durch den Zuführkanal 28 über das Einlass- bzw. Rückschlagventil 10 in die Druckkammer 11 förderbar, insbesondere saugbar.

Druck- bzw. ausgabeseitig weist der Druckerzeuger 5 optional ein nicht dargestelltes Auslassventil und insbesondere die Austragsdüse 12 zur Ausgabe und gegebenenfalls Zerstäubung des Arzneimittels 2 auf.

Der vorschlagsgemäße Druckerzeuger 5 bzw. die vorschlagsgemäße Abgabevorrichtung 1 ist beim Darstellungsbeispiel insbesondere als Zerstäuber oder Inhalator ausgebildet. Das Arzneimittel 2 wird vom Förderelement 9 bei entsprechender axialer Hin- und Her-Bewegung abwechselnd durch den Zuführkanal 28 in die Drucckammer 11 gesaugt oder dort unter Druck gesetzt und über die Austragsdüse 12 ausgegeben und dabei appliziert, vorzugsweise zerstäubt, also aus dem Arzneimittel 2 ein Sprühnebel bzw. Aerosol 14 gebildet, wie in Fig. 3 angedeutet.

In dem in Fig. 1 dargestellten nicht auslösebereiten Zustand ist die Druckkammer 11 gegenüber dem in Fig. 2 dargestellten auslösebereiten bzw. geladenen Zustand vorzugsweise verkleinert bzw. minimiert. Mit anderen Worten taucht im nicht auslösebereiten Zustand das Förderelement 9 bzw. der Kolben vorzugsweise in die Druckkammer 11 ein. Im auslösebereiten bzw. geladenen Zustand ist das Förderelement 9 bzw. der Kolben vorzugsweise weiter von der Austragsdüse 12 beabstandet und/oder aus der Druckkammer 11 zurückgezogen als im in Fig. 1 dargestellten nicht auslösebereiten Zustand.

Die Abgabevorrichtung 1 bzw. der Druckerzeuger 5 bzw. das Führungselement 23 weist vorzugsweise den Aufnahmeraum 27 für das Dichtelement 24 auf bzw. bildet oder begrenzt diesen, insbesondere zusammen mit dem Anlageelement 26.

Es ist bevorzugt, dass das Führungselement 23 den Aufnahmeraum 27 (zumindest teilweise) aufweist oder bildet bzw. zumindest partiell begrenzt, insbesondere außenseitig und/oder an einer, vorzugsweise der abgabeseitigen bzw. der der Drucckammer 11 und/oder Austragsdüse 12 zugewandten, Axial- bzw. Stirnseite.

Bedarfsweise kann der Aufnahmeraum 27 auch separat vom Führungselement 23 ausgebildet sein, vorzugsweise wobei der Aufnahmeraum 27 bzw. das den Aufnahmeraum 27 aufweisende oder bildende Bauteil in diesem Fall am Führungselement 23 anliegt bzw. dieses umgibt.

Der Aufnahmeraum 27 ist insbesondere als Ausnehmung in dem Führungselement 23, besonders bevorzugt als Nut bzw. Ringnut, Ringschulter oder als Buchse, ausgeführt.

Bevorzugt wird das Volumen des Aufnahmeraums 27 maßgeblich durch die radiale Erstreckung bzw. Breite B des Aufnahmeraums 27 im Führungselement 23 und die axiale Höhe H des Aufnahmeraums 27 bestimmt.

Vorzugsweise ist der Aufnahmeraum 27 - zumindest teilweise - durch das Führungselement 23 und das Anlageelement 26 bzw. Fixierelement 25 begrenzt. Das Führungselement 23 kann eine zylindrische bzw. hohlzylindrische Führung oder Aufnahme für das Anlageelement 26 bzw. Fixierelement 25 bilden.

Vorzugsweise umgibt der Aufnahmeraum 27 das Förderelement 9 radial und/oder ringförmig.

Das Förderelement 9 bildet vorzugsweise eine innere radiale Begrenzung des Aufnahmeraums 27.

Vorzugsweise bildet das Führungselement 23 eine axiale und/oder radiale Außenbegrenzung für den Aufnahmeraum 27.

Beim Darstellungsbeispiel weist das Förderelement 9 einen kreisförmigen Querschnitt mit einem Durchmesser von mehr als 0,25 mm, vorzugsweise mehr als 0,5 mm, insbesondere mehr als 0,75 mm und/oder weniger als 4 mm, vorzugsweise weniger als 3 mm, insbesondere weniger als 2,25 mm, auf.

Das Förderelement 9 besteht vorzugsweise aus Metall, insbesondere Edelstahl. Es ist insbesondere als Hohlelement bzw. Kapillare ausgebildet.

Das Förderelement 9 ist vorzugsweise gezogen und weist dementsprechend eine relativ geringe Toleranz hinsichtlich seines Durchmessers auf.

Das Dichtelement 24 ist vorzugsweise - zumindest teilweise - verformbar. Das Maß und/oder die Art einer Verformung des Dichtelements 24 wird vorzugsweise durch einen Verformungswert angegeben.

Das Dichtelement 24 ist vorzugsweise durchgehend ringförmig bzw. als Formdichtung oder Dichtring, insbesondere angepasst zur Aufnahme in dem Aufnahmeraum 27, ausgebildet.

Das Dichtelement 24 weist vorzugsweise eine, insbesondere zentrale, Öffnung 24a auf.

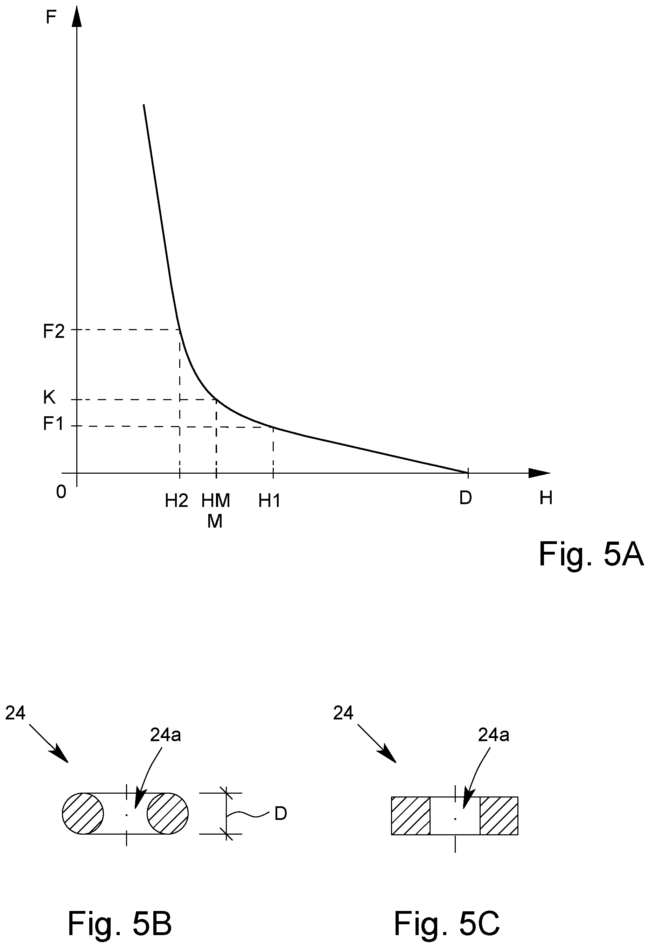

Insbesondere handelt es sich bei dem Dichtelement 24 um einen O-Ring mit im nicht eingebauten bzw. nicht verformten Zustand zumindest im Wesentlichen kreisförmigem Querschnitt, wie in Fig. 5B dargestellt. Jedoch kann das Dichtelement 24 auch als sonstige Formdichtung mit insbesondere nicht kreisrundem Querschnitt im nicht verformten Zustand ausgebildet sein.

Das Dichtelement 24 hat im unverformten Zustand vorzugsweise einen anderen Querschnitt bzw. ein anderes Volumen als der Aufnahmeraum 27 der (fertig montierten) Abgabevorrichtung 1. Vorzugsweise wird das Dichtelement 24 daher bei der Montage der Abgabevorrichtung 1, insbesondere bei der Befestigung des Anlageelements 26 bzw. Fixierelements 25 an dem Führungselement 23, verformt. Durch die Verformung des Dichtelements 24 wird eine elastische Vorspannung des Dichtelements 24 erzeugt, die einer optimalen Dichtwirkung des Dichtelements 24 zuträglich und/oder durch die eine Abdichtung mittels des Dichtelements 24 erreicht werden kann bzw. wird.

Die Verformung bzw. der Verformungswert des Dichtelements 24 ist vorzugsweise nicht direkt bestimmbar bzw. einstellbar, sondern wird insbesondere mittels eines dazu korrelierenden Kennwerts bestimmt oder aus einem Kennwert abgeleitet. Durch die Verwendung eines Montageparameters M kann insbesondere sichergestellt werden, dass in der fertig montierten Abgabevorrichtung 1 die gewünschte Verformung des Dichtelements 24 erreicht wird bzw. der Verformungswert des Dichtelements 24 den gewünschten Wert annimmt.

Die Dichtelemente 24 werden vorzugsweise in Chargen - also gruppenweise - hergestellt. Insbesondere wird eine Charge aus einer bestimmten Menge möglichst homogenen Ausgangsmaterials hergestellt. Vorzugsweise weisen die Dichtelemente 24 einer Charge hinsichtlich ihrer signifikanten Größen, wie Ringdurchmesser bzw. wirksamer Ringdurchmesser, Querschnitt, Volumen, Kompressibilität oder dergleichen, nur eine geringe Varianz bzw. eine hohe Reproduzierbarkeit auf.

Besonders bevorzugt weichen die Volumina der Dichtelemente 24 einer Charge um weniger als 10 %, insbesondere weniger als 5 %, besonders bevorzugt weniger als 4 % oder 2 %, vom mittleren Volumen bzw. Soll-Volumen der Dichtelemente 24 der Charge ab.

In einem konkreten Beispiel haben die Dichtelemente 24 ein Soll-Volumen bzw. mittleres Volumen von mehr als 5 mm³ und/oder weniger als 10 mm³, besonders bevorzugt etwa 7 mm³ bis 8 mm³, wobei alle Dichtelemente 24 der Charge ein Volumen aufweisen, das um höchstens 0,3 mm³ von diesem Soll-Volumen bzw. mittleren Volumen abweicht.

Die Dichtelemente 24 werden vorzugsweise durch Spritzgießen, insbesondere mittels eines nicht dargestellten Spritzgusswerkzeuges mit einer Vielzahl von Kavitäten, hergestellt. Entsprechend werden bei jedem Spritzgussvorgang eine Vielzahl von Dichtelementen 24 erzeugt.

Bevorzugt ist das Dichtelement 24 ein Einzelteil bzw. separates Bauteil, insbesondere in Form eines O-Rings oder Dichtrings. Alternativ kann das Dichtelement 24 jedoch auch, insbesondere mittels zwei Komponenten-Spritzguss, direkt an dem Führungselement 23 oder Anlageelement 26 angeformt sein. In diesem Fall würden dann entsprechend die Chargen aus Führungselementen 23 bzw. Anlageelementen 26 mit darin bzw. daran angeformten Dichtelementen 24 bestehen, anstatt, wie oben erläutert, lediglich aus Dichtelementen 24.

Die Dichtelemente 24 können von Charge zu Charge variieren, insbesondere hinsichtlich signifikanter Größen, wie Ringdurchmesser bzw. wirksamer Ringdurchmessers, Querschnitt, Volumen, Kompressibilität o. dgl. Neben den werkzeuggebundenen Maßen (Ringdurchmesser, Dicke, Volumen, Oberflächenbeschaffenheit) können auch materialbedingte oder verfahrenstechnisch bedingte Größen, wie die Kompressibilität, variieren.

Die Dichtelemente 24 verschiedener Chargen unterscheiden sich vorzugsweise lediglich durch Fertigungstoleranzen, die durch die Produktion in verschiedenen Chargen entstehen.

Es ist jedoch auch möglich, dass die Dichtelemente verschiedener Chargen sich deutlicher voneinander unterscheiden, insbesondere grundsätzlich andere (Soll-)Abmessungen aufweisen, aus unterschiedlichen Materialien bestehen, unterschiedliche Verformungseigenschaften - beispielsweise unterschiedliche Kompressionsmoduln, Elastizitätsmoduln, Stauchhärten und/oder Eindrückhärten - und/oder unterschiedliche Druckverformungsreste aufweisen.

Das Dichtelement 24 ist vorzugsweise zur - insbesondere gasdichten bzw. diffusionsdichten - Abdichtung des Förderelements 9 gegenüber dem Führungselement 23 ausgebildet. Insbesondere ist das Dichtelement 24 derart ausgebildet bzw. angeordnet, dass kein Arzneimittel 2 bzw. Fluid, insbesondere kein Gas und/oder keine Flüssigkeit, aus der Druckkammer 11 und/oder zwischen dem Führungselement 23 und dem Förderelement 9 entweichen kann.

Der Querschnitt bzw. die (wirksame) Schnurstärke D des nicht eingebauten Dichtelements 24 beträgt beim Darstellungsbeispiel vorzugsweise mehr als 0,3 mm, insbesondere mehr als 0,5 mm, besonders bevorzugt 1 mm oder mehr, und/oder weniger als 3 mm, insbesondere weniger als 2 mm, besonders bevorzugt weniger als 1,5 mm. Der Innendurchmesser bzw. die Größe der zentralen Öffnung 24a des Dichtelements 24 entspricht vorzugsweise etwa dem äußeren Durchmesser des Förderelements 9.

Bevorzugt besteht das Dichtelement 24 aus einem elastischen Material. Ein elastisches Material im Sinne der Erfindung ist vorzugsweise ein Material mit einem Elastizitätsmodul von weniger als 100 MPa, vorzugsweise als 50 MPa, insbesondere weniger als 10 MPa.

Das Dichtelement 24 besteht vorzugsweise aus einem für Pharmazeutika oder Lebensmittel geeigneten, gummielastischen Material oder Kautschuk. Vorzugsweise besteht das Dichtelement 24 aus Silikon, Fluorkautschuk (FKM), einem thermoplastischen Elastomer (TPE), Ethylen-Propylen-Dien-Kautschuk (EPDM), Chlor-Isobuten-Isopren-Kautschuk bzw. Chlorbutyl-Kautschuk (CIIR), Brombutylkautschuk (BIIR), Polyurethan (PUR) und/oder Nitrilkautschuk (NBR).

Unterschiedliche Materialien, die für das Dichtelement 24 in Frage kommen, weisen unterschiedliche Eigenschaften auf. Einerseits gibt es Materialien, die bei Kontakt mit einem Pharmazeutikum, bei erhöhter Temperatur und/oder bei erhöhtem Druck eine erhöhte Leckrate und/oder eine verringerte Diffusionsdichtigkeit aufweisen. Mit anderen Worten ist es also möglich, dass bei Kontakt mit einem Pharmazeutikum oder bei erhöhter Temperatur oder erhöhtem Druck die Dichtwirkung durch das Dichtelement 24 dadurch verringert wird, dass eine erhöhte Diffusion durch das Dichtelement 24 auftritt.

Bei einem aus einem solchen Material bestehenden Dichtelement 24 ist eine statische Abdichtung bzw. gute Lagerdichtigkeit dadurch erschwert, dass Stoffe durch das Dichtungsmaterial bzw. Dichtelement 24 hindurch diffundieren können. Der Diffusionsverlust ist hierbei vorzugsweise insbesondere proportional zu der Größe der freien Oberfläche und/oder antiproportional zur Erstreckung des Dichtelements 24 in Richtung der Diffusion.

Des Weiteren gibt es Materialien, bei denen sich bei einer erhöhten Temperatur und/oder einem erhöhten Druck die Kontaktspannung an den Dichtflächen des Dichtelements 24 abbaut, insbesondere also den Flächen, die mit der Begrenzung des Aufnahmeraums 27 in Kontakt stehen. Mit anderen Worten wird bei diesen Materialien unter erhöhtem Druck und/oder erhöhter Temperatur ein Kriechen des Materials bzw. Dichtelements 24 beobachtet.

In diesem Fall entsteht eine Lagerundichtigkeit bzw. schlechte statische Abdichtung insbesondere dadurch, dass bei einer zu geringen Druckspannung des Dichtungsmaterials bzw. Dichtelements 24 auf die Gegenfläche, im vorliegenden Fall insbesondere die Fläche des Förderelements 9, die Rauigkeit bzw. Mikroporosität der Oberfläche zu einer Undichtigkeit führt.

Die Problematik bei der Abdichtung des Förderelements 9 durch das Dichtelement 24 besteht daher insbesondere darin, zwei gewissermaßen miteinander konkurrierende Anforderungen zu erfüllen: Einerseits ist ein großer Vorspanndruck bzw. eine starke Verformung des Dichtelements 24 in dem Aufnahmeraum 27 erwünscht bzw. dahingehend von Vorteil, dass hierdurch eine Lagerundichtigkeit vermieden bzw. die statische Abdichtung verbessert werden kann. Andererseits führt aber ein hoher Vorspanndruck bzw. eine starke Verformung des Dichtelements 24 dazu, dass sich die dynamische Abdichtung, also die Abdichtung des Förderelements 9 während einer Bewegung des Förderelements 9 relativ zu dem Dichtelement 24, verschlechtert, da sich mit einem erhöhten Vorspanndruck das Kriechen bzw. Fließen des Materials verstärkt und auch der Abrieb durch die Relativbewegung zwischen dem Förderelement 9 und dem Dichtelement 24 erhöht. Zudem führt ein zu hoher Vorspanndruck nicht nur zu einem erhöhten Abrieb, sondern auch zu einer erschwerten Bedienung bzw. Bewegung des Förderelements 9. Dies kann dazu führen, dass eine Bewegung des Förderelement 9 nicht oder nicht wie vorgesehen erfolgt, sodass z. B. eine zu geringe Dosis des Arzneimittels 2 bei einem Förderhub ausgegeben wird und/oder eine unzureichende Zerstäubung erfolgt.

Insbesondere ist der Verformungswert ein Maß dafür, wie stark das Dichtelement 24 verformt ist bzw. wie das Verhältnis zwischen dem Querschnitt bzw. Volumen des (verformten) Dichtelements 24 und dem Querschnitt bzw. Volumen des Aufnahmeraums 27 in der fertig montierten Abgabevorrichtung 1 ist. Der Verformungswert ist daher insbesondere ein Maß dafür, wie stark die elastische Vorspannung des Dichtelements 24 in dem Aufnahmeraum 27 ist.

Das Dichtelement 24 sollte insbesondere derart verformt werden bzw. der Verformungswert des Dichtelements 24 sollte so gewählt oder eingestellt werden, dass die erläuterten konkurrierenden Anforderungen möglichst optimal erfüllt werden, also einerseits die Verformung ausreichend groß ist, dass eine elastische Vorspannung in dem Dichtelement 24 herrscht, durch die eine Lagerundichtigkeit vermieden werden kann, und andererseits die Verformung ausreichend klein ist, dass eine gute dynamische Abdichtung gewährleistet ist bzw. ein Fließen und/oder Kriechen des Materials verhindert wird.

Durch das vorgeschlagene Verfahren zur Montage des Dichtelements 24 und die vorgeschlagene Abgabevorrichtung 1 soll insbesondere sichergestellt werden, dass das Dichtelement 24 derart in dem Aufnahmeraum 27 angeordnet wird bzw. mit einem solchen Vorspanndruck in dem Aufnahmeraum 27 fixiert wird, dass sowohl eine gute statische Abdichtung als auch eine dynamische Abdichtung realisiert bzw. erreicht werden. Des Weiteren ist das vorschlagsgemäße Verfahren vorzugsweise für eine Vielzahl von Materialien bzw. unterschiedliche Materialien geeignet.

Im eingebauten Zustand - also bei montiertem Druckerzeuger 5 - ist das Dichtelement 24 zumindest im Wesentlichen in dem Aufnahmeraum 27 aufgenommen, wie in Fig. 3 dargestellt. Das Anlageelement 26 bzw. Fixierelement 25 liegt vorzugsweise axial an dem Dichtelement 24 an und fixiert das Dichtelement 24 axial in dem Aufnahmeraum 27. Weiter liegt das Dichtelement 24 vorzugsweise radial an dem das Dichtelement 24 durchgreifenden Förderelement 9 dichtend an. Das Dichtelement 24 ist insbesondere in den Aufnahmeraum 27 eingespannt bzw. zusammengedrückt, also im eingebauten Zustand verformt. Das Dichtelement 24 weist im eingebauten Zustand im Querschnitt vorzugsweise eine im Wesentlichen rechteckige Form oder zumindest eine flache Anlageseite zum Förderelement 9 hin auf.

Um eine gute Abdichtung und dementsprechend genaue Dosierung erreichen zu können, beträgt der gewünschte Füllgrad, also der "Soll-Füllgrad", im Mittel vorzugsweise mehr als 90 %, insbesondere mehr als 95 %, und/oder weniger als 110 %, insbesondere weniger als 105 %, besonders bevorzugt zumindest im Wesentlichen 100 %, insbesondere mit einer Toleranz von 4 %, 2 % oder weniger.

Der "Füllgrad" entspricht dem Quotienten aus dem Volumen des unverformten Dichtelements 24 (wie insbesondere in Fig. 5B dargestellt) durch das Volumen des Aufnahmeraums 27. Das erfindungsgemäße Verfahren und die vorschlagsgemäße Abgabevorrichtung 1 sind vorzugsweise dazu ausgebildet, diesen oder einen sonstigen gewünschten Füllgrad zu realisieren bzw. zu reproduzieren.

Das Volumen des Aufnahmeraums 27 ist vorzugsweise variabel und/oder durch bzw. bei der Montage einstellbar bzw. festlegbar.

Beim Darstellungsbeispiel ist das Anlageelement 26 insbesondere in seiner axial gegen das Dichtelement 24 gespannten Position vorzugsweise durch das Fixierelement 25 am Führungselement 23 festgesetzt bzw. befestigt. Durch entsprechende axiale bzw. stirnseitige Anlageflächen kann eine definierte Lage des Anlageelements 26 und damit eine definierte axiale Länge bzw. Höhe H des Aufnahmeraums 27 für das Dichtelement 24 erreicht werden.

Die Höhe H des Aufnahmeraums 27 ist insbesondere der (axiale) Abstand zwischen einem Boden 27a bzw. der von dem Führungselement 23 gebildeten Ringfläche oder -schulter des Aufnahmeraums 27 und der dem Boden 27a bzw. Dichtelement 24 zugewandten Stirnseite des Anlageelements 26 bzw. der Stirnseite eines Kontaktabschnitts 26a des Anlageelements 26, wie in Fig. 3 angedeutet.

Vorzugsweise ist das Fixierelement 25 kappenartig ausgebildet und/oder übergreift es das Anlageelement 26 an einer Stirnseite oder am freien Ende.

Das Anlageelement 26 ist vorzugsweise zumindest im Wesentlichen ringförmig ausgebildet. Vorzugsweise weist das Anlageelement eine zentrale Öffnung auf. Bevorzugt entspricht der Durchmesser der zentralen Öffnung dem Durchmesser des Kanals 23b oder ist der Durchmesser der zentralen Öffnung größer als der Durchmesser des Kanals 23b und/oder des Förderelements 9.

Das Anlageelement 26 weist vorzugsweise einen Kontaktabschnitt 26a auf, der zur direkten Anlage an dem Dichtelement 24 ausgebildet ist. Der Kontaktabschnitt 26a ist insbesondere durch einen axial endseitigen Abschnitt des Anlageelements 26 gebildet. In der fertig montierten Abgabevorrichtung 1 kontaktiert der Kontaktabschnitt 26a vorzugsweise das Dichtelement 24 bzw. fixiert der Kontaktabschnitt 26a das Dichtelement 24 in dem Aufnahmeraum 27 oder spannt der Kontaktabschnitt 26a das Dichtelement 24a in dem Aufnahmeraum 27 ein.

Die dem Dichtelement 24 zugewandte Stirnfläche des Kontaktabschnitts 26a ist vorzugsweise in radialer Richtung gewölbt und/oder an die Form des Dichtrings bzw. Dichtelements 24 angepasst. Hierdurch kann insbesondere der Totraum, insbesondere also der nicht durch das Dichtelement 24 ausgefüllte Bereich des Aufnahmeraums 27, verkleinert bzw. minimiert werden. Vorzugsweise weist der Kontaktabschnitt 26a eine dem Dichtelement 24 zugewandte Dichtlippe auf. Die Dichtlippe dient vorzugsweise als Extrusionssperre und/oder ist dazu ausgebildet, einem Kriechen des Dichtelements 24 in den Kanal 23b entgegenzuwirken.

Der Kontaktabschnitt 26a ist im Querschnitt (also im Schnitt senkrecht zur Achse A) vorzugsweise ringförmig, insbesondere kreisringförmig, ausgebildet.

Das Anlageelement 26 weist vorzugsweise eine Anlagefläche bzw. einen Anschlag 26b für das Fixierelement 25 auf. Insbesondere ist der Anschlag 26b durch eine Ringschulter bzw. einen Flansch des Fixierelements 25 gebildet.

Das Fixierelement 25 weist vorzugsweise eine dem Anschlag 26b zugeordnete Gegenfläche 25a auf, um den Anschlag 26b zu kontaktieren bzw. axial den Anschlag 26b und damit das Anlageelement 26 zu halten bzw. zu fixieren.

Das Anlageelement 26 weist vorzugsweise einen insbesondere konischen Einführabschnitt 26c auf, durch den das Einführen des Förderelements 9 in das Anlageelement 26 erleichtert wird. Alternativ oder zusätzlich wird durch den Einführabschnitt 26c auch das Einführen eines Stößels 29 bzw. eines Zentralelements 30 während einer Bestimmung eines Montageparameters M erleichtert. Der Einführabschnitt 26c weist vorzugsweise eine Abschrägung und/oder Abrundung auf oder ist hierdurch gebildet. Insbesondere ist eine zentrale Öffnung des Anlageelements 26 durch den Einführabschnitt 26c bzw. die Abschrägung/Abrundung zum freien Ende des Anlageelements 26 hin radial aufgeweitet bzw. vergrößert.

Vorzugsweise ist die, insbesondere axiale, Position des Fixierelements 25 variierbar. Das Fixierelement 25 ist vorzugsweise in verschiedenen, insbesondere axialen, Positionen relativ zu dem Führungselement 23 an dem Führungselement 23 befestigbar. Hierzu weist das Führungselement 23 vorzugsweise einen Befestigungsabschnitt 23a auf. Der Befestigungsabschnitt 23a ist vorzugsweise an einer Umfangsseite des Führungselements 23 angeordnet bzw. gebildet.

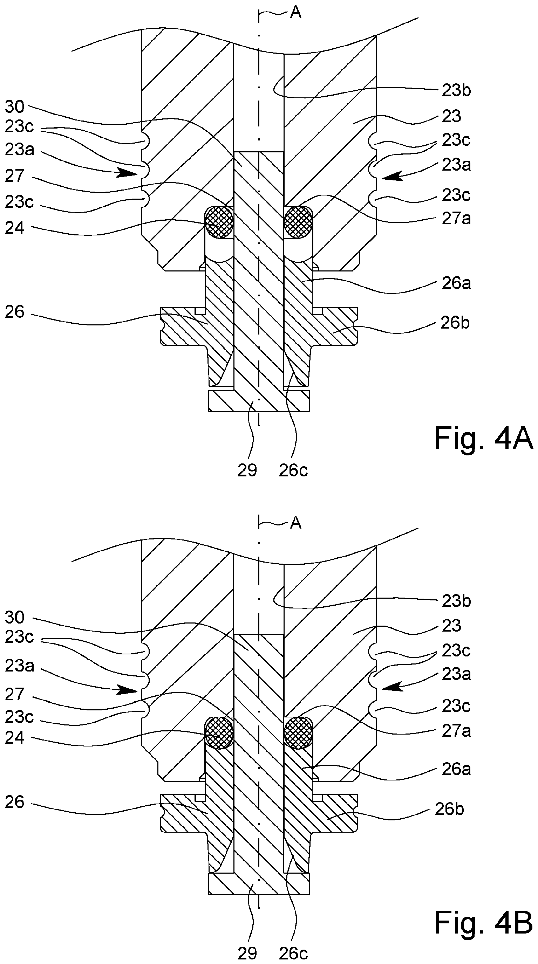

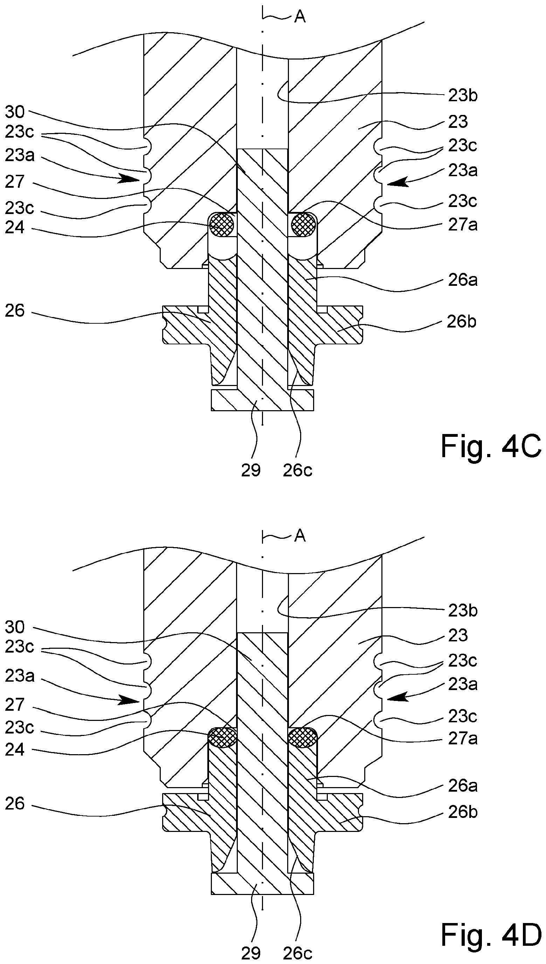

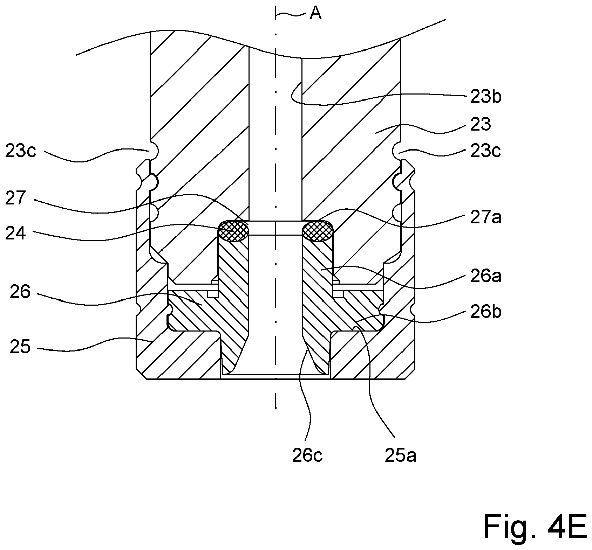

Im Darstellungsbeispiel gemäß Fig. 4 weist der Befestigungsabschnitt 23a vorzugsweise eine oder mehrere Eingriffsmöglichkeiten, wie Ausnehmungen 23c oder ein Gewinde, auf. Der Befestigungsabschnitt 23a kann jedoch auch anders ausgebildet sein, beispielsweise eine Klebefläche aufweisen oder dadurch gebildet sein.

Vorzugsweise ist das Fixierelement 25 bzw. Anlageelement 26 in verschiedenen diskreten Positionen relativ zu dem Führungselement 23 an dem Führungselement 23 befestigbar. Eine "diskrete Position" im Sinne der vorliegenden Erfindung ist insbesondere eine Position, die durch die geometrische Form des Fixierelements 25, des Anlageelements 26 und/oder des Führungselements 23 bzw. Befestigungsabschnitts 23a definiert bzw. festgelegt ist.

Das Fixierelement 25 ist beispielsweise durch Aufschrauben oder Verkleben wie in Fig. 3 angedeutet oder beispielsweise durch Verformen, insbesondere unter Bildung einer oder mehrerer Sicken, und/oder durch insbesondere radialen Eingriff vorzugsweise in eine oder mehrere Ausnehmungen 23c des Führungselements 23, wie in Fig. 4E angedeutet, an dem Führungselement 23 befestigbar. Insbesondere kann das Führungselement 23 bzw. der Befestigungsabschnitt 23a mehrere Ausnehmungen 23c aufweisen, die an unterschiedlichen axialen Positionen angeordnet sind.

Insbesondere werden durch die mehreren Ausnehmungen 23c mehrere diskrete Positionen des Fixierelements 25 bzw. Anlageelements 26 relativ zu dem Führungselement 23 festgelegt bzw. realisiert. Mit anderen Worten ist vorzugsweise durch die Ausnehmungen 23c das Fixierelement 25 bzw. Anlageelement 26 in verschiedenen diskreten Positionen relativ zu dem Führungselement 23 an dem Führungselement 23 befestigbar.

Insbesondere ist also die axiale Lage des Fixierelements 25 bzw. Anlageelements 26 und damit die Höhe H und so letztendlich das Volumen des Aufnahmeraums 27 einstellbar bzw. variierbar.

Mit anderen Worten ist es bevorzugt, dass in der fertig montierten Abgabevorrichtung 1 das Fixierelement 25 in einer festen bzw. definierten Position an dem Führungselement 23 bzw. dem Befestigungsabschnitt 23a befestigt ist bzw. das Fixierelement 25 und/oder Anlageelement 26 in einer festen bzw. nicht veränderbaren Position relativ zu dem Führungselement 23 festgesetzt bzw. fixiert sind, wobei aber bei der Festsetzung bzw. Befestigung des Fixierelements 25 und/oder Anlageelements 26 verschiedene, insbesondere axiale und/oder diskrete, Positionen relativ zu dem Führungselement 23 wählbar bzw. realisierbar sind. Insbesondere kann hierdurch das Volumen des Aufnahmeraums 27 bzw. das Verhältnis zwischen dem Volumen des Aufnahmeraums 27 und dem Volumen des Dichtelements 24 ausgewählt bzw. an das Dichtelement 24 angepasst werden. Die Variierbarkeit der Position des Fixierelements 25 bzw. Anlageelements 26 bei der Montage bildet insbesondere einen unabhängig realisierbaren Aspekt der vorliegenden Erfindung.

Insbesondere wird durch die Position des Fixierelements 25 und/oder Anlageelementes 26 eine Verformung des Dichtelements 24 und/oder eine Größe bzw. ein Volumen des Aufnahmeraums 27 definiert, so dass die Verformung des Dichtelements 24 und/oder die Größe bzw. das Volumen des Aufnahmeraums 27 durch eine Variation der Position des Fixierelements 25 und/oder Anlageelements 26 variierbar sind.

Es bestehen vorzugsweise verschiedene Möglichkeiten zur Befestigung des Fixierelements 25 an dem Führungselement 23. Das Fixierelement 25 kann kraftschlüssig, formschlüssig und/oder stoffschlüssig an dem Führungselement 23 befestigt sein bzw. werden.

Bevorzugte Möglichkeiten zur Befestigung des Fixierelements 25 sind ein Festschrauben des Fixierelements 25, eine (formschlüssige) Befestigung durch Stifte, eine urformende Befestigung (beispielsweise Gießen oder eine stoffschlüssige Befestigung durch aushärtendes Harz, insbesondere durch UV-härtbares Harz, und/oder einen Klebstoff), eine umformende Befestigung (beispielsweise Crimpen oder Heißverstemmen), eine spanende Befestigung, ein Schweißen, ein Löten oder dergleichen.

Gemäß einem Beispiel kann das Fixierelement 25 als Mutter bzw. Überwurfmutter ausgebildet sein und durch Festschrauben, insbesondere mit einem definierten Drehmoment, in der durch den Montageparameter M vorgegebenen Position an dem Führungselement 23 befestigt werden. Bevorzugt sind die hierbei verwendeten Gewinde als Steilgewinde und/oder nicht selbsthemmend ausgebildet. Dies ist beispielhaft in Fig. 3 angedeutet.

Gemäß einem weiteren Beispiel kann das Fixierelement 25 als Crimphülse ausgebildet sein und in der durch den Montageparameter M vorgegebenen Position an dem Führungselement 23 bzw. dem Befestigungsabschnitt 23a festgecrimpt werden.

Hierzu kann das Führungselement 23 eine oder mehrere Ausnehmungen 23c aufweisen oder hierdurch gebildet sein. Die Ausnehmungen 23c sind vorzugsweise außenseitig an dem Führungselement 23 angeordnet. Insbesondere korrespondieren die Ausnehmungen 23c zu verschiedenen (axialen) und/oder diskreten Positionen des Fixierelements 25 bzw. legen die Ausnehmungen 23c verschiedene axiale und/oder diskrete Positionen des Fixierelements 25 bzw. Anlageelements 26 fest. Dies ist beispielhaft in Fig. 4A-E angedeutet.

Die Ausnehmungen 23c sind vorzugsweise als Nuten bzw. Sicken ausgebildet. Die Ausnehmungen 23c, insbesondere Nuten bzw. Sicken, können kontinuierlich bzw. das Führungselement 23 vollständig umlaufend ausgebildet sein oder in Umfangsrichtung durch mehrere voneinander getrennte Ausnehmungen 23c gebildet sein.

Bei der in Fig. 4A-E dargestellten Ausführungsform mit mehreren Ausnehmungen 23c ist das Fixierelement 25 vorzugsweise in verschiedenen diskreten Positionen, die insbesondere einzelnen Ausnehmungen 23c entsprechen, an dem Führungselement 23 befestigbar bzw. fixierbar.

Alternativ oder zusätzlich kann nur eine bzw. genau eine derartige Ausnehmung 23c bzw. Nut bzw. Sicke vorgesehen sein, wobei mehrere Crimphülsen mit verschiedenen axialen Längen vorgesehen sind, sodass unterschiedliche Montageparameter M zu verschiedenen Fixierelementen 25 bzw. Crimphülsen mit unterschiedlichen axialen Längen korrespondieren bzw. durch unterschiedliche Fixierelemente 25 realisiert werden.

Das Fixierelement 25 kann zum Eingreifen in eine Ausnehmung 23c bereits vorgeformt sein, beispielsweise dadurch, dass das Fixierelement 25 einen umlaufenden oder anderweitig zu der Ausnehmung 23c korrespondierend ausgebildeten Rand zum Eingreifen in die Ausnehmung 23c aufweist. Dies ist jedoch nicht zwingend erforderlich. Auch durch eine solche Vorformung des Fixierelements 25 in Kombination mit den Ausnehmungen 23c wird eine diskrete Position des Fixierelements 25 bzw. Anlageelements 26 festgelegt. Es ist auch möglich, dass das Fixierelement 25 keine derartige Vorformung aufweist, sondern lediglich das Führungselement 23 bzw. der Befestigungsabschnitt 23a eine oder mehrere Ausnehmungen 23c aufweist, wobei das Fixierelement 25 kontinuierlich relativ zu dem Führungselement 23 bzw. Befestigungsabschnitt 23a verschiebbar ist und zum Befestigen bzw. Fixieren des Fixierelements 25 ein Verformungswerkzeug das Fixierelement 25 in die Ausnehmung 23c hineinverformt.

Gemäß einem weiteren Beispiel kann das Fixierelement 25 in der durch den Montageparameter M vorgegebenen Position an dem Führungselement 23 festgeklebt oder festgeschweißt werden oder sein, insbesondere durch Laserschweißen, Ultraschallschweißen und/oder Reibschweißen.

Optional kann das Fixierelement 25 am Anlageelement 26 befestigt werden, wobei die Befestigung des Fixierelements 25 an dem Anlageelement 26 auf die gleiche Weise erfolgen kann wie die Befestigung des Fixierelements 25 an dem Führungselement 23. Die vorstehenden Ausführungen zur Befestigung des Fixierelements 25 an dem Führungselement 23 gelten daher analog für die Befestigung des Fixierelements 25 an dem Anlageelement 26. Bevorzugt wird dabei eine feste Verbindung zwischen Anlageelement 26 und Fixierelement 25 erst nach Positionierung des Anlageelements 26 hergestellt.

Bei dem in Fig. 4E dargestellten Ausführungsbeispiel weist das Anlageelement 26 bzw. dessen Anschlag 26b radial außen eine Nut bzw. Sicke auf, wobei das Fixierelement 25 durch Crimpen in diese Nut bzw. Sicke hinein verformt bzw. an dem Anlageelement 26 befestigt wird.

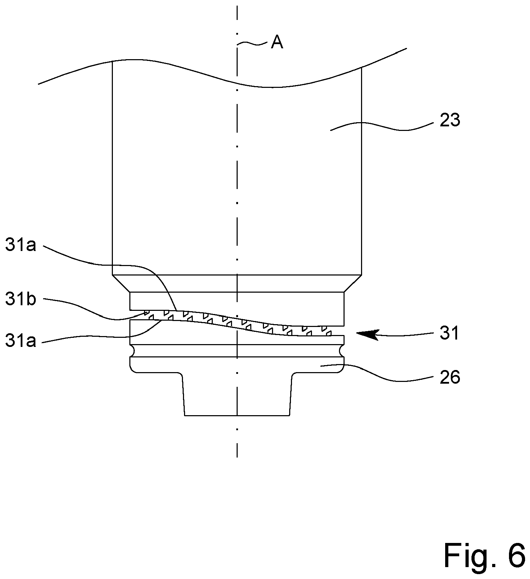

In Fig. 6 ist ein Teil der Abgabevorrichtung 1 bzw. des Druckerzeugers 5 gemäß einem weiteren bevorzugten Ausführungsbeispiel schematisch dargestellt. Die Fig. 6 zeigt eine Seitenansicht des in Fig. 4E in einer Schnittansicht dargestellten Druckerzeugers 5, wobei das Fixierelement 25 weggelassen ist und lediglich das Anlageelement 26 und das Führungselement 23 dargestellt sind.

Gemäß der in Fig. 6 dargestellten Ausführungsform ist das Fixier- bzw. Anlageelement 25, 26 vorzugsweise in verschiedenen axialen und/oder diskreten Positionen relativ zu dem Führungselement 23 an dem Führungselement 23 einrastbar. Insbesondere sind hierdurch verschiedene diskrete Positionen des Fixier- bzw. Anlageelements 25, 26 relativ zu dem Führungselement 23 festgelegt bzw. festlegbar.

Vorzugsweise weist das Fixier- bzw. Anlageelement 25, 26 und/oder das Führungselement 23 eine Positioniereinrichtung 31 zur Positionierung des Fixier- bzw. Anlageelements 25, 26 relativ zu dem Führungselement 23 auf. Die Positioniereinrichtung 31 ist vorzugsweise durch zueinander korrespondierende Flächen bzw. Abschnitte des Anlageelements 26 und des Führungselements 23 gebildet oder weist solche Flächen auf.

Die Positioniereinrichtung 31 ist vorzugsweise an einer dem Führungselement 23 zugeordneten bzw. zugewandten Stirnseite des Anlageelements 26 und/oder an einer dem Anlageelement 26 zugeordneten bzw. zugewandten Stirnseite des Führungselements 23 angeordnet.

Im Darstellungsbeispiel ist die Positioniereinrichtung 31 insbesondere durch eine oder mehrere Helixstrukturen 31a gebildet oder weist diese auf. Vorzugsweise weisen sowohl das Anlageelement 26 als auch das Führungselement 23 eine Helixstruktur 31a auf.

Durch die Helixstruktur 31a ist die (insbesondere axiale) Position des Anlageelements 26 relativ zu dem Führungselement 23 vorzugsweise einstellbar bzw. variierbar. Insbesondere ist die Position des Anlageelements 26 relativ zu dem Führungselement 23 durch Drehen des Anlageelements 26 um die Achse A bzw. durch Drehen des Anlageelements 26 relativ zu dem Führungselement 23 veränderbar. Bei einer solchen Rotation gleiten die Helixstrukturen 31a des Anlageelements 26 und des Führungselements 23 vorzugsweise aneinander entlang und verändern so die relative axiale Position des Führungselements 23 und Anlageelements 26 zueinander.

Anstelle der Helixstruktur 31a kann jedoch auch lediglich eine schiefe Ebene bzw. Schrägfläche oder dergleichen vorgesehen sein.

Vorzugsweise ist die axiale Position des Fixierelements 25 bzw. Anlageelements 26, insbesondere relativ zu dem Führungselement 23, durch die Drehlage des Fixierelements 25 bzw. Anlageelements 26 festgelegt bzw. festlegbar. Dies wird vorzugsweise durch die Positioniereinrichtung 31 ermöglicht bzw. realisiert, insbesondere durch die schiefe Ebene bzw. Schrägfläche bzw. Helixstruktur 31a.

Die Positioniereinrichtung 31 weist vorzugsweise eine oder mehrere Rastelemente 31b auf oder ist dadurch gebildet. Insbesondere ist durch die Rastelemente 31b das Anlageelement 26 in einer (definierten) axialen Position relativ zu dem Führungselement 23 an dem Führungselement 23 einrastbar.

Vorzugsweise weisen das Anlageelement 26 und das Führungselement 23 zueinander korrespondierende Rastelemente 31b auf.

Insbesondere sind die Rastelemente 31b des Anlageelements 26 und Führungselements 23 dazu ausgebildet, ineinander einzugreifen bzw. zusammenzuwirken.

Besonders bevorzugt sind die Rastelemente 31b derart ausgebildet, dass eine Rotation des Anlageelements 26 relativ zu dem Führungselement 23 nur in eine Rotationsrichtung um die Achse A ermöglicht ist und eine Rotation in eine dieser Rotationsrichtung entgegensetzte Richtung verhindert wird. Dies kann, wie in Fig. 6 dargestellt, durch eine entsprechende Ausbildung der Rastelemente 31B mit schrägen und senkrechten Flächen erfolgen.

Wenn das Anlageelement 26 an dem Führungselement 23 eingerastet ist bzw. nach erfolgter Montage der Abgabevorrichtung 1 stehen die Positioniereinrichtungen 31, Helixstrukturen 31a und/oder Rastelemente 31b des Anlageelements 26 und Führungselements 23 vorzugsweise in Kontakt bzw. liegen aneinander an. Lediglich für eine deutliche Darstellung ist in Fig. 6 ein Abstand zwischen dem Anlageelement 26 und dem Führungselement 23 dargestellt.

Durch die Positioniereinrichtung 31, insbesondere die schiefen Ebenen oder Helixstrukturen 31a und/oder Rastelemente 31b, werden vorzugsweise verschiedene diskrete Positionen des Fixier- bzw. Anlageelements 25, 26 relativ zu dem Führungselement 23 festgelegt. Mit anderen Worten ist vorzugsweise durch die Positioniereinrichtung 31, insbesondere die schiefen Ebenen oder Helixstrukturen 31a und/oder Rastelemente 31b, das Fixierelement 25 bzw. Anlageelement 26 in verschiedenen diskreten Positionen relativ zu dem Führungselement 23 an dem Führungselement 23 befestigbar, insbesondere einrastbar.

Nachfolgend wird insbesondere auf vorschlagsgemäße Verfahren zur Montage der Abgabevorrichtung 1 bzw. zur Montage (einer Vielzahl) von Abgabevorrichtungen 1 eingegangen.

Insbesondere wird nachfolgend zunächst ein Montageverfahren beschrieben, bei dem Dichtelemente 24 in Chargen vorliegen. Hierbei ist innerhalb einer Charge die Varianz bezüglich der signifikanten Größen der Dichtelemente 24 vorzugsweise gering. Bei diesem Verfahren wird vorzugsweise zunächst anhand einer Stichprobe einer kleinen Menge von Dichtelementen 24 der Charge ein Montageparameter M bestimmt und dieser Montageparameter M dann später bei der Montage der Dichtelemente 24 der Charge für jedes Dichtelement 24 verwendet. Insbesondere erfolgt also eine (chargenweise) Bestimmung des Montageparameters M vor bzw. unabhängig von der tatsächlichen Montage von Dichtelementen 24. Dieses Verfahren wird insbesondere als Chargenverfahren bezeichnet.

Bei einem weiteren, im Anschluss an das Chargenverfahren beschriebenen Montageverfahren wird vorzugsweise für jedes zu montierende Dichtelement 24 individuell bzw. separat ein Montageparameter M bestimmt und das Dichtelement 24 unter Verwendung dieses Montageparameters M montiert. Insbesondere erfolgt hierbei die Montage bzw. Fixierung des Dichtelements 24 während bzw. unmittelbar nach der Bestimmung des Montageparameters M. Bei diesem Verfahren können die Dichtelemente 24 hinsichtlich ihrer signifikanten Größen stärker voneinander abweichen und/oder aus verschiedenen Chargen stammen. Dennoch kann dieses Verfahren auch bei Dichtelementen 24 angewendet werden, die in Chargen vorliegen und eine geringe Varianz bezüglich der signifikanten Größen aufweisen. Dieses Verfahren wird insbesondere als Individualverfahren bezeichnet.

Zunächst wird nun jedoch auf das Chargenverfahren näher eingegangen.

Vorzugsweise wird für jede Charge von Dichtelementen 24 separat ein Montageparameter M bestimmt. Das Vorgehen bei der Bestimmung des Montageparameters M wird weiter unten detaillierter beschrieben, nachdem ein Überblick über das gesamte Chargenverfahren gegeben wurde.

Eine Charge weist vorzugsweise eine Vielzahl von Dichtelementen 24 auf, vorzugsweise mehr als 10.000, bevorzugt mehr als 100.000, insbesondere mehr als 200.000, Dichtelemente 24, und/oder weniger als 1.000.000, insbesondere weniger als 800.000, Dichtelemente 24.

Die Bestimmung des Montageparameters M erfolgt vorzugsweise anhand einer Stichprobe oder Teilmenge von Dichtelementen 24 der Charge.

Die Stichprobe weist insbesondere eine geringe Anzahl, vorzugsweise mindestens 10, bevorzugt mindestens 50, insbesondere mindestens 80 und/oder höchstens 250, bevorzugt höchstens 150, insbesondere höchstens 125, von Dichtelementen 24 der Charge auf. Vorzugsweise hängt die Größe der Stichprobe von der Größe der Charge ab, insbesondere wobei bei größeren Chargen größere Stichproben verwendet werden.

Bevorzugt weist die Stichprobe weniger als 50 %o, bevorzugt weniger als 20 %o, insbesondere weniger als 10 ‰, besonders bevorzugt weniger als 5 ‰, ganz besonders bevorzugt weniger als 2 %o, der Dichtelemente 24 der Charge auf.

Bevorzugt erfolgt die Bestimmung des Montageparameters M bzw. dessen Wert separat von der tatsächlichen Montage der Dichtelemente 24 bzw. der Abgabevorrichtung(en) 1, insbesondere in einer nicht dargestellten Prüfanlage.

Der Aufnahmeraum 27 der Prüfanlage weist vorzugsweise die gleichen Abmessungen auf wie der Aufnahmeraum 27 der Abgabevorrichtung 1. Nachfolgend wird jedoch der Einfachheit halber das Verfahren anhand eines Aufnahmeraums 27 der Abgabevorrichtung 1 beschrieben.

Ebenso muss das Anlageelement kein Anlageelement 26 der Abgabevorrichtung 1 sein, sondern kann auch durch ein Prüfelement der Prüfanlage gebildet sein. In diesem Fall ist das Prüfelement vorzugsweise ähnlich oder identisch ausgebildet wie das Anlageelement 26.

Die Prüfanlage weist vorzugsweise keine vollständige Abgabevorrichtung 1 bzw. keinen vollständigen Druckerzeuger 5 auf, sondern lediglich einen Aufnahmeraum 27 mit den gleichen Abmessungen wie der Aufnahmeraum 27 der Abgabevorrichtung 1 bzw. des Druckerzeugers 5. Vorzugsweise ist der Aufnahmeraum der Prüfanlage auch aus dem gleichen Material hergestellt wie der Aufnahmeraum 27 der Abgabevorrichtung 1.

Es ist nicht zwingend, dass die Prüfanlage ein mit dem (den Aufnahmeraum 27 aufweisenden bzw. bildenden) Führungselement 23 der Abgabevorrichtung 1 baugleiches Führungselement aufweist bzw. der Aufnahmeraum der Prüfungsanlage durch ein Führungselement gebildet ist, das baugleich mit einem in der Abgabevorrichtung 1 verwendeten Führungselement 23 ist. Entscheidend für eine Simulation der Abgabevorrichtung 1 bzw. der Verformung des Dichtelements 24 in der Abgabevorrichtung 1 bzw. des Aufnahmeraums 27 ist vielmehr lediglich, dass der in der Prüfanlage simulierte Aufnahmeraum in Abmessungen und/oder Material möglichst genau dem Aufnahmeraum 27 der Abgabevorrichtung 1 entspricht. Vorzugsweise ist daher, wie erwähnt, das Prüfelement der Prüfanlage ähnlich oder identisch ausgebildet wie das Anlageelement 26. Bevorzugt besteht das Prüfelement aus demselben Material wie das Anlageelement 26 und/oder weist das Prüfelement den gleichen Durchmesser bzw. Querschnitt senkrecht zur Achse A auf wie das Anlageelement 26.

Ferner ist es bevorzugt, dass die Prüfanlage einen Stößel 29 bzw. ein Zentralelement 30 aufweist. Durch den Stößel 29 bzw. das Zentralelement 30 wird vorzugsweise das Förderelement 9 simuliert. Es ist bevorzugt, dass der Stößel 29 bzw. das Zentralelement 30 aus demselben Material wie das Förderelement 9 besteht und/oder den gleichen Durchmesser bzw. Querschnitt senkrecht zur Achse A wie das Förderelement 9 aufweist. Der Stößel 29 und das Zentralelement 30 werden später noch detaillierter beschrieben.

Vorzugsweise wird in der Prüfanlage auf Bauteile der Abgabevorrichtung 1 bzw. des Druckerzeugers 5, die nicht für die Abdichtung des Aufnahmeraums 27 relevant sind, zum Beispiel die Austragsdüse 12 oder sämtliche Teile, die nicht Teil des Druckerzeugers 5 sind, verzichtet bzw. weist die Prüfanlage solche Bauteile nicht auf.

In diesem Sinne wird in der Prüfanlage vorzugsweise der Aufnahmeraum 27 bzw. der Druckerzeuger 5 bzw. die Abgabevorrichtung 1 lediglich simuliert.

Bei dem Chargenverfahren bzw. der chargenweisen Bestimmung des Montageparameters M wird vorzugsweise für alle Dichtelemente 24 der Stichprobe zunächst ein Montageparameter M auf die nachfolgend beschriebene Art und Weise bestimmt. Vorzugsweise wird dann aus den separat für die Dichtelemente 24 der Stichprobe bestimmten Montageparametern M der Mittelwert gebildet. Dieser Mittelwert stellt dann vorzugsweise den Montageparameter M der Charge dar und wird insbesondere bei jeder Montage eines Dichtelements 24 der Charge verwendet.

Der Montageparameter M wird vorzugsweise so gewählt bzw. eingestellt, dass sich für unterschiedliche Chargen von Dichtelementen 24 - zumindest im Wesentlichen - das gleiche Verhältnis zwischen dem Volumen des Aufnahmeraums 27 und dem Volumen des Dichtelements 24 in dem Aufnahmeraum 27 und/oder der gleiche Verformungswert des Dichtelements 24 ergibt. Mit anderen Worten wird der Montageparameter M vorzugsweise so gewählt, dass sich bei jeder Montage einer Abgabevorrichtung 1 der gleiche Verformungswert bzw. Füllgrad des Dichtelements 24 in dem Aufnahmeraum 27 ergibt. Die Abdichtung bzw. Dichtwirkung wird maßgeblich durch den Verformungswert bzw. Füllgrad bestimmt, so dass hierdurch eine zuverlässige Abdichtung auch bei Unterschieden zwischen den Dichtelementen 24 verschiedener Chargen erreicht werden kann.

Unter einem "gleichen" bzw. "zumindest im Wesentlichen gleichen" Verformungswert bzw. Füllgrad werden vorzugsweise Füllgrade bzw. Verformungswerte verstanden, die nur gering voneinander abweichen und/oder innerhalb einer Toleranz identisch sind. Insofern ist es also möglich, dass gleiche Verformungswerte bzw. Füllgrade nicht exakt übereinstimmen bzw. gering voneinander abweichen. Zwei Verformungswerte bzw. Füllgrade sind insbesondere dann gleich, wenn sie voneinander um weniger als 10 %, vorzugsweise weniger als 5 %, insbesondere weniger als 2 %, ganz besonders bevorzugt weniger als 1%, voneinander abweichen.

Der Montageparameter M ist vorzugsweise ein einstellbarer, insbesondere geometrischer, Wert, der bei der Montage der Abgabevorrichtung 1 einzuhalten ist und/oder bei der fertig montierten Abgabevorrichtung 1 realisiert wird. Vorzugsweise kann der Montageparameter M in den Einstellungen eines Montageablaufs bevorzugt variabel vorgegeben werden. Der Montageparameter M stellt vorzugsweise eine relative Position des Fixierelements 25 und/oder Anlageelements 26 in Bezug auf das Führungselement 23 dar oder korrespondiert hierzu. Vorzugsweise ist der Montageparameter M ein geometrischer Parameter. Insbesondere ist der Montageparameter M bzw. dessen Wert eine axiale Position des Fixierelements 25 und/oder Anlageelement 26. Mit anderen Worten wird diese Position durch den Montageparameter M bzw. dessen Wert vorzugsweise definiert. Da die Position des Fixierelements 25 und/oder Anlageelements 26, wie weiter oben erläutert, vorzugsweise zu dem Volumen des Aufnahmeraums 27 und damit vorzugsweise auch zu einer Verformung des Dichtelements 24 korreliert, kann durch die (chargenweise) Verwendung bzw. Vorgabe des Montageparameters M auf einfache Weise sichergestellt werden, dass mit unterschiedlichen Chargen von Dichtelementen 24 eine zuverlässige Abdichtung des Förderelements 9 durch das Dichtelement 24 erreicht wird.

Es ist auch möglich, dass der Montageparameter M nur mittelbar zu einer Position des Fixierelements 25 bzw. Anlageelements 26 korrespondiert, beispielsweise indem der Montageparameter M durch einen Zustellweg definiert oder gebildet ist, der von einem Teil einer Vorrichtung zur Montage der Abgabevorrichtung 1 bzw. zur Befestigung des Fixierelements 25 an dem Führungselement 23 durchfahren wird o. dgl.

Gemäß einem weiteren Beispiel, bei dem der Montageparameter M insbesondere nur mittelbar zu einer Position des Fixierelements 25 bzw. Anlageelements 26 korrespondiert, kann der der Montageparameter M durch einen Drehwinkel, einen Schraubvortrieb oder dergleichen eines zu befestigenden, insbesondere zu verschraubenden, Bauteils definiert oder gebildet sein. Insbesondere kann der Montageparameter M durch einen Drehwinkel, einen Schraubvortrieb oder dergleichen gebildet sein, wenn das Fixierelement 25 als Mutter bzw. Überwurfmutter ausgebildet ist, wie voranstehend beispielhaft erläutert. Durch die Vorgabe eines Drehwinkels oder Schraubvortriebs kann insbesondere die Endposition des Bauteils bzw. der Mutter und damit mittelbar dessen/deren axiale Position bzw. die Höhe des Aufnahmeraums 27 festgelegt werden.

Vorzugsweise korrespondieren unterschiedliche Montageparameter M bzw. unterschiedliche Werte des Montageparameters M zu unterschiedlichen Volumina des Aufnahmeraums 27 und/oder zu unterschiedlichen Höhen H des Aufnahmeraums 27. Mit anderen Worten entspricht vorzugsweise jeder Montageparameter M bzw. jeder Wert des Montageparameters M einem bestimmten Volumen des Aufnahmeraums 27 bzw. einer bestimmten Höhe H des Aufnahmeraums 27.

Alternativ oder zusätzlich kann der Montageparameter M jedoch auch zu einer bestimmten Kraft korrespondieren, mittels der das in dem Aufnahmeraum 27 fixierte Dichtelement 24 verformt bzw. fixiert wird. Vorzugsweise sind das Volumen des Aufnahmeraums 27 bzw. die Höhe H des Aufnahmeraums 27 jeweils direkt korreliert mit einer bestimmten Kraft bzw. einem Druck, mit dem das Dichtelement 24 in dem yAufnahmeraum 27 verformt wird. Daher kann anstelle eines geometrischen Parameters wie einer Position des Anlageelements 26 oder einer Höhe H des Aufnahmeraums 27 auch eine bestimmte Kraft zur Definition des Montageparameters M bzw. als Montageparameter M verwendet werden. Insbesondere korrespondiert diese Kraft dann zu einem bestimmten Volumen bzw. einer bestimmten Höhe H des Aufnahmeraums 27.

Mittels des Montageparameters M ist vorzugsweise das Volumen bzw. die Höhe H des Aufnahmeraums 27 einstellbar bzw. festlegbar. Insbesondere können für verschiedene Dichtelemente 24 bzw. verschiedene Chargen von Dichtelementen 24 unterschiedliche Montageparameter M - genauer gesagt unterschiedliche Werte des Montageparameters M - gewählt werden, so dass das Volumen des Aufnahmeraums 27 auf das jeweilige Dichtelement 24 bzw. die jeweilige Charge von Dichtelementen 24 anpassbar ist und für unterschiedliche Dichtelemente 24 bzw. Chargen von Dichtelementen 24 der gleiche Füllgrad bzw. die gleiche Dichtwirkung bzw. Abdichtung erreicht wird.

Vorzugsweise wird der Montageparameter M für eine Charge zunächst anhand einer Teilmenge bzw. Stichprobe von Dichtelementen 24 der Charge bestimmt. Anschließend wird der bestimmte bzw. gewählte Montageparameter M vorzugsweise bei jeder Montage einer Abgabevorrichtung 1 bzw. Fixierung eines Dichtelements 24 der jeweiligen Charge in einem Aufnahmeraum 27 verwendet. Insbesondere ist der Montageparameter M ein Wert, der bei der Montage der Abgabevorrichtung 1 (einfach) eingestellt werden kann und/oder nicht gemessen bzw. bestimmt werden muss, so dass eine schnelle und effiziente, insbesondere automatisierte, Montage einer Vielzahl von Abgabevorrichtungen 1 ermöglicht ist.

Die Montage der Dichtelemente 24 bzw. Abgabevorrichtungen 1 erfolgt vorzugsweise in einem von der Bestimmung des Montageparameters M separaten Prozess und/oder in einer anderen Anlage als die Bestimmung des Montageparameters M.

Vorzugsweise erfolgt die Montage der Dichtelemente 24 bzw. Abgabevorrichtungen 1 erfolgt in einem vollautomatisierten Prozess. Hierbei wird insbesondere eine große Anzahl von Abgabevorrichtungen 1 in einem hohen Takt montiert. Insbesondere werden mehrere hundert oder mehrere tausend Abgabevorrichtungen 1 pro Stunde montiert.

Zur Montage wird zunächst ein Dichtelement 24 in einen Aufnahmeraum 27 einer Abgabevorrichtung 1 eingelegt. Anschließend wird ein Fixierelement 25 und/oder Anlageelement 26 in den Aufnahmeraum 27 eingeführt. Wie bereits voranstehend beschrieben, können das Fixierelement 25 und das Anlageelement 26 entweder durch verschiedene, voneinander separate Bauteile gebildet sein oder verschiedene Abschnitte eines einzigen Bauteils darstellen. In einem bevorzugten Ausführungsbeispiel, das auch in den Figuren dargestellt ist, sind das Fixierelement 25 und das Anlageelement 26 durch zwei separate Bauteile gebildet. In diesem Fall wird das Anlageelement 26 in den Aufnahmeraum 27 eingeführt.

Das Fixierelement 25 bzw. Anlageelement 26 wird vorzugsweise so weit in den Aufnahmeraum 27 hineinbewegt, dass das Dichtelement 24 verformt wird und insbesondere der Verformungswert des Dichtelements 24 den Schwellwert erreicht oder überschreitet. Hierzu wird insbesondere der vorab bestimme Montageparameter M verwendet, durch den vorzugsweise festgelegt ist, wie weit das Fixierelement 25 bzw. Anlageelement 26 in den Aufnahmeelement 27 hineinbewegt wird bzw. in welcher Position das Fixierelement 25 und/oder Anlageelement 26, insbesondere das Anlageelement 26 mittels des Fixierelements 25, fixiert werden soll.

Vorzugsweise wird das Anlageelement 26 mittels eines Stempels oder Stößels in die gewünschte Position bewegt. Der Stempel oder Stößel kann beispielsweise grundsätzlich ähnlich wie der später näher beschriebene Stößel 29, der bei der Bestimmung des Montageparameters M verwendet wird und in den Fig. 4A-D dargestellt ist, ausgebildet sein. Vorzugsweise weist der bei der Montage verwendete Stempel oder Stößel jedoch kein Zentralelement 30 auf und/oder durchgreift der Stempel oder Stößel bei der Montage nicht das Anlageelement 26 und/oder das Dichtelement 24.

Das Fixierelement 25 wird vorzugsweise über das Anlageelement 26 und/oder das Führungselement 23 geschoben, nachdem das Anlageelement 26 in der zu dem Montageparameter M korrespondierenden bzw. durch den Montageparameter M definierten Position positioniert wurde.

Es ist jedoch auch möglich, dass das Fixierelement 25 bei der Positionierung des Anlageelements 26 bereits über das Anlageelement 26 geschoben ist und/oder das Anlageelement 26 kontaktiert, sodass das Fixierelement 25 und das Anlageelement 26 gleichzeitig in Richtung des Dichtelements 24 bewegt werden. Hierbei wird das Anlageelement 26 mittelbar über das Fixierelement 25 in Richtung des Dichtelements 24 bewegt. In diesem Fall kontaktiert der Stempel oder Stößel zur Bewegung des Fixierelements 25 und Anlageelements 26 vorzugsweise das Fixierelement 25 und wirkt nur mittelbar über das Fixierelement 25 auf das Anlageelement 26 ein.

Wenn das Fixierelement 25 und/oder Analageelement 26 die gewünschte bzw. durch den Montageparameter M definierte bzw. zu dem Montageparameter M korrespondierende Position erreicht haben, wird das Fixierelement 25 vorzugsweise an dem Führungselement 23 befestigt bzw. fixiert.

Es ist bevorzugt, dass das Fixierelement 25 zusätzlich an dem Anlageelement 26 fixiert bzw. befestigt wird.

Unter einer "Fixierung" eines Dichtelements 24 wird insbesondere eine Festsetzung oder Einspannung des Dichtelements 24 in dem Aufnahmeraum 27 derart verstanden, dass sich das Dichtelement 24 in dem Aufnahmeraum 27 nicht bewegen und/oder nicht entformt werden kann, insbesondere also nicht in die unverformte Ausgangsform des Dichtelements 24 zurückkehren kann. Dies wird vorzugsweise dadurch erreicht, dass das Dichtelement 24 mittels des Fixierelements 25 und optional mittels des Anlageelements 26 in dem Aufnahmeraum 27 festgelegt bzw. eingespannt wird, wobei das Fixierelement 25 bzw. Anlageelement 26 auf das Dichtelement 24 drückt und es somit verformt, so dass das Dichtelement 24 großflächig bzw. möglichst allseitig an den Begrenzungen des Aufnahmeraums 27, insbesondere also dem Führungselement 23 und/oder dem Fixierelement 25 bzw. Anlageelement 26 anliegt. Bei der fertig montierten Abgabevorrichtung 1 liegt das Dichtelement 24 darüber hinaus vorzugsweise an dem durch das Dichtelement 24 geführten Förderelement 9 an. Durch den Aufnahmeraum 27 bzw. das Führungselement 23 und das Fixierelement 25 bzw. Anlageelement 26 und vorzugsweise das Förderelement 9 wird vorzugsweise eine weitere Verformung des Dichtelements 24 nach der Montage begrenzt bzw. verhindert.

Eine Fixierung des Dichtelements 24 erfolgt also vorzugsweise über eine Fixierung des Anlageelements 26 und/oder durch die Befestigung des Fixierelements 25 an dem Führungselement 23 und/oder Anlageelement 26. Vorzugsweise wird durch die Befestigung des Fixierelements 25 an dem Führungselement 23 die Höhe H und/oder das Volumen des Aufnahmeraums 27 definiert bzw. festgelegt oder fixiert.

Nach dem Fixieren des Fixierelements 25 und/oder Anlageelements 26 wird vorzugsweise das Förderelement 9 montiert. Hierzu wird das Förderelement 9 durch das Fixierelement 25 durch das Anlageelement 26 und/oder Dichtelement 24 in den Kanal 23b eingeführt. Grundsätzlich kann die Montage bzw. das Einführen des Förderelements 9 jedoch auch vor dem Fixieren des Fixierelements 25 und/oder Anlageelements 26 erfolgen.

Bevorzugt ist bei der Montage der Abgabevorrichtung die relative Position des Fixierelements 25 und/oder Anlageelements 26 gegenüber dem Führungselement 23 mittels des Montageparameters M vorgebbar bzw. einstellbar. Vorzugsweise findet bei der Montage eine Wegmessung bzw. Höhenmessung statt, mittels der die Höhe H des Aufnahmeraums 27 und/oder die relative Position des Fixierelements 25 bzw. Anlageelement 26, insbesondere relativ zu dem Aufnahmeraum 27 bzw. dessen Boden 27a und/oder dem Führungsteil 23, direkt oder indirekt überprüfbar und/oder steuerbar ist. Hierbei sind vorzugsweise alle gängigen Wegmessverfahren anwendbar, beispielsweise taktile, optische, induktive Verfahren oder dgl.

Insbesondere ist es durch die chargenweise Bestimmung des Montageparameters M bzw. dessen Werts nicht nötig, den Montageparameter M bzw. dessen Wert bei jeder Montage einer Abgabevorrichtung 1 neu zu bestimmen bzw. einzustellen, sondern der Montageparameter M bzw. dessen Wert wird vorzugsweise zunächst (anhand einer Teilmenge bzw. Stichprobe von Dichtelementen 24 der Charge) für eine Charge bestimmt und dann für jedes Dichtelement 24 dieser Charge verwendet. Hierdurch kann das Verfahren zur Montage von Abgabevorrichtungen 1 vereinfacht und beschleunigt werden.

Nachfolgend wird nun die Bestimmung des Montageparameters M detaillierter erläutert, insbesondere unter Bezugnahme auf die Figuren 4A bis 4D und 5.

Zur Bestimmung des Montageparameters M wird das jeweilige Dichtelement 24 zunächst in dem Aufnahmeraum 27 angeordnet bzw. in den Aufnahmeraum 27 eingelegt. Anschließend wird vorzugsweise das Fixierelement 25 bzw. Anlageelement 26 auf das Dichtelement 24 aufgesetzt bzw. in Kontakt mit dem Dichtelement 24 gebracht.

Dies ist in den Figuren 4A-D dargestellt. In Fig. 4A ist dargestellt, dass das Anlageelement 26 bereits in den Aufnahmeraum 27 eingeführt wurde, aber noch nicht in Kontakt mit dem Dichtelement 24 steht. In Fig. 4B wurde das Anlageelement 26 so weit in den Aufnahmeraum 27 hineinbewegt, dass es in Kontakt mit dem Dichtelement 24 steht.

Die Fig. 4C zeigt die gleiche Situation wie Fig. 4A. Die Fig. 4D zeigt die gleiche Situation wie Fig. 4B. Die Figuren 4C und 4D unterscheiden sich von den Figuren 4A und 4B lediglich dadurch, dass das Dichtelement 24 in den Figuren 4C und 4D ein kleineres Volumen bzw. einen kleineren Durchmesser bzw. eine kleinere Schnurstärke D aufweist, als das Dichtelement 24 in den Figuren 4A und 4B.

Anschließend wird vorzugsweise das Dichtelement 24, insbesondere mittels des Fixierelements 25 und/oder Anlageelements 26 in dem Aufnahmeraum 27 verformt bzw. in den Aufnahmeraum 27 gedrückt. Dies erfolgt insbesondere durch Ausübung einer Kraft bzw. eines Drucks auf das Dichtelement 24, insbesondere mittels des Fixierelements 25 und/oder Anlageelements 26. Eine Verformung des Dichtelements 24 durch das Anlageelement 26 zur Bestimmung des Montageparameters M bei bzw. während der Bestimmung des Montageparameters M ist insbesondere in Figur 4D dargestellt.

Vorzugsweise wird das Dichtelement 24 durch eine axial auf das Dichtelement 24 einwirkende Kraft bzw. einen axial auf das Dichtelement 24 einwirkenden Druck verformt. Grundsätzlich ist es jedoch auch möglich, das Dichtelement 24 alternativ oder zusätzlich durch eine radiale Kraft zu verformen. Bevorzugt wird hierbei nach Begrenzung des Aufnahmeraums 27 in axialer Richtung (d.h. insbesondere nach Aufsetzen des Fixierelements 25 bzw. Anlageelements 26) eine radial nach innen wirkende Kraft verwendet.

Vorzugsweise wird mittels eines Stößels 29 die Kraft bzw. der Druck auf das Dichtelement 24 ausgeübt bzw. das Dichtelement 24 verformt. Insbesondere wird mittels des Stößels 29 das Fixierelement 25 bzw. Anlageelement 26 in Richtung des Dichtelements 24 bzw. in den Aufnahmeraum 27 hineinbewegt, sodass das Fixierelement 25 bzw. Anlageelement 26 das Dichtelement 24 kontaktiert und durch Ausübung einer Kraft bzw. eines Drucks auf das Dichtelement 24 dieses verformt. Der Stößel 29 ist beispielhaft in Fig. 4A-D dargestellt.

Vorzugsweise wirkt der Stößel 29 nur mittelbar auf das Dichtelement 24 ein. Es ist bevorzugt vorgesehen, dass der Stößel 29, insbesondere axial, auf das Fixierelement 25 und/oder Anlageelement 26 einwirkt bzw. drückt. Insbesondere wird der Stößel 29 axial zu dem Fixierelement 25 bzw. Anlageelement 26 verfahren, so dass hierdurch das Fixierelement 25 bzw. Anlageelement 26 axial in Richtung des Dichtelements 24 bewegt und dadurch das Dichtelement 24 verformt wird.

Der Stößel 29 ist vorzugsweise kein Teil der Abgabevorrichtung 1, sondern ein Teil einer Prüfanlage, in der die Bestimmung des Montageparameters M vorgenommen wird.

Zur bzw. bei der Bestimmung des Montageparameters M wird vorzugsweise ein Zentralelement 30 in den Kanal 23b bzw. durch die (Zentral-)Öffnung 24a des Dichtelements 24 geführt. Das Volumen des Aufnahmeraums 27 wird vorzugsweise durch das Zentralelement 30 verringert oder begrenzt. Das Zentralelement 30 ist beispielhaft in Fig. 4A-D dargestellt.

Insbesondere stellt das Zentralelement 30 eine innere radiale Begrenzung des Aufnahmeraums 27 während der Korrelationsmessung bzw. Bestimmung des Montageparameters M dar.

Das Zentralelement 30 weist vorzugsweise den gleichen Durchmesser bzw. in radialer Richtung die gleichen äußeren Abmessungen wie das Förderelement 9 auf.

Vorzugsweise ist das Zentralelement 30 ein Abschnitt des Stößels 29 bzw. einstückig mit dem Stößel 29 ausgebildet. Das Zentralelement 30 kann jedoch auch separat von dem Stößel 29 gebildet sein bzw. ein separates Bauteil bilden.

Das Zentralelement 30 kann zumindest in einem Abschnitt verjüngt, konisch ausgebildet und/oder abgerundet sein, so dass sich bei axialem Vorschieben des Zentralelements 30 die durch das Zentralelement 30 begrenzte radiale Erstreckung bzw. Breite B des Aufnahmeraums 27 verringert. Hierdurch kann das Einführen des Zentralelements 30 in den Kanal 23b bzw. durch die Öffnung 24a des Dichtelements 24 erleichtert und/oder eine Beschädigung des Dichtelements 24 verhindert werden.

Grundsätzlich kann das Zentralelement 30 auch durch das Förderelement 9 gebildet sein.

Vorzugsweise wird bei der Korrelationsmessung bzw. Bestimmung des Montageparameters M das Dichtelement 24 gegen das Zentralelement 30 gedrückt und insbesondere dabei verformt.

Nach der Bestimmung des Montageparameters M wird das Zentralelement 30 vorzugsweise wieder aus dem Kanal 23b bzw. der Öffnung 24a des Dichtelements 24 entfernt.

Der Montageparameter M wird vorzugsweise derart gewählt, dass bei einer Fixierung des Dichtelements 24 ein zu der Verformung des Dichtelements 24 korrespondierender Verformungswert einen Schwellwert erreicht oder überschreitet.

Unter einem "Überschreiten" des Schwellwerts durch den Verformungswert wird sowohl verstanden, wenn der Verformungswert zunächst kleiner als der Schwellwert ist und anschließend einen Wert erreicht, der größer als der Schwellwert ist, als auch umgekehrt, also wenn der Verformungswert zunächst größer als der Schwellwert ist und anschließend einen Wert erreicht, der kleiner als der Schwellwert ist.

Es können auch mehrere Schwellwerte vorgegeben sein, wobei der Montageparameter M vorzugsweise so gewählt wird, dass der Verformungswert zwischen den Schwellwerten liegt. Insbesondere können die Schwellwerte also einen Maximalwert und einen Minimalwert für den Verformungswert darstellen.

Der Verformungswert korrespondiert vorzugsweise zu der Verformung und/oder einer Kompression des Dichtelements 24. Mit anderen Worten ist der Verformungswert vorzugsweise ein Wert bzw. eine Maßzahl, der/die angibt, wie stark und/oder auf welche Weise das Dichtelement 24 verformt und/oder komprimiert ist. Der Verformungswert wird vorzugsweise mittelbar anhand eines Kennwerts ermittelt, wie nachfolgend noch näher erläutert wird.

In den Figuren 5B und 5C ist die Verformung des Dichtelements 24 schematisch dargestellt.

Fig. 5B zeigt das unverformte Dichtelement 24, insbesondere vor einem Einlegen in den Aufnahmeraum 27. Im Darstellungsbeispiel gemäß Fig. 5B weist das Dichtelement 24 einen kreisförmigen Querschnitt mit einem Durchmesser bzw. einer Schnurstärke D auf. Grundsätzlich kann das Dichtelement 24 bzw. dessen Querschnitt jedoch beliebig geformt sein.

In Fig. 5C ist ein verformtes Dichtelement 24 dargestellt. Insbesondere entspricht die Darstellung in Figur 5C einem in einer fertig montierten Abgabevorrichtung 1 bzw. deren Aufnahmeraum 27 eingesetzten und fixierten Dichtelement 24. Wie aus Fig. 5C ersichtlich ist, wurde das Dichtelement 24 im Vergleich zur Ausgangssituation in Fig. 5B verformt und ist insbesondere in seiner äußeren Form an die Form des Aufnahmeraums 27 angepasst.

Bei der Verformung des Dichtelements 24 erfolgt vorzugsweise zunächst eine, vorzugsweise elastische, Deformation, bei der sich das Volumen des Dichtelements 24 nicht (wesentlich) ändert bzw. lediglich die äußere Form des Dichtelements 24 geändert bzw. dem Aufnahmeraum 27 angepasst wird. Typischerweise setzt bei einer weiteren Erhöhung der Druck- bzw. Kraftausübung bzw. Verformung des Dichtelements 24 anschließend an die reine Formänderung eine Volumenkompression, also Änderung bzw. Verringerung des Volumens, des Dichtelements 24 ein, insbesondere zusätzlich zu der primär reinen Formänderung.

Besonders bevorzugt entspricht der Schwellwert einem Verformungswert, der zu diesem Einsetzen der Volumenkompression des Dichtelements 24 korrespondiert bzw. bei dem die Volumenkompression einsetzt bzw. beginnt. Abhängig vom Material des Dichtelements 24 kann es jedoch auch wünschenswert bzw. vorteilhaft sein, als Schwellwert einen Wert zu wählen, der gezielt oberhalb oder unterhalb des Werts liegt, bei dem die Volumenkompression einsetzt. Beispielsweise kann es für ein Material mit hohem Kriechverhalten, zum Beispiel ein thermoplastisches Elastomer (TPE), vorteilhaft sein, den Schwellwert so anzusetzen, dass noch keine Volumenkompression einsetzt.

Der Montageparameter M wird vorzugsweise anhand des Verformungsverhaltens und/oder Reibungsverhaltens des Dichtelements 24 während einer Verformung des Dichtelements 24 in dem Aufnahmeraum 27 bestimmt.

Die Untersuchung bzw. Bestimmung des Verformungsverhaltens und/oder Reibungsverhaltens wird nachfolgend anhand der Fig. 4A-D und 5 näher erläutert.

Zur Untersuchung bzw. Bestimmung des Verformungsverhaltens wird vorzugsweise mittels des Stößels 29, Fixierelements 25 und/oder Anlageelements 26 eine (zunehmende) Kraft auf das Dichtelement 24 ausgeübt und dabei das Dichtelement 24 verformt. Insbesondere wird bei mehreren unterschiedlichen Verformungen bzw. Verformungswerten des Dichtelements 24 jeweils die zum Erreichen dieser Verformung bzw. dieses Verformungswertes benötigte Kraft bestimmt. Vorzugsweise wird ebenfalls ein zu der Kraft korrespondierender Weg bestimmt.

Zur Untersuchung bzw. Bestimmung des Reibungsverhaltens werden vorzugsweise eine Begrenzung des Aufnahmeraums 27, insbesondere das Zentralelement 30, und das Dichtelement 24 relativ zueinander bewegt und zu mehreren Zeitpunkten während der Verformung jeweils die Reibungskraft zwischen dem Dichtelement 24 und der Begrenzung bestimmt.

Durch die bzw. während der Untersuchung bzw. Bestimmung des Verformungsverhaltens und/oder Reibungsverhaltens des Dichtelements 24 wird vorzugsweise der Verformungswert zumindest mittelbar bestimmt. Der Verformungswert selbst muss jedoch nicht unmittelbar bestimmt werden bzw. bekannt sein. Beispielsweise ist es möglich, dass aus vorherigen Versuchen bekannt ist, zu welchen zur Verformung benötigten Kräften bzw. Reibungskräften zwischen dem Dichtelement 24 und der Begrenzung ein bestimmter Verformungswert korrespondiert.

Zur Untersuchung des Verformungs- und/oder Reibungsverhaltens wird vorzugsweise ein Kennwert gemessen. Über den Kennwert lassen sich insbesondere Rückschlüsse auf den Verformungswert ziehen bzw. lässt sich der Verformungswert bestimmen. Mit anderen Worten ist der Kennwert vorzugsweise eine Größe bzw. ein Wert, die/der bestimmt bzw. gemessen wird, um den Verformungswert zu bestimmen, zu berechnen und/oder aus dem Kennwert abzuleiten. Der Kennwert ist vorzugsweise mit dem Verformungswert korreliert. Vorzugsweise korrespondiert also zu jedem Wert des Kennwerts ein bestimmter Verformungswert.

Der Kennwert ist vorzugsweise eine Kraft und/oder ein Druck, die/der zur Verformung auf das Dichtelement 24 ausgeübt wird und/oder eine, insbesondere axiale, Position des Stößels 29, mit dem das Dichtelement 24 verformt wird. Mit anderen Worten wird also vorzugsweise die Kraft und/oder der Druck gemessen, mit der/dem das Dichtelement 24 verformt wird, und/oder wird die Position des Stößels 29 gemessen, mit dem das Dichtelement 24 verformt wird. Insbesondere kann die Kraft mittelbar durch die Position des Stößels 29 bekannt sein. Hierdurch kann für unterschiedliche Dichtelemente 24 der gewünschte Verformungswert bzw. (damit) eine zuverlässige Abdichtung bzw. Dichtwirkung erreicht werden.

Die Kraft ist vorzugsweise jeweils auf eine bestimmte Fläche bezogen, insbesondere die Fläche des Aufnahmeraums 27 bzw. des Bodens 27a. Insofern korrespondiert die Kraft jeweils direkt zu einem Druck bzw. ist die Kraft jeweils eine auf eine Fläche normierte bzw. bezogene Kraft. Der Begriff "Kraft" ist vorzugsweise auch durch den Begriff "Druck" austauschbar.

Das Verformungsverhalten wird vorzugsweise mittels der Kraft bzw. des Drucks, die zur Verformung auf das Dichtelement 24 ausgeübt wird, bestimmt. Alternativ oder zusätzlich wird das Verformungsverhalten (mittelbar) mittels einer, insbesondere axialen, Position des Stößels 29 und/oder Fixier- bzw. Anlageelements 25, 26 bestimmt.

Durch die Position des Stößels 29 und/oder Fixier- bzw. Anlageelements 25, 26 ist insbesondere eine Höhe H des Aufnahmeraums 27 festgelegt.

Insbesondere kann also die Höhe H des Aufnahmeraums 27 oder eine dazu korrelierte Größe, wie eine Zustellung oder axiale Position des Stößels 29 und/oder Fixier- bzw. Anlageelements 25, 26, vorgegeben oder eingestellt werden und die hierzu korrespondierende Kraft bestimmt bzw. gemessen werden. In einer Umkehrung dieses Prinzips ist es jedoch ebenfalls möglich, die Kraft vorzugeben oder einzustellen und die zu der jeweils eingestellten oder vorgegebenen Kraft korrespondierende Höhe H des Aufnahmeraums 27 bzw. dazu korrelierte Größe zu bestimmen bzw. messen.

Die Kraft bzw. der Druck und/oder die Position des Stößels 29 werden vorzugsweise gemessen. Es ist jedoch auch möglich, dass die Kraft, der Druck und/oder die Position ohne eine explizite Messung bekannt, vorgegeben oder berechenbar bzw. bestimmbar sind, beispielsweise aus der Größe eines zur Bewegung des Stößels 29 verwendeten Kontrollparameters, wie einer (elektrischen) Spannung o. dgl., durch den die von dem Stößel 29 ausgeübte Kraft gesteuert oder geregelt wird.

Alternativ oder zusätzlich zur Bestimmung des Verformungsverhaltens des Dichtelements 24 während einer Verformung kann, wie bereits erwähnt, auch eine Bestimmung des Reibungsverhaltens erfolgen, während das Dichtelement 24 verformt wird. Die Verformung des Dichtelements 24 in dem Aufnahmeraum 27 lässt sich nämlich auch (indirekt) über die Messung einer Messhilfsgröße wie z. B. einer Reibungskraft bestimmen, die zwischen dem Dichtelement 24 und einer mit dem Dichtelement 24 in Kontakt stehenden und relativ zu dem Dichtelement 24 bewegten Begrenzung des Aufnahmeraums 27 auftritt. Mit zunehmendem Anpressdruck des Dichtelements 24 auf die Begrenzung steigt die Reibungskraft, wobei der Anstieg der Reibungskraft davon abhängt, welche Verformung beim Dichtelement 24 stattfindet und/oder wie groß die Kontaktfläche zwischen dem Dichtelement 24 und der Begrenzung des Aufnahmeraums 27 ist. Insbesondere nimmt mit zunehmender Verformung des Dichtelements 24 der Anpressdruck zu, was in einer veränderten, insbesondere erhöhten, Reibungskraft zwischen dem Dichtelement 24 und der Begrenzung des Aufnahmeraums 27 resultiert. Anfänglich erfolgt hierbei eine (zumindest überwiegend elastische) Deformation des Dichtelements 24, die mit zunehmender Verformung sukzessiv in eine Kompression des Dichtelements 24 übergeht. Insbesondere ist der Anstieg bzw. Gradient der Reibungskraft bei einer Kompression des Dichtelements 24 größer als bei einer elastischen Deformation des Dichtelements. Über das Reibungsverhalten ist es somit möglich, Rückschlüsse auf die Verformung bzw. das Verformungsverhalten des Dichtelements 24 ziehen, insbesondere die Verformung bzw. das Verformungsverhalten des Dichtelements 24 (indirekt oder mittelbar) zu bestimmen.

Zur Bestimmung des Reibungsverhaltens wird vorzugsweise das Dichtelement 24 relativ zu einer Begrenzung des Aufnahmeraums 27 bewegt. Dies kann beispielsweise dadurch erfolgen, dass der Stößel 29 bzw. das Zentralelement 30 relativ zu dem Dichtelement 24 bewegt wird, insbesondere axial bzw. entlang der Achse A. In diesem Fall bildet das Zentralelement 30 die Begrenzung des Aufnahmeraums 27, die relativ zu dem Dichtelement 24 bewegt wird. Vorzugsweise wird hierbei der Stößel 29 bzw. das Zentralelement 30 oszilliert bzw. hin und her bewegt. Dabei wird insbesondere die zur Bewegung des Stößels 29 bzw. Zentralelements 30 benötigte Kraft gemessen. Diese gemessene Kraft entspricht vorzugsweise der Reibungskraft zwischen dem Stößel 29 bzw. Zentralelement 30 und dem Dichtelement 24 oder ist dazu korreliert. Die gemessene Kraft bzw. Reibungskraft stellt als (insbesondere indirekte) Messhilfsgröße vorzugsweise den Kennwert dar, aus dem der Verformungswert und damit insbesondere auch der Schwellwert bestimmt, berechnet oder abgeleitet wird.

Bei diesem Verfahren ist vorzugsweise vorgesehen, dass das Fixierelement 25 bzw. Anlageelement 26 mittels eines weiteren, in den Fig. 4A-D nicht dargestellten Stößels fixiert bzw. in den Aufnahmeraum 27 bewegt bzw. gedrückt wird.

Grundsätzlich kann die Reibungskraft jedoch auch auf andere Art und Weise gemessen werden, beispielsweise indem der Stößel 29 bzw. das Zentralelement 30 relativ zu dem Dichtelement 24 und/oder um die Achse A rotiert wird und die dabei auftretende Torsionsreibung bzw. Torsionsreibungskraft gemessen wird.

Ein Vorteil der Bestimmung des Reibungsverhaltens während der Verformung des Dichtelements 24 besteht darin, dass durch eine Kurzzeit-Prüfung, nämlich die Messung der Reibungskraft, das Langzeitverhalten des Dichtelements 24 in der Abgabevorrichtung 1 vorhersagbar ist.

Ein weiterer Vorteil der Bestimmung des Reibungsverhaltens während der Verformung des Dichtelements 24 besteht darin, dass die Bestimmung des Schwellwerts bzw. Montageparameters M direkt anhand eines Parameters bzw. Kennwerts, nämlich der Reibungskraft, erfolgt, der sich auf die Eigenschaften des mit der Abgabevorrichtung 1 erzeugten Aerosols 14 auswirkt. Je größer die Reibungskraft zwischen dem Dichtelement 24 und dem Förderelement 9 ist, die beim Bewegen des Förderelements 9 zur Erzeugung des Aerosols 14 auftritt, desto weniger Energie steht für die Erzeugung des Aerosols 14 aus dem Fluid zur Verfügung, denn ein Teil der insgesamt zur Verfügung stehenden Energie geht über die Reibung verloren. Eine zu große Reibungskraft führt daher dazu, dass die Tröpfchen des erzeugten Aerosols 14 eine ungleichmäßigere und/oder kleinere Größe aufweisen und/oder die Dauer eines Sprühstoßes beeinträchtigt, insbesondere verkürzt, wird. Dies wirkt sich nachteilig auf die Wirksamkeit und exakte Dosierung des mit der Abgabevorrichtung 1 als Aerosol 14 versprühten Arzneimittels aus.

Insbesondere wird zur Untersuchung bzw. Bestimmung des Verformungsverhaltens bzw. Reibungsverhaltens und/oder zur Bestimmung des Montageparameters M eine Kraft-Weg-Kurve aufgenommen. Ganz besonders bevorzugt wird der Montageparameter M anhand des Verlaufs der Kraft-Weg-Kurve bestimmt.

In Fig. 5A ist beispielhaft eine Kraft-Weg-Kurve dargestellt. Hierbei ist auf der x-Achse ein Weg aufgetragen und auf der y-Achse eine Kraft F aufgetragen.

In Figur 5A ist der Weg durch die Höhe H des Aufnahmeraums 27 repräsentiert. Der Weg kann jedoch auch dem zurückgelegten Weg bzw. der Position des Stößels 29 und/oder Fixier- bzw. Anlageelements 25, 26 entsprechen, mit dem das Dichtelement 24 verformt wird. Der Weg kann gemessen werden oder auf sonstige Weise bekannt sein, beispielsweise aus einem Kontrollparameter, wie einer elektrischen Spannung oder dergleichen, durch den ein Vorschub des Stößels 29 und/oder Fixier- bzw. Anlageelements 25, 26 gesteuert oder geregelt wird. Insbesondere entspricht ein bestimmter Wert des Wegs bzw. der Höhe H einem bestimmten Volumen des Aufnahmeraums 27.

Die Kraft F ist beispielsweise die bereits erläuterte Kraft, die zur Verformung auf das Dichtelement 24 ausgeübt wird oder eine hierzu korrespondierende Größe, wie der ausgeübte Druck und/oder die bei der Relativbewegung zwischen dem Dichtelement 24 und der Begrenzung des Aufnahmeraums 27 bzw. dem Zentralelement 30 auftretende Reibungskraft.

Eine bestimmte Verformung bzw. ein bestimmter Verformungswert des Dichtelements 24 korrespondiert vorzugsweise zu einer bestimmten Kraft, die zum Erreichen dieser Verformung bzw. dieses Verformungswertes benötigt wird, und/oder zu einer bestimmten Höhe H des Aufnahmeraums 27 bzw. einer dazu korrelierten Größe, bei der diese Verformung bzw. dieser Verformungswert erreicht wird. Insbesondere lässt sich daher der Verformungswert mittels der Kraft und/oder der Höhe H bestimmen.

Beispielhaft sind in Fig. 5A zwei verschiedene Kräfte F1, F2 und die zugehörigen Wege bzw. Höhen H1, H2 eingezeichnet. Der Weg bzw. die Höhe H ist vorzugsweise mit dem zurückgelegten Weg bzw. der Position des Stößels 29 korreliert, mit dem das Dichtelement 24 verformt wird.

Die Fig. 5A wird nachfolgend "von rechts nach links" erläutert, also beginnend bei großen Werten von H. Die Kraft-Weg-Kurve beginnt bei dem Wert D für die Höhe H, wobei D die Höhe des unverformten Dichtelements 24, insbesondere die axiale Höhe bzw. Querschnittshöhe eines ringförmigen Dichtelements 24, besonders bevorzugt die Schnurstärke eines torusförmigen Dichtelements 24, ist, wie beispielsweise in Fig. 5B dargestellt. An diesem Punkt kontaktiert das Fixierelement 25 bzw. Anlageelement 26 das noch unverformte Dichtelement 24, wie beispielsweise in Fig. 4B dargestellt. An dieser Stelle hat die Kraft F den Wert Null, da noch keine Verformung erfolgt bzw. keine Kraft auf das Dichtelement 24 ausgeübt wird.

Zur Verformung bzw. (elastischen) Deformation des Dichtelements 24 ist zunächst vorzugsweise nur eine geringe Kraft erforderlich. Bei zunehmender Verformung steigt die Kraft zunächst leicht an, wie in Fig. 5A dargestellt. Dies ist erkennbar an dem nur leichten Anstieg der Kraft F von der Stelle H=D zur Stelle H=H1. Kleinere Werte von H korrespondieren hierbei zu einer stärkeren Verformung des Dichtelements 24, da bei kleineren Werten von H und entsprechend einer axialen Zustellung des Stößels 29 in Richtung des Dichtelements 24 und/oder Aufnahmeraums 27 das Volumen des Aufnahmeraums 27 verringert wird und entsprechend das Dichtelement 24 weiter bzw. fester in den Aufnahmeraum 27 eingedrückt wird und sich hierbei verformt.

Aufgrund der Elastizität des Dichtelements 24 ist die zur reinen Formänderung bzw. Deformation benötigte Kraft verhältnismäßig gering.

Bei abnehmender Höhe H verringert sich das Volumen des Aufnahmeraums 27 und damit der Füllgrad, also das Verhältnis zwischen dem Volumen des Dichtelements 24 und des Aufnahmeraums 27.

Die Kraft F erhöht sich mit abnehmender Höhe H bzw. abnehmendem Volumen des Aufnahmeraums 27.

Insbesondere setzt bei weiter abnehmender Höhe H eine Kompression des Dichtelements 24 ein, wie bereits erläutert. Bei Einsetzen der Kompression erhöht sich insbesondere die Steigung der Kraft-Weg-Kurve, da zur Kompression des Dichtelements 24 eine größere Kraft nötig ist als zur reinen Formänderung.

Die Verformung, insbesondere das Einsetzen der Kompression, ist daher insbesondere aus der Kraft-Weg-Kurve bzw. deren Verlauf, insbesondere deren Steigung und/oder Krümmung, ablesbar bzw. bestimmbar.

Insbesondere ändert sich die Steigung und/oder Krümmung der Kraft-Weg-Kurve bei Einsetzen der Volumenkompression des Dichtelements 24. Dies erfolgt beim Darstellungsbeispiel aus Fig. 5A insbesondere etwa zwischen den dort eingezeichneten Positionen H1 und H2 bzw. Werten F1 und F2. Wird die Höhe H noch weiter verringert, so erfolgt im Wesentlichen bzw. überwiegend eine Volumenkompression. Hierfür ist eine hohe Kraft erforderlich, sodass im linken Bereich in Fig. 5A hohe Kräfte F erreicht werden bzw. die Kraft-Weg-Kurve eine hohe Steigung aufweist bzw. (zu kleineren Werten von H hin) steil ansteigt.

Das Einsetzen der Volumenkompression korrespondiert vorzugsweise zu der stärksten Krümmung der Kraft-Weg-Kurve. Bei Einsetzen der Volumenkompression kann die Kraft-Weg-Kurve auch einen Knick oder dergleichen aufweisen. Insbesondere kann das Einsetzen der Volumenkompression daher durch eine Ableitung der Kraft-Weg-Kurve zuverlässig bzw. genau bestimmt werden, beispielsweise dadurch, dass die Ableitung der Kraft-Weg-Kurve beim Einsetzen der Volumenkompression eine Unstetigkeit oder sonstige starke Veränderung des Verlaufs aufweist.

Vorzugsweise wird zur Bestimmung des Montageparameters M anhand der Kraft-Weg-Kurve bestimmt oder festgestellt, wann eine Volumenkompression des Dichtelements 24 einsetzt bzw. ein spezifischer Kennwert K bestimmt, bei dem die Volumenkompression des Dichtelements 24 einsetzt bzw. der zu einem Einsetzen der Volumenkompression korreliert.

Der spezifische Kennwert K ist mit anderen Worten insbesondere derjenige Wert des Kennwerts, bei dem die Volumenkompression des Dichtelements 24 einsetzt.

Die Volumenkompression setzt nach den vorhergehenden Erläuterungen insbesondere an dem Punkt ein, an dem die Kraft-Weg-Kurve die stärkste Krümmung und/oder einen Knick aufweist. Der spezifische Kennwert K entspricht daher vorzugsweise der Kraft, bei der die Kraft-Weg-Kurve die (zumindest im Wesentlichen) stärkste Krümmung und/oder einen Knick aufweist, und korreliert beim Darstellungsbeispiel gemäß Fig. 5A mit einer bei der Montage gewünschten bzw. bevorzugten Höhe HM, die ihrerseits zu dem gewünschten Montageparameter M korreliert.

Für unterschiedliche Dichtelemente 24 bzw. Chargen von Dichtelementen 24 können sich unterschiedliche Verläufe der Kraft-Weg-Kurve und damit unterschiedliche spezifische Kennwerte K bzw. Höhe HM bzw. Montageparameter M ergeben.

Der Montageparameter M wird vorzugsweise so gewählt bzw. bestimmt, dass er zu einer einsetzenden Volumenkompression des Dichtelements 24 korrespondiert. Entsprechend den obigen Erläuterungen wird der Montageparameter M daher vorzugsweise so gewählt, dass die Höhe des Aufnahmeraums 27 bei der fertig montierten Abgabevorrichtung 1 zumindest im Wesentlichen der Höhe HM entspricht, bei der die Kraft-Weg-Kurve die stärkste Krümmung und/oder einen Knick aufweist. Insbesondere entspricht der Montageparameter M derjenigen Höhe HM des Aufnahmeraums 27, bei der das Volumen des (verformten) Dichtelements 24 dem Volumen des Aufnahmeraums 27 entspricht bzw. ein Füllgrad von zumindest im Wesentlichen 100 % erreicht ist.

Der Montageparameter M kann jedoch auch so gewählt bzw. bestimmt werden, dass er zu einer anderen Verformung bzw. einem anderen Verformungswert des Dichtelements 24 korrespondiert, beispielsweise zu einem Verformungswert, bei dem die Volumenkompression noch nicht eingesetzt hat bzw. bei dem das Dichtelement 24 noch nicht komprimiert ist und/oder bei dem das Dichtelement 24 lediglich oder zumindest überwiegend elastisch deformiert ist. Vorzugsweise kann eine definierte Differenz zwischen den zu dem Montageparameter M korrespondierenden Verformungswert und dem Verformungswert, der zu einer einsetzenden Volumenkompression korrespondiert, gewählt werden. Ebenso ist es möglich, den Montageparameter M so zu wählen bzw. zu bestimmen, dass er zu einer Verformung bzw. einem Verformungswert des Dichtelements 24 korrespondiert, bei dem die Volumenkompression bereits eingesetzt hat bzw. das Dichtelement 24 bereits komprimiert ist.

Der Montageparameter M kann also auch zu anderen Füllgraden korrespondieren bzw. einem anderen Füllgrad entsprechen als 100 %, beispielsweise einem (beliebigen) Füllgrad von mehr als 95 % und/oder weniger als 105 %. Dies kann - in Abhängigkeit von dem Material des Dichtelements 24 - vorteilhaft sein bzw. zu einer besseren Abdichtung und/oder Lebensdauer führen als ein Füllgrad von 100 %.

Vorzugsweise wird für jede Charge anhand einer Stichprobe bestimmt, unter welchen Bedingungen bzw. bei welchem spezifischen Kennwert K, insbesondere bei welcher Kraft in der Kraft-Weg-Kurve, der Verformungswert einen gewünschten Schwellwert erreicht oder überschreitet. Anschließend wird der Montageparameter M so gewählt, dass bei der Montage eines Dichtelements 24 der Charge der Verformungswert den Schwellwert erreicht oder überschreitet. Der Montageparameter M korrespondiert daher insbesondere zu der entsprechenden Höhe HM bzw. dem spezifischen Kennwert K. Die Wahl des Montageparameters M erfolgt vorzugsweise dadurch, dass bei der bzw. durch die Position, in der das Fixierelement 25 an dem Führungselement 23 befestigt bzw. das Anlageelement 26 in einer Position festgelegt wird, das gleiche Volumen des Aufnahmeraums 27 realisiert wird, wie dies der Fall ist, wenn bei der Bestimmung des Montageparameters M mittels der Verformung des Stößels 29 der Verformungswert zu dem Schwellwert korrespondiert bzw. der Verformungswert den Schwellwert annimmt. Mit anderen Worten wird der Montageparameter M vorzugsweise so gewählt, dass die Höhe (und damit insbesondere das Volumen) des Aufnahmeraums 27 der fertig montierten Abgabevorrichtung 1 den gleichen Wert hat wie der Weg bzw. die Höhe HM in der Kraft-Weg-Kurve, der/die dem spezifischen Kennwert K entspricht.

Zur Bestimmung bzw. bei der Bestimmung des Montageparameters M wird der Verformungswert, zu dem der gewählte bzw. bestimmte Montageparameter M korrespondiert, vorzugsweise überschritten. Beispielsweise kann der in Fig. 5A dargestellte Wert F2 die maximale Kraft sein, die bei der Bestimmung des Montageparameters M auf das Dichtelement 24 ausgeübt wird und der in Fig. 5A dargestellte Montageparameter M der ausgewählte bzw. bestimmte Montageparameter M sein.

Vorzugsweise wird zur Bestimmung des Montageparameters M die Kraft-Weg-Kurve also über eine Höhe H hinaus durchfahren, zu der der Montageparameter M korrespondiert. Dies ist insbesondere dadurch bedingt, dass die stärkste Krümmung bzw. Steigung erst dann zuverlässig bestimmbar ist, wenn der Punkt der stärksten Krümmung bzw. Steigung bereits überschritten wurde.

Bei dem bisher erläuterten Chargenverfahren wird, wie voranstehend beschrieben, zunächst in einem separaten Prozess der Montageparameter M für eine Charge von Dichtelementen 24, insbesondere anhand einer Stichprobe, bestimmt und anschließend bei der Montage der Dichtelemente 24 der Charge verwendet.

Die Trennung der Schritte der Bestimmung des Montageparameters M und der tatsächlichen Montage einer Abgabevorrichtung 1 ist jedoch nicht zwingend erforderlich.

Gemäß einem weiteren, auch unabhängig realisierbaren Aspekt, der nachfolgend beschrieben wird, betrifft die vorliegende Erfindung nämlich auch ein Verfahren zur Montage von Abgabevorrichtungen 1, bei dem zunächst für ein Dichtelement 24 ein Montageparameter M bestimmt wird und das Dichtelement 24 unmittelbar nach der Bestimmung des Montageparameters M in dem Aufnahmeraum 27 fixiert wird bzw. wobei die Montage der Abgabevorrichtung 1 unmittelbar nach der Bestimmung des Montageparameters M erfolgt. Hierbei wird das Dichtelement 24 im Gegensatz zu dem voranstehend beschriebenen Chargenverfahren vorzugsweise in der Abgabevorrichtung 1 bzw. dem Aufnahmeraum 27 fixiert bzw. festgesetzt, in der/dem auch die Bestimmung des Montageparameters M erfolgt. Insbesondere erfolgt die Bestimmung des Montageparameters M und die Montage der Abgabevorrichtung 1 zeitlich unmittelbar aufeinander und/oder in derselben Anlage. Dieses Verfahren wird insbesondere als Individualverfahren bezeichnet.

Das Individualverfahren ist insbesondere dann vorteilhaft, wenn bei einer, insbesondere automatischen bzw. serienmäßigen, Montage von Abgabevorrichtungen 1 ohne eine vorherige Bestimmung eines Montageparameters M gearbeitet werden soll. Dies kann insbesondere dann der Fall sein, wenn die Dichtelemente 24 einer Charge bzw. deren signifikante Größen zu stark voneinander abweichen. In diesem Fall kann möglicherweise kein gemeinsamer Montageparameter M für alle Dichtelemente 24 der Charge bestimmt werden bzw. durch Bestimmung eines einzelnen Montageparameters M für alle Dichtelemente 24 der Charge keine zuverlässige Abdichtung bzw. Dichtwirkung sichergestellt werden.

Dennoch ist das nachfolgend näher beschriebene Individualverfahren grundsätzlich auch anwendbar, wenn die Dichtelemente 24 in Chargen vorliegen und die Dichtelemente 24 einer Charge voneinander so wenig abweichen, dass grundsätzlich auch das Chargenverfahren anwendbar wäre.

Die Bestimmung des Montageparameters M erfolgt bei dem Individualverfahren vorzugsweise - zumindest im Wesentlichen - so wie voranstehend für das Chargenverfahren in Zusammenhang mit Fig. 4A-E und 5 beschrieben, insbesondere anhand der Bestimmung des Verformungsverhaltens und/oder Reibungsverhaltens des Dichtelements 24 während einer Verformung in dem Aufnahmeraum 27 bzw. anhand des Verlaufs einer Kraft-Weg-Kurve.

Die voranstehenden Ausführungen bezüglich der Bestimmung des Montageparameters M gelten daher vorzugsweise auch für das Individualverfahren.

Im Gegensatz zu dem in Fig. 4A-D dargestellten Stößel 29, der bei dem Chargenverfahren verwendet wird, weist bei dem Individualverfahren der Stößel 29 vorzugsweise kein Zentralelement 30 auf bzw. wird der Stößel 29 nicht durch das Anlageelement 26 und/oder das Dichtelement 24 hindurchgeführt.

Vorzugsweise wird jedoch - an Stelle des Zentralelements 30 - bei bzw. während der Bestimmung des Montageparameters M das Förderelement 9 bereits durch das Anlageelement 26 und/oder das Dichtelement 24 geführt bzw. in den Kanal 23b eingeführt. In diesem Sinne wird vorzugsweise das Zentralelement 30 durch das Förderelement 9 ersetzt. Vorzugsweise gelten daher die voranstehenden Ausführungen bezüglich des Zentralelements 30 analog für das Verfahren, bei dem das Förderelement 9 statt des Zentralelements 30 verwendet wird.

Wie beim Chargenverfahren beschrieben wird auch beim Individualverfahren zur Bestimmung des Montageparameters M das Dichtelement 24 verformt, wobei bei der Bestimmung des Montageparameters M die gewünschte Verformung des Dichtelements 24 bzw. der gewünschte Verformungswert des Dichtelements 24 überschritten wird.

Dementsprechend wird, nachdem die Bestimmung des Montageparameters M erfolgt bzw. abgeschlossen ist, der Stößel und damit das Fixierelement 25 bzw. Anlageelement 26 vorzugsweise wieder ein Stück zurückbewegt, also insbesondere in die dem Dichtelement 24 abgewandte Richtung des Fixierelements 25 bzw. Anlageelements 26 bewegt. Insbesondere wird hierdurch das Fixierelement 25 bzw. Anlageelement 26 so positioniert, dass der Montageparameter M realisiert wird bzw. die Position des Fixierelements 25 und/oder Anlageelements 26 dem bestimmten Montageparameter M entspricht. Hierdurch wird erreicht, dass das Dichtelement 24 den gewünschten Verformungswert annimmt bzw. der gewünschte Verformungswert realisiert wird.

In dieser Position wird dann vorzugsweise das Fixierelement 25 bzw. Anlageelement 26 bzw. Dichtelement 24 fixiert, insbesondere indem das Fixierelement 25 an dem Führungselement 23 bzw. Führungsabschnitt 23a und/oder dem Anlageelement 26 fixiert bzw. befestigt wird. Die Befestigung des Fixierelements 25 erfolgt vorzugsweise wie weiter oben beschrieben, beispielsweise also durch Crimpen.

Insbesondere erfolgt beim Individualverfahren das Fixieren bzw. die Montage des Dichtelements 24 bzw. der Abgabevorrichtung 1 unmittelbar nach dem Bestimmen des Montageparameters M und/oder in der gleichen Anlage wie die Bestimmung des Montageparameters M.

Im Unterschied zum Chargenverfahren wird bei dem Individualverfahren vorzugsweise bereits während der Bestimmung des Montageparameters M das Fixierelement 25 mitgeführt. Bei der Ausführung des Fixierelements 25 und Anlageelements 26 als separate Teile, wie in den Figuren dargestellt, wird beim Chargenverfahren, wie voranstehend erläutert, vorzugsweise lediglich das Anlageelement 26 in den Aufnahmeraum 27 eingeführt bzw. lediglich mit dem Anlageelement 26 das Dichtelement 24 verformt. Beim Individualverfahren wird hingegen vorzugsweise bei der Bestimmung des Montageparameters M bzw. Verformung des Dichtelements 24 mittels des Anlageelements 26 das Fixierelement 25 bereits mitgeführt, sodass unmittelbar nach Bestimmung des Montageparameters M und/oder korrekten Positionierung des Anlageelements 26 und/oder Fixierelements 25 das Fixierelement 25 in dieser Position fixiert bzw. an dem Führungselement 23 befestigt werden kann.

Ein "Arzneimittel" im Sinne der vorliegenden Erfindung ist ein Stoff oder eine Zubereitung aus Stoffen, der/die zur Anwendung im oder am menschlichen oder tierischen Körper bestimmt ist und als Mittel mit Eigenschaften zur Heilung oder Linderung oder zur Verhütung menschlicher oder tierischer Krankheiten oder krankhafter Beschwerden bestimmt ist oder der/die am menschlichen oder tierischen Körper angewendet oder einem Menschen oder Tier verabreicht werden kann, um entweder die physiologischen Funktionen durch eine pharmakologische, immunologische oder metabolische Wirkung wiederherzustellen, zu korrigieren oder zu beeinflussen oder eine medizinische Diagnose zu erstellen.

Ein "Montageparameter" im Sinne der vorliegenden Erfindung ist vorzugsweise ein insbesondere einstellbarer und/oder geometrischer Wert, der bei der Montage der Abgabevorrichtung vorgegeben wird und/oder einzuhalten ist bzw. bei der fertig montierten Abgabevorrichtung zu realisieren ist bzw. realisiert wird. Vorzugsweise kann der Montageparameter in den Einstellungen eines Montageablaufs bevorzugt variabel vorgegeben werden. Vorzugsweise ist der Montageparameter ein geometrischer Parameter, insbesondere eine, vorzugsweise relative, Position eines Bauteils oder ein Streckenmaß, wie eine Höhe des Aufnahmeraums, der Abgabevorrichtung. Insbesondere ist der Montageparameter eine Position eines Fixierelements relativ zu einem Führungselement, wobei das Dichtelement mittels des Fixierelements in dem Führungselement bzw. einem Aufnahmeraum des Führungselements fixiert wird. Mit anderen Worten wird diese Position durch den Montageparameter bzw. dessen Wert vorzugsweise definiert. Es ist auch möglich, dass der Montageparameter nur mittelbar zu einer Position des Fixierelements bzw. Anlageelements korrespondiert, beispielsweise indem der Montageparameter durch einen Zustellweg definiert oder gebildet ist, der von einem Teil einer Vorrichtung zur Montage der Abgabevorrichtung bzw. zur Befestigung des Fixierelements an dem Führungselement durchfahren wird o. dgl.

Ein "Verformungswert" im Sinne der vorliegenden Erfindung ist vorzugsweise ein Maß dafür, wie stark das Dichtelement verformt, insbesondere komprimiert, ist. Insbesondere ist der Verformungswert ein Wert, der zu einer Verformung des Dichtelements korrespondiert bzw. eine Verformung des Dichtelements angibt. Der Verformungswert kann beispielsweise aus einem oder mehreren Messwerten bzw. Kennwerten bestimmt, berechnet und/oder abgeleitet sein. Der Verformungswert gibt vorzugsweise an, ob und/oder in welchem Maße das Dichtelement (elastisch) deformiert und/oder komprimiert ist. Insbesondere ist der Verformungswert eine (temporäre) Eigenschaft bzw. ein temporärer Zustand des Dichtelements, der nicht direkt gemessen oder bestimmt werden kann, sondern der vorzugsweise aus einem gemessenen Kennwert bestimmt, berechnet oder abgeleitet wird.

Ein "Schwellwert" im Sinne der vorliegenden Erfindung ist vorzugsweise ein Wert bzw. Grenzwert, der zur Wahl bzw. Bestimmung des Montageparameters dient. Der Schwellwert ist insbesondere ein bestimmter Wert bzw. Grenzwert des Verformungswerts. Der Montageparameter wird vorzugsweise derart gewählt bzw. bestimmt, dass bei einer Fixierung des Dichtelements der Verformungswert den Schwellwert erreicht oder überschreitet. Vorzugsweise ist der Schwellwert vorgegeben oder vorgebbar. Vorzugsweise korrespondiert der Schwellwert zu einer einsetzenden Volumenkompression des Dichtelements bzw. ist der Schwellwert derjenige Wert des Verformungswerts, bei dem eine Volumenkompression des Dichtelements einsetzt. Abhängig vom Material des Dichtelements kann es jedoch auch wünschenswert bzw. vorteilhaft sein, als Schwellwert einen Wert zu wählen, der gezielt oberhalb oder unterhalb des Werts liegt, bei dem die Volumenkompression einsetzt.

Ein "Kennwert" im Sinne der vorliegenden Erfindung ist vorzugsweise ein Wert, der bestimmt bzw. gemessen wird, um den Verformungswert zu bestimmen, zu berechnen und/oder aus dem Kennwert abzuleiten. Der Kennwert kann beispielsweise eine, insbesondere gemessene, Kraft (insbesondere eine Druckkraft oder eine Reibungskraft), ein Druck oder eine Position sein. Insbesondere ist der Kennwert also ein Messwert, aus dem sich Rückschlüsse auf den Verformungswert ziehen lassen.

Ein "Druckverformungsrest" im Sinne der vorliegenden Erfindung ist vorzugsweise eine Materialeigenschaft eines Materials, aus dem ein Dichtelement hergestellt ist. Insbesondere ist der Druckverformungsrest ein Maß dafür, wie sich das Material, insbesondere ein Elastomer, bei lang andauernder und/oder konstanter Druckverformung und vorzugsweise anschließender Entspannung verhält. Die Bestimmung des Druckverformungsrests erfolgt vorzugsweise nach DIN ISO 815-1 :2016-09. Vorzugsweise wird zur Bestimmung des Druckverformungsrests ein zylindrischer Prüfkörper zusammengedrückt, insbesondere um 25 %, und bei einer bestimmten Temperatur über einen bestimmten Zeitraum so gelagert. Nach einer Entlastung, vorzugsweise 30 min nach der Entlastung, wird aus dem Vergleich zwischen der Höhe des Prüfkörpers vor dem Zusammendrücken und der Höhe des Prüfkörpers nach dem Entlasten die bleibende Verformung des Prüfkörpers ermittelt. Insbesondere bedeutet hierbei ein Druckverformungsrest von 0 %, dass der Prüfkörper seine ursprüngliche Höhe wieder voll erreicht hat. Ein Druckverformungsrest von 100 % bedeutet, dass der Körper während des Versuchs völlig verformt wurde und keine Rückstellung zeigt. Insbesondere ist der Druckverformungsrest der Quotient (L0 - L2) / (L0 - L1), wobei L0 die Höhe des Prüfkörpers vor der Prüfung ist, L1 die Höhe des Prüfkörpers während der Prüfung ist und L2 die Höhe des Prüfkörpers nach der Prüfung ist.

Ein "Kriechverhalten" bzw. "Kriechen" eines Materials bzw. Dichtelements im Sinne der vorliegenden Erfindung ist besondere das Verhalten des Materials bzw. Dichtelements unter konstanter Last. Insbesondere ist das Kriechverhalten bzw. Kriechen ein zeit- und/oder temperaturabhängiges plastisches Verformen und einer konstanten Last. Das Kriechverhalten bzw. Kriechen wird insbesondere durch den Kriechmodul bestimmt bzw. charakterisiert.

Eine "Fixierung" eines Dichtelements in einem Aufnahmeraum im Sinne der vorliegenden Erfindung ist insbesondere eine Festlegung bzw. Festsetzung des Dichtelements in dem Aufnahmeraum derart, dass sich das Dichtelement in dem Aufnahmeraum nicht weiter verformt bzw. verformen kann.

Die voranstehend erläuterten Aspekte und Merkmale können unabhängig voneinander, aber auch in verschiedenen Kombinationen realisierbar sein. Insbesondere können das Chargenverfahren und das Individualverfahren miteinander kombinierbar sein. Mit anderen Worten können Schritte, die im Zusammenhang mit dem Chargenverfahren erläutert wurden, auch Schritte des Individualverfahrens darstellen und umgekehrt.

### Bezugszeichenliste:

| | | | |
|---|---|---|---|
| 1 | Abgabevorrichtung | 26b | Anschlag |
| 2 | Arzneimittel | 26c | Einführabschnitt |
| 3 | Behälter | 27 | Aufnahmeraum |
| 4 | Fluidraum | 27a | Boden |
| 5 | Druckerzeuger | 28 | Zuführkanal |
| 6 | Halterung | 29 | Stößel |
| 7 | Antriebsfeder | 30 | Zentralelement |
| 8 | Sperrelement | 31 | Positioniereinrichtung |
| 8a | Auslösetaste | 31a | Helixstruktur |
| 9 | Förderelement | 31b | Rastelemente |
| 10 | Rückschlagventil | | |
| 11 | Druckkammer | A | Achse |
| 12 | Austragsdüse | B | Breite |
| 13 | Mundstück | D | Schnurstärke |
| 14 | Aerosol | F | Kraft |
| 15 | Zuluftöffnung | K | spezifischer Kennwert |
| 16 | (oberes) Gehäuseteil | H | Höhe |
| 17 | Innenteil | HM | Höhe für Montage |
| 17a | oberer Teil (Innenteil) | M | Montageparameter |
| 17b | unterer Teil (Innenteil) | | |
| 18 | (unteres) Gehäuseteil | | |
| 19 | Halteelement | | |
| 20 | Feder | | |
| 21 | Behälterboden | | |
| 22 | Anstechelement | | |
| 23 | Führungselement | | |
| 23a | Befestigungsabschnitt | | |
| 23b | Kanal | | |
| 23c | Ausnehmung | | |
| 24 | Dichtelement | | |
| 24a | Öffnung | | |
| 25 | Fixierelement | | |
| 25a | Gegenfläche | | |
| 26 | Anlageelement | | |
| 26a | Kontaktabschnitt 26a | | |

## Patentansprüche

1. Verfahren zur Montage von Abgabevorrichtungen (1) zur Abgabe eines Arzneimittels (2),
wobei die zu montierenden Abgabevorrichtungen (1) jeweils ein insbesondere axial bewegbares Förderelement (9) zur Förderung des Arzneimittels (2), ein Führungselement (23) zur Führung des Förderelements (9) und ein vorzugsweise ringförmiges Dichtelement (24) zur Abdichtung des Förderelements (9) gegen das Führungselement (23) aufweisen, wobei das Führungselement (23) einen Aufnahmeraum (27) zur Aufnahme des Dichtelements (24) zumindest partiell begrenzt und das Dichtelement (24) in dem Aufnahmeraum (27) fixierbar ist,
wobei verfahrensgemäß ein vorzugsweise ringförmiges Dichtelement (24) in einem Aufnahmeraum (27) der Abgabevorrichtung (1) oder in einem Aufnahmeraum (27) in einer Prüfanlage, wobei der Aufnahmeraum in der Prüfanlage die gleichen Abmessungen wie der Aufnahmeraum der Abgabevorrichtung aufweist, angeordnet wird,
**dadurch gekennzeichnet,**
**dass** das in dem Aufnahmeraum (27) angeordnete Dichtelement (24) verformt und dabei anhand des Verformungsverhaltens und/oder Reibungsverhaltens des Dichtelements (24) während der Verformung ein Montageparameter (M) bestimmt wird,
wobei das Dichtelement (24) oder ein anderes zu montierendes Dichtelement (24) unter Verwendung des Montageparameters (M) in einem Aufnahmeraum (27) einer Abgabevorrichtung (1) fixiert wird.

2. Verfahren nach Anspruch 1, **dadurch gekennzeichnet, dass** mehrere Dichtelemente (24) in Chargen vorliegen und für jede Charge separat anhand einer Stichprobe von Dichtelementen (24) der jeweiligen Charge ein Montageparameter (M) bestimmt wird, wobei der für diese Charge bestimmte Montageparameter (M) bei jeder Fixierung eines Dichtelements (24) dieser Charge in einem Aufnahmeraum (27) verwendet wird.

3. Verfahren nach Anspruch 2, **dadurch gekennzeichnet, dass** der Montageparameter (M) jeweils so gewählt wird, dass sich bei fertig montierten Abgabevorrichtungen (1) für unterschiedliche Chargen von Dichtelementen (24) jeweils der gleiche Verformungswert des Dichtelements (24) ergibt, vorzugsweise wobei der Verformungswert ein Maß dafür ist, wie stark das Dichtelement (24) verformt, insbesondere komprimiert, ist.

4. Verfahren nach Anspruch 2 oder 3, **dadurch gekennzeichnet, dass** zur Bestimmung des Montageparameters (M) einer Charge zunächst für jedes Dichtelement (24) der Stichprobe separat ein Montageparameter (M) bestimmt wird und ein Mittelwert dieser separat bestimmten Montageparameter (M) als Montageparameter (M) für alle Dichtelemente (24) dieser Charge festgelegt wird.

5. Verfahren nach einem der Ansprüche 2 bis 4, **dadurch gekennzeichnet, dass** zur Bestimmung des Montageparameters (M) ein Zentralelement (30) durch eine Öffnung (24a) des Dichtelements (24) bzw. durch den Aufnahmeraum (27) geführt wird, wobei das Volumen des Aufnahmeraums (27) durch das Zentralelement (30) verringert oder begrenzt wird.

6. Verfahren nach einem der Ansprüche 2 bis 5, **dadurch gekennzeichnet, dass** die Anzahl von Dichtelementen (24) der Stichprobe weniger als 50 %o, bevorzugt weniger als 20 %o, insbesondere weniger als 10 %o, besonders bevorzugt weniger als 5 %o, ganz besonders bevorzugt weniger als 2 %o, der Anzahl von Dichtelementen (24) der Charge beträgt und/oder dass die Volumina der Dichtelemente (24) einer Charge um weniger als 10 %, vorzugsweise weniger als 5 %, besonders bevorzugt weniger als 4 %, vom mittleren Volumen der Dichtelemente (24) der Charge abweichen.

7. Verfahren nach Anspruch 1, **dadurch gekennzeichnet, dass** das Dichtelement (24) unmittelbar nach dem Verformen und Bestimmen des Montageparameters (M) mittels eines auf das Dichtelement (24) wirkenden Fixierelements (25) bzw. Anlageelements (26) in dem Aufnahmeraum (27) endgültig fixiert wird.

8. Verfahren nach Anspruch 1 oder 7, **dadurch gekennzeichnet, dass** der Montageparameter (M) für jedes zu montierende Dichtelement (24) separat bestimmt wird und jeweils nur bei der Fixierung desjenigen Dichtelements (24) verwendet wird, für das er bestimmt wurde.

9. Verfahren nach einem der voranstehenden Ansprüche, wobei der Montageparameter (M) ein, insbesondere einstellbarer und/oder geometrischer, Wert ist, der bei der Montage der Abgabevorrichtung (1) vorgegeben wird und/oder realisiert wird, vorzugsweise wobei der Montageparameter (M) in den Einstellungen eines Montageablaufs bevorzugt variabel vorgebbar ist.

10. Verfahren nach einem der voranstehenden Ansprüche, **dadurch gekennzeichnet,**
**dass** der Montageparameter (M) eine, insbesondere axiale, Position eines Fixierelements (25) bzw. Anlageelements (26) relativ zu dem Aufnahmeraum (27) bzw. einem Führungselement (23) der Abgabevorrichtung (1) darstellt oder
**dass** eine, insbesondere axiale, Position eines Fixierelements (25) bzw. Anlageelements (26) relativ zu dem Aufnahmeraum (27) bzw. einem Führungselement (23) der Abgabevorrichtung (1) durch den Montageparameter (M) definiert wird,
insbesondere wobei das Führungselement (23) den Aufnahmeraum (27) zumindest partiell begrenzt und das Fixierelement (25) bzw. Anlageelement (26) zum Fixieren des Dichtelements (24) in dem Aufnahmeraum (27) verwendet wird.

11. Verfahren nach einem der voranstehenden Ansprüche, **dadurch gekennzeichnet, dass** der Montageparameter (M) zu einem Volumen des Aufnahmeraums (27) und/oder zu einem Verformungswert, insbesondere einer einsetzenden Volumenkompression, des Dichtelements (24) korrespondiert.

12. Verfahren nach einem der voranstehenden Ansprüche, **dadurch gekennzeichnet, dass** der Montageparameter (M) so gewählt bzw. bestimmt wird, dass bei einer Fixierung des Dichtelements (24) in dem Aufnahmeraum (27) ein zu einer Verformung des Dichtelements (24) korrespondierender Verformungswert einen Schwellwert erreicht oder überschreitet, vorzugsweise wobei der Verformungswert ein Maß dafür ist, wie stark das Dichtelement (24) verformt ist, und der Schwellwert zu einer einsetzenden Volumenkompression des Dichtelements (24) korrespondiert.

13. Verfahren nach einem der voranstehenden Ansprüche, **dadurch gekennzeichnet, dass** zur Bestimmung des Verformungsverhaltens und/oder Reibungsverhaltens des jeweiligen Dichtelements (24) bei unterschiedlichen Verformungen jeweils ein Kennwert gemessen wird, der zu einem Verformungswert des Dichtelements (24) korrespondiert und/oder aus dem der Verformungswert des Dichtelements (24) berechnet, bestimmt und/oder abgeleitet werden kann.

14. Verfahren nach Anspruch 13, **dadurch gekennzeichnet, dass** der Kennwert eine Kraft, insbesondere eine Reibungskraft oder eine Druckkraft, ein Druck oder eine Position ist.

15. Verfahren nach einem der voranstehenden Ansprüche, **dadurch gekennzeichnet, dass** zur Bestimmung des Verformungsverhaltens des jeweiligen Dichtelements (24) bei unterschiedlichen Verformungen jeweils die zur Verformung benötigte Kraft bestimmt wird.

16. Verfahren nach einem der voranstehenden Ansprüche, **dadurch gekennzeichnet, dass** zur Bestimmung des Reibungsverhaltens des jeweiligen Dichtelements (24) eine Begrenzung des Aufnahmeraums (27) und das Dichtelement (24) relativ zueinander bewegt werden und bei unterschiedlichen Verformungen jeweils die Reibungskraft zwischen dem Dichtelement (24) und der Begrenzung bestimmt wird.

17. Verfahren nach einem der voranstehenden Ansprüche, **dadurch gekennzeichnet, dass** zur Bestimmung des Verformungsverhaltens bzw. Reibungsverhaltens und/oder zur Bestimmung des Montageparameters (M) eine Kraft-Weg-Kurve aufgenommen wird, vorzugsweise wobei anhand des Verlaufs bzw. der Krümmung der Kraft-Weg-Kurve der Montageparameter (M) bestimmt wird.

18. Verfahren nach einem der voranstehenden Ansprüche, **dadurch gekennzeichnet, dass** das Dichtelement (24) zur Abdichtung eines Förderelements (9) zur Förderung des Arzneimittels (2) gegen ein den Aufnahmeraum (27) aufweisendes oder bildendes Führungselement (23), in dem das Förderelement (9) geführt ist, ausgebildet ist.

19. Abgabevorrichtung (1) zur Abgabe eines Arzneimittels (2), mit einem insbesondere axial bewegbaren Förderelement (9) zur Förderung des Arzneimittels (2), einem Führungselement (23) zur Führung des Förderelements (9) und einem insbesondere ringförmigen Dichtelement (24) zur Abdichtung des Förderelements (9) gegen das Führungselement (23), wobei das Führungselement (23) einen Aufnahmeraum (27) zur Aufnahme des Dichtelements (24) zumindest partiell begrenzt und das Dichtelement (24) in dem Aufnahmeraum (27) mittels eines an dem Führungselement (23) befestigbaren Fixierelements (25) bzw. Anlageelements (26) fixierbar ist,
**dadurch gekennzeichnet,**
**dass** das Fixier- bzw. Anlageelement (25, 26) in verschiedenen diskreten Positionen an dem Führungselement (23) befestigbar ist und dadurch das Dichtelement (24) mit einem zuvor bestimmten Montageparameter (M) fixierbar ist, wobei das Fixier- bzw. Anlageelement (25, 26) in den verschiedenen diskreten Positionen an dem Führungselement (23) einrastbar ist und wobei die verschiedenen diskreten Positionen axial verschiedene Positionen des Fixier- bzw. Anlageelements (25, 26) relativ zu dem Führungselement (23) sind.

20. Abgabevorrichtung nach Anspruch 19, **dadurch gekennzeichnet, dass** das Fixier- bzw. Anlageelement (25, 26) und/oder das Führungselement (23) eine Positioniereinrichtung (31) zur Positionierung des Fixier- bzw. Anlageelements (25, 26) relativ zu dem Führungselement (23) aufweist bzw. aufweisen, wobei die Positioniereinrichtung (31) eine oder mehrere Rastelemente (31b) aufweist oder hierdurch gebildet ist.

21. Abgabevorrichtung nach Anspruch 20, **dadurch gekennzeichnet, dass** die Positioniereinrichtung (31) eine schiefe Ebene oder Helixstruktur (31a) aufweist oder hierdurch gebildet ist.

22. Abgabevorrichtung nach einem der Ansprüche 19 bis 21, **dadurch gekennzeichnet, dass** die Drehlage des Fixier- bzw. Anlageelements (25, 26), insbesondere relativ zu dem Führungselement (23) bzw. der Abgabevorrichtung (1), die axiale Position des Fixier- bzw. Anlageelements (25, 26) festlegt.

23. Abgabevorrichtung nach einem der Ansprüche 19 bis 22, **dadurch gekennzeichnet, dass** die Position des Fixier- bzw. Anlageelements (25, 26) eine Verformung des Dichtelements (24) und/oder eine Größe des Aufnahmeraums (27) definiert, sodass die Verformung des Dichtelements (24) und/oder die Größe des Aufnahmeraums (27) durch eine Variation der Position des Fixierelements (25) bzw. Anlageelements (26) variierbar ist/sind.

## Claims

1. A method for assembling dispensing devices (1) for dispensing a medicament (2),
wherein the dispensing devices (1) which are to be assembled each comprise
a conveying element (9) which can be moved in particular axially for conveying the medicament (2),
a guide element (23) for guiding the conveying element (9), and
a preferably ring-shaped sealing element (24) for sealing the conveying element (9) against the guide element (23), wherein the guide element (23) at least partially delimits a receiving space (27) for receiving the sealing element (24) and the sealing element (24) can be fixed in the receiving space (27),
wherein according to the method, a preferably ring-shaped sealing element (24) is arranged in a receiving space (27) of the dispensing device (1) or in a receiving space (27) in a test system, wherein the receiving space in the test system has the same dimensions as the receiving space of the dispensing device,
**characterized in that**
the sealing element (24) arranged in the receiving space (27) is deformed, and an assembling process parameter (M) is determined in the process on the basis of the deformation behavior and/or friction behavior of the sealing element (24) during the deformation,
wherein the sealing element (24), or a different sealing element (24) to be installed, is fixed in a receiving space (27) of a dispensing device (1) using the assembling process parameter (M).

2. The method according to claim 1, **characterized in that** a plurality of sealing elements (24) is provided in batches, and an assembling process parameter (M) is determined separately for each batch using a random sample of sealing elements (24) of the given batch, wherein the assembling process parameter (M) determined for this batch is used for each fixing of a sealing element (24) of the batch in a receiving space (27).

3. The method according to claim 2, **characterized in that** the assembling process parameter (M) is selected in each case in such a way that the same deformation value of the sealing element (24) results for completely assembled dispensing devices (1) for different batches of sealing elements (24), preferably wherein the deformation value is a measure of how much the sealing element (24) is deformed, and in particular compressed.

4. The method according to claim 2 or 3, **characterized in that**, to determine the assembling process parameter (M) of a batch, an assembling process parameter (M) is first determined separately for each sealing element (24) in the random sample, and a mean value of these assembling process parameters (M) determined separately is defined as the assembling process parameter (M) for all sealing elements (24) of this batch.

5. The method according to any of claims 2 to 4, **characterized in that**, to determine the assembling process parameter (M), a central element (30) is guided through an opening (24a) in the sealing element (24) and/or through the receiving space (27), and the volume of the receiving space (27) is reduced or delimited by the central element (30).

6. The method according to any of claims 2 to 5, **characterized in that** the number of sealing elements (24) in the random sample is less than 50‰, preferably less than 20‰, in particular less than 10‰, particularly preferably less than 5‰, very particularly preferably less than 2‰ of the number of sealing elements (24) of the batch, and/or **in that** the volumes of the sealing elements (24) of a batch deviate by less than 10%, preferably less than 5%, particularly preferably less than 4%, from the mean volume of the sealing elements (24) of the batch.

7. The method according to claim 1, **characterized in that** immediately after the deformation and determination of the assembling process parameter (M), the sealing element (24) is finally fixed in the receiving space (27) by means of a fixing element (25) or contact element (26) acting on the sealing element (24).

8. The method according to claim 1 or 7, **characterized in that** the assembling process parameter (M) is determined separately for each sealing element (24) to be installed and is used in each case only when fixing the sealing element (24) for which it was determined.

9. The method according to any of the preceding claims, wherein the assembling process parameter (M) is a particularly adjustable and/or geometric value which is prespecified for and/or implemented in the assembly of the dispensing device (1), preferably wherein the assembling process parameter (M) can be prespecified in a preferably variable manner in the configurations of an assembling process.

10. The method according to any of the preceding claims, **characterized in**
**that** the assembling process parameter (M) is a position, in particular an axial position, of a fixing element (25) or contact element (26) relative to the receiving space (27) and/or to a guide element (23) of the dispensing device (1), or
**that** a position, in particular an axial position, of a fixing element (25) or contact element (26) relative to the receiving space (27) and/or to a guide element (23) of the dispensing device (1) is defined by the assembling process parameter (M),
in particular wherein the guide element (23) at least partially delimits the receiving space (27), and the fixing element (25) or contact element (26) is used for fixing the sealing element (24) in the receiving space (27).

11. The method according to any of the preceding claims, **characterized in that** the assembling process parameter (M) corresponds to a volume of the receiving space (27) and/or to a deformation value, in particular to an onset of volume compression, of the sealing element (24).

12. The method according to any of the preceding claims, **characterized in that** the assembling process parameter (M) is selected or determined in such a manner that, when the sealing element (24) is fixed in the receiving space (27), a deformation value corresponding to a deformation of the sealing element (24) reaches or exceeds a threshold value, preferably wherein the deformation value is a measure of how much the sealing element (24) is deformed, and the threshold value corresponds to an onset of volume compression of the sealing element (24).

13. The method according to any one of the preceding claims, **characterized in that**, to determine the deformation behavior and/or friction behavior of each of the sealing elements (24) with different deformations, in each case a characteristic value is measured which corresponds to a deformation value of the sealing element (24) and/or from which the deformation value of the sealing element (24) can be calculated, determined, and/or derived.

14. The method according to claim 13, **characterized in that** the characteristic value is a force, in particular a frictional force or a compressive force, a pressure, or a position.

15. The method according to any of the preceding claims, **characterized in that**, to determine the deformation behavior of each of the sealing elements (24), the force required for deformation is determined in each case for different deformations.

16. The method according to any of the preceding claims, **characterized in that**, to determine the friction behavior of each of the sealing elements (24), the sealing element (24) and a boundary of the receiving space (27) are moved relative to one another, and the frictional force between the sealing element (24) and the boundary is determined in each case for different deformations.

17. The method according to any of the preceding claims, **characterized in that** a force/displacement curve is recorded to determine the deformation behavior or friction behavior and/or to determine the assembling process parameter (M), preferably wherein the assembling process parameter (M) is determined on the basis of the profile and/or the curvature of the force/displacement curve.

18. The method according to any of the preceding claims, **characterized in that** the sealing element (24) is designed to seal a conveying element (9) for conveying the medicament (2) against a guide element (23) which comprises or forms the receiving space (27) and in which the conveying element (9) is guided.

19. A dispensing device (1) for dispensing a medicament (2), having a conveying element (9) which can be moved in particular axially for conveying the medicament (2), a guide element (23) for guiding the conveying element (9), and a particularly ring-shaped sealing element (24) for sealing the conveying element (9) against the guide element (23), wherein the guide element (23) at least partially delimits a receiving space (27) for receiving the sealing element (24), and the sealing element (24) can be fixed in the receiving space (27) by means of a fixing element (25) or contact element (26) which can be fastened to the guide element (23),
**characterized in that**
the fixing and/or contact element (25, 26) can be fastened to the guide element (23) in different discrete positions, and the sealing element (24) can thereby be fixed with a previously determined assembling process parameter (M), wherein the fixing and/or contact element (25, 26) can be locked on the guide element (23) in the different discrete positions and wherein the different discrete positions are axially different positions of the fixing and/or contact element (25, 26) relative to the guide element (23).

20. The dispensing device according to claim 19, **characterized in that** the fixing and/or contact element (25, 26) and/or the guide element (23) has/have a positioning device (31) for positioning the fixing and/or contact element (25, 26) relative to the guide element (23), wherein the positioning device (31) has or is formed by one or more locking elements (31b).

21. The dispensing device according to claim 20, **characterized in that** the positioning device (31) has or is formed by an inclined plane or helix structure (31a).

22. The dispensing device according to any of claims 19 to 21, **characterized in that** the rotational position of the fixing and/or contact element (25, 26), in particular relative to the guide element (23) or the dispensing device (1), defines the axial position of the fixing and/or contact element (25, 26).

23. The dispensing device according to any of claims 19 to 22, **characterized in that** the position of the fixing and/or contact element (25, 26) defines a deformation of the sealing element (24) and/or a size of the receiving space (27), such that the deformation of the sealing element (24) and/or the size of the receiving space (27) can be varied by varying the position of the fixing element (25) or contact element (26).

## Revendications

1. Procédé de montage de dispositifs de distribution (1) destinés à distribuer un médicament (2), dans lequel les dispositifs de distribution (1) à monter présentent respectivement un élément de convoyage (9) pouvant être déplacé en particulier axialement, destiné à convoyer le médicament (2), un élément de guidage (23) destiné à guider l'élément de convoyage (9) et un élément d'étanchéité (24) de préférence annulaire destiné à étanchéifier l'élément de convoyage (9) contre l'élément de guidage (23), dans lequel l'élément de guidage (23) délimite au moins partiellement un espace de réception (27) destiné à recevoir l'élément d'étanchéité (24) et l'élément d'étanchéité (24) peut être fixé dans l'espace de réception (27),
dans lequel selon le procédé, un élément d'étanchéité (24) de préférence annulaire est disposé dans un espace de réception (27) du dispositif de distribution (1) ou dans un espace de réception (27) dans une installation de contrôle, dans lequel l'espace de réception dans l'installation de contrôle présente les mêmes dimensions que l'espace de réception du dispositif de distribution,
**caractérisé en ce**
**que** l'élément d'étanchéité (24) disposé dans l'espace de réception (27) est déformé et ce faisant un paramètre de montage (M) est défini à l'aide du comportement de déformation et/ou du comportement de frottement de l'élément d'étanchéité (24) pendant la déformation,
dans lequel l'élément d'étanchéité (24) ou un autre élément d'étanchéité (24) à monter est fixé dans un espace de réception (27) d'un dispositif de distribution (1) en utilisant le paramètre de montage (M).

2. Procédé selon la revendication 1, **caractérisé en ce que** plusieurs éléments d'étanchéité (24) sont présents en lots et un paramètre de montage (M) est défini séparément pour chaque lot à l'aide d'un échantillon d'éléments d'étanchéité (24) du lot respectif, dans lequel le paramètre de montage (M) défini pour ledit lot est utilisé lors de chaque blocage d'un élément d'étanchéité (24) dudit lot dans un espace de réception (27).

3. Procédé selon la revendication 2, **caractérisé en ce que** le paramètre de montage (M) est choisi respectivement de telle sorte qu'il résulte respectivement la même valeur de déformation de l'élément d'étanchéité (24) pour des dispositifs de distribution (1) complètement montés pour différents lots d'éléments d'étanchéité (24), de préférence dans lequel la valeur de déformation est une mesure de la force, avec laquelle l'élément d'étanchéité (24) est déformé, en particulier comprimé.

4. Procédé selon la revendication 2 ou 3, **caractérisé en ce que** pour définir le paramètre de montage (M) d'un lot, un paramètre de montage (M) est défini séparément en premier lieu pour chaque élément d'étanchéité (24) de l'échantillon et une valeur moyenne desdits paramètres de montage (M) définis séparément est arrêtée en tant que paramètre de montage (M) pour tous les éléments d'étanchéité (24) dudit lot.

5. Procédé selon l'une quelconque des revendications 2 à 4, **caractérisé en ce que** pour définir le paramètre de montage (M), un élément central (30) est guidé à travers une ouverture (24a) de l'élément d'étanchéité (24) ou à travers l'espace de réception (27), dans lequel le volume de l'espace de réception (27) est réduit ou limité par l'élément central (30).

6. Procédé selon l'une quelconque des revendications 2 à 5, **caractérisé en ce que** le nombre d'éléments d'étanchéité (24) de l'échantillon est inférieur à 50 ‰, de manière préférée inférieur à 20 ‰, en particulier inférieur à 10 ‰, de manière particulièrement préférée inférieur à 5 °o, de manière très particulièrement préférée inférieur à 2 °o, au nombre d'éléments d'étanchéité (24) du lot, et/ou que les volumes des éléments d'étanchéité (24) d'un lot divergent de moins de 10 %, de préférence de moins de 5 %, de manière particulièrement préférée de moins de 4 %, par rapport au volume moyen des éléments d'étanchéité (24) du lot.

7. Procédé selon la revendication 1, **caractérisé en ce que** l'élément d'étanchéité (24) est fixé de manière définitive dans l'espace de réception (27) directement après la déformation et la définition du paramètre de montage (M) au moyen d'un élément de blocage (25) ou d'un élément d'appui (26) agissant sur l'élément d'étanchéité (24).

8. Procédé selon la revendication 1 ou 7, **caractérisé en ce que** le paramètre de montage (M) est défini séparément pour chaque élément d'étanchéité (24) à monter et n'est utilisé respectivement que lors du blocage dudit élément d'étanchéité (24), pour lequel précisément il a été défini.

9. Procédé selon l'une quelconque des revendications précédentes, dans lequel le paramètre de montage (M) est une valeur, en particulier réglable et/ou géométrique, qui est spécifiée et/ou réalisée lors du montage du dispositif de distribution (1), de préférence dans lequel le paramètre de montage (M) peut être spécifié de manière préférée de manière variable dans les réglages d'un déroulement de montage.

10. Procédé selon l'une quelconque des revendications précédentes, **caractérisé en ce**
**que** le paramètre de montage (M) représente une position, en particulier axiale, d'un élément de blocage (25) ou d'un élément d'appui (26) par rapport à l'espace de réception (27) ou à un élément de guidage (23) du dispositif de distribution (1), ou
**qu'**une position, en particulier axiale, d'un élément de blocage (25) ou d'un élément d'appui (26) par rapport à l'espace de réception (27) ou à un élément de guidage (23) du dispositif de distribution (1) est définie par le paramètre de montage (M),
en particulier dans lequel l'élément de guidage (23) délimite au moins en partie l'espace de réception (27) et l'élément de blocage (25) ou l'élément d'appui (26) est utilisé pour fixer l'élément d'étanchéité (24) dans l'espace de réception (27).

11. Procédé selon l'une quelconque des revendications précédentes, **caractérisé en ce que** le paramètre de montage (M) correspond à un volume de l'espace de réception (27) et/ou à une valeur de déformation, en particulier une compression de volume d'utilisation, de l'élément d'étanchéité (24).

12. Procédé selon l'une quelconque des revendications précédentes, **caractérisé en ce que** le paramètre de montage (M) est choisi ou défini de telle sorte que lors d'un blocage de l'élément d'étanchéité (24) dans l'espace de réception (27), une valeur de déformation correspondant à une déformation de l'élément d'étanchéité (24) atteint ou dépasse une valeur de seuil, de préférence dans lequel la valeur de déformation est une mesure de la force, avec laquelle l'élément d'étanchéité (24) est déformé, et la valeur de seuil correspond à une compression de volume d'utilisation de l'élément d'étanchéité (24).

13. Procédé selon l'une quelconque des revendications précédentes, **caractérisé en ce que** pour définir le comportement de déformation et/ou le comportement de frottement de l'élément d'étanchéité (24) respectif, une valeur caractéristique est respectivement mesurée lors de différentes déformations, laquelle correspond à une valeur de déformation de l'élément d'étanchéité (24) et/ou à partir de laquelle la valeur de déformation de l'élément d'étanchéité (24) peut être calculée, définie et/ou déduite.

14. Procédé selon la revendication 13, **caractérisé en ce que** la valeur caractéristique est une force, en particulier une force de frottement ou une force de pression, une pression ou une position.

15. Procédé selon l'une quelconque des revendications précédentes, **caractérisé en ce que** pour définir le comportement de déformation de l'élément d'étanchéité (24) respectif, respectivement la force requise pour la déformation est définie pour différentes déformations.

16. Procédé selon l'une quelconque des revendications précédentes, **caractérisé en ce que** pour définir le comportement de frottement de l'élément d'étanchéité (24) respectif, une limitation de l'espace de réception (27) et l'élément d'étanchéité (24) sont déplacés l'un par rapport à l'autre et respectivement la force de frottement entre l'élément d'étanchéité (24) et la limitation est définie pour différentes déformations.

17. Procédé selon l'une quelconque des revendications précédentes, **caractérisé en ce que** pour définir le comportement de déformation ou le comportement de frottement et/ou pour définir le paramètre de montage (M), une courbe de force-déplacement est relevée, de préférence dans lequel le paramètre de montage (M) est défini à l'aide de l'évolution ou de la courbure de la courbe de force-déplacement.

18. Procédé selon l'une quelconque des revendications précédentes, **caractérisé en ce que** l'élément d'étanchéité (24) est réalisé pour étanchéifier un élément de convoyage (9) pour convoyer le médicament (2) contre un élément de guidage (23) présentant ou formant l'espace de réception (27), dans lequel l'élément de convoyage (9) est guidé.

19. Dispositif de distribution (1) destiné à distribuer un médicament (2), avec un élément de convoyage (9) pouvant être déplacé en particulier axialement, destiné à convoyer le médicament (2), un élément de guidage (23) destiné à guider l'élément de convoyage (9) et un élément d'étanchéité (24) en particulier annulaire destiné à étanchéifier l'élément de convoyage (9) contre l'élément de guidage (23), dans lequel l'élément de guidage (23) délimite au moins en partie un espace de réception (27) pour recevoir l'élément d'étanchéité (24) et l'élément d'étanchéité (24) peut être fixé dans l'espace de réception (27) au moyen d'un élément de blocage (25) ou d'un élément d'appui (26) pouvant être maintenu sur l'élément de guidage (23),
**caractérisé en ce**
**que** l'élément de fixation ou l'élément d'appui (25, 26) peut être maintenu dans des positions discrètes différentes sur l'élément de guidage (23) et ainsi l'élément d'étanchéité (24) peut être fixé avec un paramètre de montage (M) défini au préalable, dans lequel l'élément de blocage ou l'élément d'appui (25, 26) peut être enclenché dans les différentes positions discrètes sur l'élément de guidage (23) et dans lequel les différentes positions discrètes sont des positions axialement différentes de l'élément de blocage ou d'appui (25, 26) par rapport à l'élément de guidage (23) .

20. Dispositif de distribution selon la revendication 19, **caractérisé en ce que** l'élément de blocage ou d'appui (25, 26) et/ou l'élément de guidage (23) présente ou présentent un système de positionnement (31) destiné au positionnement de l'élément de blocage ou de l'élément d'appui (25, 26) par rapport à l'élément de guidage (23), dans lequel le système de positionnement (31) présente un ou plusieurs éléments d'enclenchement (31b) ou en est formé.

21. Dispositif de distribution selon la revendication 20, **caractérisé en ce que** le système de positionnement (31) présente un plan incliné ou une structure hélicoïdale (31a) ou en est formé.

22. Dispositif de distribution selon l'une quelconque des revendications 19 à 21, **caractérisé en ce que** la position rotative de l'élément de blocage ou d'appui (25, 26), en particulier par rapport à l'élément de guidage (23) ou au dispositif de distribution (1), arrête la position axiale de l'élément de blocage ou d'appui (25, 26) .

23. Dispositif de distribution selon l'une quelconque des revendications 19 à 22, **caractérisé en ce que** la position de l'élément de blocage ou de l'élément d'appui (25, 26) définit une déformation de l'élément d'étanchéité (24) et/ou une dimension de l'espace de réception (27) si bien que la déformation de l'élément d'étanchéité (24) et/ou la dimension de l'espace de réception (27) peuvent varier par une variation de la position de l'élément de blocage (25) ou de l'élément (26) .
